(19) Europäisches Patentamt
European Patent Office
Office européen des brevets

(11) EP 0 830 859 B1

(12) **EUROPEAN PATENT SPECIFICATION**

(45) Date of publication and mention of the grant of the patent:
**10.12.2003 Bulletin 2003/50**

(51) Int Cl.$^7$: **A61K 9/16**, A61K 9/22

(21) Application number: **97202533.2**

(22) Date of filing: **18.08.1997**

(54) **Polyetheresters copolymers as drug delivery matrices**

Polyätherester-Copolymere als Matrixmaterialien für die Arzneistoffabgabe

Copolymères de polyétheresters comme matrice pour la délivrance de médicaments

(84) Designated Contracting States:
**AT BE CH DE DK ES FI FR GB GR IE IT LI LU MC NL PT SE**

(30) Priority: **16.08.1996 US 699896**

(43) Date of publication of application:
**25.03.1998 Bulletin 1998/13**

(60) Divisional application:
**02077025.1 / 1 247 522**

(73) Proprietor: **Chienna B.V.**
**3723 MB Bilthoven (NL)**

(72) Inventors:
- **Goedemoed, Jacob Hillebrand**
**1071 RR Amsterdam (NL)**
- **Hennink, Wilhelmus Everhardus**
**2743 CZ Waddinxveen (NL)**
- **Bezemer, Jeroen Mattijs**
**7556 ZK Hengelo (NL)**
- **Feijen, Jan**
**7552 GD Hengelo (NL)**
- **Van Blitterswijk, Clemens Antoni**
**3467 PD Hekendorp (NL)**
- **De Bruijn, Joost Dick**
**2517 AG Den Haag (NL)**

(74) Representative: **Prins, Adrianus Willem et al**
**Vereenigde,**
**Nieuwe Parklaan 97**
**2587 BN Den Haag (NL)**

(56) References cited:
**EP-A- 0 717 999**
**US-A- 5 384 333**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 830 859 B1

Printed by Jouve, 75001 PARIS (FR)

## Description

This invention relates to drug delivery matrices which contain and release an active agent. More particularly, this invention relates to a pharmaceutical composition which includes a biologically active agent encapsulated in a matrix comprising a polyetherester copolymer, such as, for example, a polyethylene glycol terephthalate/polybutylene terephthalate copolymer.

Considerable research has been undertaken in the field of delivery matrices which contain and deliver various biologically active agents. One reason is to develop delivery systems which prolong the release time of existing drugs. Another reason is that many new drugs which have been developed have poor pharmacokinetic profiles. In particular, peptides and proteins cause pharmacokinetic difficulties. Such substances must be administered parenterally if a systemic action is required. Also, many new drugs have short half lives, which necessitates frequent injection schedules. Patient compliance and the high cost associated with frequent dosing protocols for parenterally administered drugs provide strong stimuli for alternative dosage forms and dosing regimens.

Polymeric systems which presently are under investigation as drug delivery matrices include polylactic acid (PLA) and copolymers of polylactic acid with glycolic acid. Such copolymers also are known as PLGA polymers. (Brannon-Peppas, *Int. J. Pharmaceutics*, Vol. 116, 1-9 (1995); Couvreur, et al., *Advanced Drug Delivery Reviews*, Vol. 10, 141-162 (1993).) Conti, et al., *J. Microencapsulation*, Vol. 9, 153-166 (1992), describe a number of methods to prepare drug loaded microspheres from PLGA polymers. PLGA-containing microspheres, however, have disadvantages which include the following. Firstly, the ability to manipulate the release of an encapsulated protein is limited because for most proteins, diffusion in PLGA matrices is negligible. The release of proteins from PLGA, therefore, depends upon the diffusion via pores present in the matrix and on the degradation or dissolution time of the microsphere. Also, during degradation of the PLGA, a low pH is generated in the polymeric matrix, which may be deleterious for many proteins. US 5,439,688 describes microparticles containing a peptide in a poly(butylene succinate) matrix having sustained release properties. EP-A-0 717 999 and US-A-5 384 333 describe injectable drug compositions which provide long-term drug release.

In accordance with an aspect of the present invention, there is provided a composition for delivering a biologically active agent to a host, which composition does not have the limitations of prior art delivery systems. The composition comprises a product including the biologically active agent encapsulated in a matrix comprising a copolymer of a polyalkylene glycol and an aromatic polyester.

The copolymer comprises from 30 wt. % to 99 wt. %, especially 30 to 90 wt. %, based upon the weight of the copolymer, of a first component which comprises a polyalkylene glycol and has units of the formula -OLO-CO-Q-CO-, wherein L is a divalent organic radical remaining after removal of terminal hydroxyl groups from a poly(oxyalkylene) glycol, and Q is a divalent organic radical, and from 1 wt. % to 70 wt. %, especially 10 to 70 wt. %, based on the weight of the copolymer, of a second component which is an aromatic polyester, having units of the formula -O-E-O-CO-R-CO- wherein E is a substituted or unsubstituted alkylene or oxydialkylene radical having from 2 to 8 carbon atoms, and R is a substituted or unsubstituted divalent aromatic radical.

The polyalkylene glycol, in one embodiment, is selected from polyethylene glycol, polypropylene glycol, and polybutylene glycol and copolymers thereof such as poloxamers. In one embodiment, the polyalkylene glycol is polyethylene glycol. The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-butylene (tetramethylene) and similarly for higher alkylene homologues. The polyalkylene glycol component is terminated with a dicarboxylic acid residue -CO-Q-CO- if necessary to provide a coupling to the polyester component. The group Q may be an aromatic group having the same definition as R, or may be an aliphatic group such as ethylene, propylene, butylene.

In the polyester component, the divalent aromatic group R may be phenylene, pyridylene, naphthylene, biphenyl, oxydiphenyl, sulphodiphenyl, methylenediphenyl, optionally substituted by up to four, preferably up to two substituents selected from chloro, nitro, and $C_1$-$C_4$ alkoxy and further fluoro, hydroxy and $C_1$-$C_4$ alkyl. Preferably, the aromatic radical R is unsubstituted, and most preferably, R is 1,4-phenylene. Examples of the polyester -O-E-O-CO-R-CO- comprise poly(ethylene terephthalate), poly(propylene terephthalate), poly(butylene terephthalate), poly(butylene isophthalate), poly(ethylene 4,4'-methylenediphenyldicarboxylate), poly(butylene 5,6,7,8-tetrahydronaphthalene-1,4-dicarboxylate).

In a preferred embodiment, the polyester is selected from polyethylene terephthalate, polypropylene terephthalate, and polybutylene terephthalate. Preferably, the polyester is polybutylene terephthalate. In a most preferred embodiment, the copolymer is a polyethylene glycol/polybutylene terephthalate block copolymer.

The polyalkylene glycol may have a molecular weight of from 200 to 20,000. The exact molecular weight of the poly(oxyethylene) glycol is dependent upon a variety of factors, including the type of biologically active agent encapsulated by the matrix.

As an example, a polyethylene glycol/polybutylene terephthalate copolymer may be synthesized from a mixture of dimethyl terephthalate, butanediol (in excess), polyethylene glycol, an antioxidant, and catalyst. The mixture is placed in a reaction vessel and heated to about 180°C, and methanol is distilled as transesterification occurs. During the transesterification, the ester bond with methyl is replaced with an ester bond with butylene. In this step the polyethylene glycol does not react. After transesterification, the temperature is raised slowly to 245°C, and a vacuum (finally less than 0.1 mbar) is achieved. The excess butanediol is distilled and a prepolymer of butanediol terephthalate condenses with the polyethylene glycol to form a polyethylene glycol/polybutylene terephthalate copolymer. A terephthalate moiety connects the polyethylene glycol units to the polybutylene terephthalate units of the copolymer, and thus such copolymer also is sometimes hereinafter referred to as a polyethylene glycol terephthalate/polybutylene terephthalate copolymer, or PEGT/PBT copolymer. In another alternative, polyalkylene glycol/polyester copolymers may be prepared as described in US 3,908,201. The synthesis of the copolymers of the invention is known per se and the invention is not limited to any particular means of synthesis.

The term "biologically active agent," as used herein, means an agent which provides a therapeutic or prophylactic effect. Such agents include antimicrobial agents (including antibacterial and anti-fungal agents), anti-viral agents, anti-tumour agents, hormones, immunogenic agents, growth factors, lipids, and lipopolysaccharides.

Biologically active agents which may be contained in the copolymer matrix, include non-peptide, non-protein small-sized drugs. They have a molecular weight which in general is less than 1500, and in particular less than 500. A second important group of biologically active agents are biologically active peptides and proteins.

Examples of non-peptide, non-protein small-sized drugs which may be contained in the copolymer matrix include the following:

1. Anti-tumour agents: altretamin, fluorouracil, amsacrin, hydroxycarbamide, asparaginase, ifosfamid, bleomycin, lomustin, busulfan, melphalan, chlorambucil, mercaptopurin, chlormethin, methotrexate, cisplatin, mitomycin, cyclophosphamide, procarbazin, cytarabin, teniposid, dacarbazin, thiotepa, dactinomycin, tioguanin, daunorubicin, treosulphan, doxorubicin, tiophosphamide, estramucin, vinblastine, etoglucide, vincristine, etoposid, vindesin.
2. Antimicrobial agents

   2.1 Antibiotics
   *Penicillins*: ampicillin, nafcillin, amoxicillin, oxacillin, azlocillin, penicillin G, carbenicillin, penicillin V, dicloxacillin, phenethicillin, floxacillin, piperacillin, mecillinam, sulbenicillin, methicillin, ticarcillin, mezlocillin
   *Cephalosporins*: cefaclor, cephalothin, cefadroxil, cephapirin, cefamandole, cephradine, cefatrizine, cefsulodine, cefazolin, ceftazidim, ceforanide, ceftriaxon, cefoxitin, cefuroxime, cephacetrile, latamoxef, cephalexin
   *Aminoglycosides*: amikacin, neomycin, dibekacyn, kanamycin, gentamycin, netilmycin, kanamycin, tobramycin
   *Macrolides*: amphotericin B, novobiocin, bacitracin, nystatin, clindamycin, polymyxins, colistin, rovamycin, erythromycin, spectinomycin, lincomycin, vancomycin
   *Tetracyclines:* chlortetracycline, oxytetracycline, demeclocycline, rolitetracycline, doxycycline, tetracycline, minocycline
   *Other antibiotics*: chloramphenicol, rifamycin, rifampicin, thiamphenicol
   2.2 Chemotherapeutic agents
   *Sulfonamides*: sulfadiazine, sulfamethizol, sulfadimethoxin, sulfamethoxazole, sulfadimidin, sulfamethoxypyridazine, sulfafurazole, sulfaphenazol, sulfalene, sulfisomidin, sulfamerazine, sulfisoxazole, trimethoprim with sulfamethoxazole or sulfametrole
   *Urinary tract antiseptics*: methenamine, quinolones (norfloxacin, cinoxacin), nalidixic acid, nitro-compounds (nitrofurantoine, nifurtoinol), oxolinic acid
   *Anaerobic infections*: metronidazole

3. Drugs for tuberculosis: aminosalicyclic acid, isoniazide, cycloserine, rifampicine, ethambutol, tiocarlide, ethionamide, viomycin
4. Drugs for leprosy: amithiozone, rifampicine, clofazimine, sodium sulfoxone, diaminodiphenylsulfone (DDS, dapsone)
5. Antifungal agents: amphotericin B, ketoconazole, clotrimazole, miconazole, econazole, natamycin, flucytosine, nystatine, griseofulvin
6. Antiviral agents: aciclovir, idoxuridine, amantidine, methisazone, cytarabine, vidarabine, ganciclovir
7. Chemotherapy of amebiasis: chloroquine, iodoquinol, clioquinol, metronidazole, dehydroemetine, paromomycin, diloxanide, furoatetinidazole, emetine,
8. Anti-malarial agents: chloroquine, pyrimethamine, hydroxychloroquine, quinine, mefloquine, sulfadoxine/pyrimethamine, pentamidine, sodium suramin, primaquine, trimethoprim, proguanil
9. Anti-helminthiasis agents: antimony potassium tartrate, niridazole, antimony sodium dimercaptosuccinate, oxamniquine, bephenium, piperazine, dichlorophen, praziquantel, diethylcarbamazine, pyrantel pamoate, hycanthone, pyrivium pamoate, levamisole, stibophen, mebendazole, tetramisole, metrifonate, thiobendazole, niclosamide
10. Anti-inflammatory agents: acetylsalicyclic acid, mefenamic acid, alclofenac, naproxen, azopropanone, niflumic acid, benzydamine, oxyphenbutazone, diclofenac, piroxicam, fenoprofen, pirprofen, flurbiprofen, sodium salicyclate, ibuprofensulindac, indomethacin, tiaprofenic acid, ketoprofen, tolmetin
11. Anti-gout agents: colchicine, allopurinol
12. Centrally acting (opoid) analgesics: alfentanil, methadone, bezitramide, morphine, buprenorfine, nicomorphine, butorfanol, pentazocine, codeine, pethidine, dextromoramide, piritranide, dextropropoxyphene, sufentanil, fentanyl
13. Local anesthetics: articaine, mepivacaine, bupivacaine, prilocaine, etidocaine, procaine, lidocaine, tetracaine
14. Drugs for Parkinson's disease: amantidine, diphenhydramine, apomorphine, ethopropazine, benztropine mesylate, lergotril, biperiden, levodopa, bromocriptine, lisuride, carbidopa, metixen, chlorphenoxamine, orphenadrine, cycrimine, procyclidine, dexetimide, trihexyphenidyl
15. Centrally acting muscle relaxants: baclofen, carisoprodol, chlormezanone, chlorzoxazone, cyclobenzaprine, dantrolene, diazepam, febarbamate, mefenoxalone, mephenesin, metoxalone, methocarbamol, tolperisone
16. Hormones and hormone antagonists

16.1 Corticosteroids

16.1.1 *Mineralocorticosteroids*: cortisol, desoxycorticosterone, fluorhydrocortisone
16.1.2 *Glucocorticosteroids*: beclomethasone, betamethasone, cortisone, dexamethasone, fluocinolone, fluocinonide, fluocortolone, fluorometholone, fluprednisolone, flurandrenolide, halcinonide, hydrocortisone, medrysone, methylprednisolone, paramethasone, prednisolone, prednisone, triamcinolone (acetonide)

16.2 Androgens

16.2.1 *Androgenic steroids used in therapy*: danazole, fluoxymesterone, mesterolone, methyltestosterone, testosterone and salts thereof
16.2.2 *Anabolic steroids used in therap*y: calusterone, nandrolone and salts thereof, dromostanolone, oxandrolone, ethylestrenol, oxymetholone, methandriol, stanozolol methandrostenolone, testolactone
16.2.3 *Antiandrogens*: cyproterone acetate

16.3 Estrogens:

16.3.1 *Estrogenic steroids used in therapy: diethylstilbestrol, estradiol, estriol,* ethinylestradiol, mestranol, quinestrol
16.3.2 *Anti-estrogens*: chlorotrianisene, clomiphene, ethamoxytriphetol, nafoxidine, tamoxifen

16.4 *Progestins:* allylestrenol, desogestrel, dimethisterone, dydrogesterone, ethinylestrenol, ethisterone, ethynadiol diacetate, etynodiol, hydroxyprogesterone, levonorgestrel, lynestrenol, medroxyprogesterone, megestrol acetate, norethindrone, norethisterone, norethynodrel, norgestrel, progesterone

17. Thyroid drugs

17.1 *Thyroid drugs used in therapy*: levothyronine, liothyronine
17.2 *Anti-thyroid drugs used in therapy*: carbimazole, methimazole, methylthiouracil, propylthiouracil.

When a small-sized drug, such as those described above, is contained in a copolymer matrix, the polyalkylene glycol component of the copolymer preferably has a molecular weight of from 200 to 400. Also, the polyalkylene glycol component, optionally as the terephthalate, is present in the copolymer in an amount of from 30 wt.% to 80 wt.% of the weight of the copolymer, preferably from 50 wt.% to 60 wt.% of the weight of the copolymer. In general, the aromatic polyester is present in the copolymer in an amount of from 20 wt.% to 70 wt. % of the copolymer, preferably in an amount of from 40 wt.% to 50 wt.% of the copolymer.

When the polyetherester matrix, such as a polyethylene glycol terephthalate/polybutylene terephthalate matrix, contains a hydrophobic small-sized drug, such as, for example, a steroid hormone, the matrix also may include at least one hydrophobic antioxidant. Hydrophobic antioxidants which may be employed include tocopherols, such as $\alpha$-tocopherol, $\beta$-tocopherol, $\gamma$-tocopherol, $\delta$-tocopherol, $\varepsilon$-tocopherol, $\zeta_1$-tocopherol, $\zeta_2$-tocopherol, and $\eta$-tocopherol; and 1-ascorbic acid 6-palmitate. Such hydrophobic anti-oxidants retard the degradation of the polyetherester copolymer

matrix, and retard the release of the biologically active agent contained in the matrix. Thus, the use of a hydrophobic or lipophilic antioxidant is applicable particularly to the formation of matrices which include drugs which tend to be released quickly from the microspheres, such as, for example, small drug molecules having a molecular weight less than 500. The at least one hydrophobic antioxidant may be present in the matrix in an amount of from 0.1 wt. % to 10 wt. % of the total weight of the matrix, preferably from 0.5 wt. % to 2 wt. %.

When the matrix includes a hydrophilic small-sized drug, such as an aminoglycoside, the matrix may also include, in addition to the hydrophobic antioxidant, a hydrophobic molecule such as cholesterol, ergosterol, lithocholic acid, cholic acid, dinosterol, betuline, or oleanolic acid, which may be employed in order to retard the release rate of the agent from the copolymer matrix. Such hydrophobic molecules prevent water penetration into the matrix, but do not compromise the degradability of the matrix. In addition, such molecules have melting points from 150°C to 200°C or more. Therefore, a small percentage of these molecules increases the glass transition temperature of the matrix, which decreases the matrix diffusion coefficient for the biologically active agent, such as a small drug molecule, to be released. Thus, such hydrophobic molecules provide for a more sustained release of a biologically active agent from the matrix. The at least one hydrophobic molecule may be present in the matrix in an amount of from 0.1 wt. % to 20 wt. %, preferably from 1.0 wt. % to 5.0 wt. %.

If it is desired to increase the hydrophilicity of the polymer, and thereby increase the degradation rate and drug releasing rate of the copolymer, the copolymer may be modified by partially replacing the aromatic moiety with an aliphatic moiety such as succinate and/or by replacing partially the alkylene with dioxyethylene. Fore example, terephthalate can be replaced by succinate in an amount of from 0.1 mole % to 20 mole %, preferably from 0.1 mole % to 5 mole %, by partially replacing dimethyl terephthalate as a starting component with dimethyl succinate. As another example, butylene is replaced with oxydiethylene in an amount of from 0.1 mole % to 20 mole %, preferably from 0.5 mole % to 2 mole %, by replacing 1,4-butanediol with diethyleneglycol as a starting component.

Examples of peptides or proteins which may advantageously be contained in the matrix include immunogenic peptides or immunogenic proteins, which include the following:

*Growth factors:* bone morphogenetic proteins, transforming growth factors, fibroblast growth factors, epidermal growth factors etc.
*Toxins:* diphtheria toxin, tetanus toxin
*Viral surface antigens or parts of viruses:* adenoviruses, Epstein-Barr Virus, Hepatitis A Virus, Hepatitis B Virus, Herpes viruses, HIV-1, HIV-2, HTLV-III, Influenza viruses, Japanese encephalitis virus, Measles virus, Papilloma viruses, Paramyxoviruses, Polio Virus, Rabies Virus, Rubella Virus, Vaccinia (Smallpox) viruses, Yellow Fever Virus
*Bacterial surface antigens or parts of bacteria:* Bordetella pertussis, Helicobacter pylori, Clostridium tetani, Corynebacterium diphtheria, Escherichia coli, Haemophilus influenza, Klebsiella species, Legionella pneumophila, Mycobacterium bovis, Mycobacterium leprae, Mycobacterium tuberculosis, Neisseria gonorrhoeae, Neisseria meningitidis, Proteus species, Pseudomonas aeruginosa, Salmonella species, Shigella species, Staphylococcus aureus, Streptococcus pyogenes, Vibrio cholera, Yersinia pestis
*Surface antigens of parasites causing disease or portions of parasites:* Plasmodium vivax - malaria, Plasmodium falciparum - malaria, Plasmodium ovale - malaria, Plasmodium malariae - malaria, Leishmania tropica - leishmaniasis, Leishmania donovani - leishmaniasis, Leishmania branziliensis - leishmaniasis, Trypanosoma rhodescense - sleeping sickness, Trypanosoma gambiense - sleeping sickness, Trypanosoma cruzi - Chagas' disease, Schistosoma mansoni - schistosomiasis, Schistosoma haematobium - schistosomiasis, Schistosoma japonicum - schistosomiasis, Trichinella spiralis - trichinosis, Stronglyloides duodenale - hookworm, Ancyclostoma duodenale - hookworm, Necator americanus - hookworm, Wucheria bancrofti - filariasis, Brugia malaya - filariasis, Loa loa - filariasis, Dipetalonema perstaris - filariasis, Dracuncula medinensis - filariasis, Onchocerca volvulus - filariasis
*Immunoglobulins*: IgG, IgA, IgM, Antirabies immunoglobulin, Antivaccinia immunoglobulin
*Antitoxins:* Botulinum antitoxin, diphtheria antitoxin, gas gangrene antitoxin, tetanus antitoxin.

Other peptides or proteins which may be encapsulated include antigens which elicit an immune response against Foot and Mouth Disease, hormones and growth factors such as follicle stimulating hormone, prolactin, angiogenin, epidermal growth factor, calcitonin, erythropoietin, thyrotropic releasing hormone, insulin, growth hormones, insulin-like growth factors 1 and 2, skeletal growth factor, human chorionic gonadotropin, luteinizing hormone, nerve growth factor, adrenocorticotropic hormone (ACTH), luteinizing hormone releasing hormone (LHRH), parathyroid hormone (PTH), thyrotropin releasing hormone (TRH), vasopressin, cholecystokinin, and corticotropin releasing hormone; cytokines, such as interferons, interleukins, colony stimulating factors, and tumour necrosis factors; fibrinolytic enzymes, such as urokinase, kidney plasminogen activator; and clotting factors, such as Protein C, Factor VIII, Factor IX, Factor VII, and Antithrombin III. Examples of other proteins or peptides which may be encapsulated include albumin, atrial natriuretic factor, renin, superoxide dismutase, $\alpha_1$-antitrypsin, lung surfactant proteins, bacitracin, bestatin, cydosporine, delta sleep-inducing peptide (DSIP), endorphins, glucagon, gramicidin, melanocyte inhibiting factors, neurotensin, oxytocin, somostatin, terprotide, serum thymide factor, thymosin, DDAVP, dermorphin, Met-enkephalin, peptidoglycan, satietin, thymopentin, fibrin degradation product, des-enkephalin-α-endorphin, gonadotropin releasing hormone, leuprolide, α-MSH, and metkephamid.

When a protein having a molecular weight of greater than 10,000 is contained in the polyalkylene glycol / aromatic polyester matrix, the polyalkylene glycol component of the copolymer may have a molecular weight of from about 1,000 to about 20,000. The polyalkylene glycol terephthalate may be present in the copolymer in an amount of from 30 wt.% to 90 wt.% of the weight of the copolymer, preferably from 60 wt. % to 70 wt. %. The polybutylene terephthalate may be present in the copolymer in an amount of from 10 wt. % to 70 wt. % of the weight of the copolymer, preferably from 30 wt. % to 40 wt. %.

When the copolymer matrix contains a protein, the matrix may also include a hydrophilic antioxidant. Examples of hydrophilic antioxidants include hydroxyphenylcarbonyl, hydroxybenzofuranylcarbonyl and hydroxychromanylcarbonyl derivatives of polyalkylene glycol e.g. having one of the following formulae:

$$X^1 - Q - (CH_2\text{-}CH_2\text{-}O)_n - R^2, \qquad X^1 - Q - (CH_2\text{-}CH_2\text{-}O)_n - R^3 - Q - X^2$$

Each of $X^1$ and $X^2$ is independently selected e.g. from the following groups:

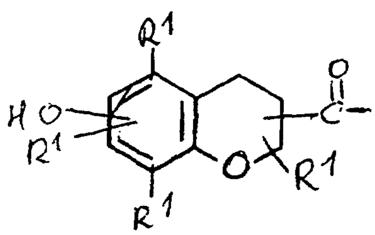
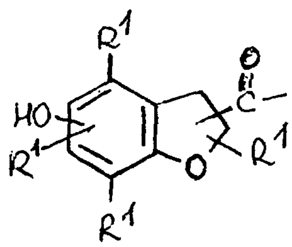
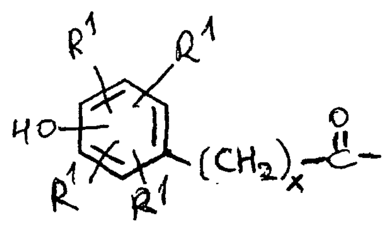

Each $R^1$ is independently selected from hydrogen and alkyl groups having 1 to 4 carbon atoms, preferably methyl. n is from 1 to 100, preferably from 4 to 22. $R^2$ is an alkyl group having from 1 to 4 carbon atoms, preferably 1 or 2 carbon atoms and $R^3$ is an alkylene group having from 1 to 4 carbon atoms, preferably 1 or 2 carbon atoms. x is a number from 0 to 4.

In one embodiment, at least one, preferably two, of the $R^1$ moieties in the hydroxyphenyl(alkyl)carbonyl structure is a tert-butyl moiety. When two of the $R^1$ moieties are tert-- butyl moieties, each tert-butyl moiety preferably is adjacent to the -OH group. In another embodiment, the hydrophilic antioxidant is N-(methyl-polyoxyethylene)-6-hydroxy-2,5,8-trimethyl-chroman-2-carboxamide, wherein poly means 21 or 22.

The at least one hydrophilic antioxidant may be present in the matrix in an amount of from 0.1 wt. % to 10 wt. % of the total weight of the matrix, preferably from 1.0 wt. % to 5.0 wt. %.

The hydrophilic antioxidants in general provide for an increased degradation rate of the polyetherester copolymer. The increased degradation of the polyetherester copolymer, however, is not accompanied by an increase in acid formation. Thus, the use of such hydrophilic antioxidants in the matrix is applicable particularly to the encapsulation of peptide or protein molecules by the matrix. Thus, when the protein or peptide molecule is released from the matrix upon the degradation thereof, the protein will not be denatured by acid degradation products. Therefore, a hydrophilic antioxidant may be employed in order to provide for an increased rate of release of peptide or protein molecule from the matrix while avoiding denaturation of the peptide or protein. Below, reference is made to a PEGT/PBT (polyethylene glycol terephthalate / poly(butylene terephthalate) polymer by way of example, but the same applies *mutatis mutandis* to the other polyether/ester copolymers defined above.

The degradation rate of the PEGT/PBT copolymer also may be increased, if desired, by replacing partially the terephthalate with succinate and/or by replacing partially the butylene with dioxyethylene in the amounts described above.

If one wishes to increase the diffusion rate of the protein having a molecular weight greater than 10,000 through the PEGT/PBT copolymer, one may add polyethylene glycol having a molecular weight of from 4,000 to 10,000 to the PEGT/PBT copolymer. Such polyethylene glycol may be present in an amount of up to 10 wt.%, based on the weight of the PEGT/PBT copolymer.

In addition, the diffusion rate of the protein may be increased by increasing the weight of the polyethylene glycol component of the PEGT/PBT copolymer. For example, when the polyethylene glycol component of the PEGT/PBT copolymer has a molecular weight of from 6,000 to 20,000, hydrophilic pores may be formed in the matrix, such

pores providing an increased diffusion rate.

When the PEGT/PBT matrix includes a peptide or protein having a molecular weight less than 10,000, the polyethylene glycol preferably has a molecular weight of from 200 to 6,000. The polyethylene glycol terephthalate may be present in the copolymer in an amount of from 30 wt.% to 80 wt. %, based on the weight of the copolymer. The polybutylene terephthalate may be present in the copolymer in an amount of from 20 wt. % to 70 wt. %, based on the weight of the copolymer.

The PEGT/PBT matrix may include a hydrophobic antioxidant, hydrophobic molecule, and/or a hydrophilic antioxidant in the amounts described above. The type and exact amount of antioxidant or hydrophobic molecule employed is dependent upon the molecular weight of the protein. If the PEGT/PBT matrix copolymer includes a peptide having a low molecular weight (such as, for example, less than 500), the PEGT/PBT copolymer may include a hydrophobic antioxidant and/or hydrophobic molecule as described above. If the PEGT/PBT copolymer matrix contains a large peptide or protein (such as, for example, insulin), the matrix also may include a hydrophilic antioxidant such as those described above and in the amounts described above, and may also include polyethylene glycol having a molecular weight from 1,000 to 4,000, in an amount of from 1 wt.% to 10 wt. %, based on the weight of the copolymer.

If desired, the degradation rate of the PEGT/PBT copolymer may be increased by replacing partially the terephthalate with succinate and/or by replacing partially the butylene with dioxyethylene, in amounts described above.

The polyetherester copolymer, such as a PEGT/PBT copolymer, with or without the at least one antioxidant, or hydrophobic molecule, or polyethylene glycol, is formed into shaped structures which contain a biologically active agent, such as a drug or a protein as described above. In general, the shaped structures may be e.g. microspheres, rods, pellets, sheets, threads etc. depending on the desired administration form of the drug or protein. Microspheres are fine spherical particles having a diameter up to 1,000 μm, and containing a biologically active agent. The microsphere may be a homogeneous or monolithic microsphere in which the biologically active agent is dissolved or dispersed throughout the polymer matrix. In another embodiment, the microsphere may be of a reservoir type in which the biologically active agent is surrounded by the polymer in the mononuclear or polynuclear state. In another embodiment, when the biologically active agent is a small hydrophilic drug, the drug may first be dispersed in a hydrophobic or lipophilic excipient, which combination then is dispersed in the form of particles, droplets, or microsuspensions in the polyetherester matrix. Microspheres then can be formed from the emulsion.

The microspheres may be prepared by techniques known to those skilled in the art, including solvent evaporation and spray drying techniques. In one embodiment, a solvent evaporation technique, the polyetherester is placed in an organic solvent such as methylene chloride, and polyvinyl alcohol is employed as an emulsifying agent. The mean particle diameter and the particle size depend upon the viscosity or concentration of the aqueous polyvinyl alcohol phase. Other parameters, such as the concentration of the polyetherester solution and the stirring rate, also have an effect on the particle size and distribution. The concentration of the polyetherester solution determines the porosity of the microspheres. At higher polyetherester concentrations, such as, for example, 20%, dense and smooth microspheres are obtained. At lower concentrations of polyetherester, such as, for example, 5%, porous microspheres are obtained.

When microspheres are formed by a spray drying process, a low concentration of polyetherester copolymer, from 0.5% to 5.0%, preferably 2%, in the organic solvent, such as methylene chloride, is employed. Spray drying results in general in the production of porous, irregularly shaped particles.

Films, sheets and the like may can be prepared by known methods, such as casting.

As the shaped administration forms are being formed, a biologically active agent is encapsulated in the matrix. In general, when the solvent evaporation technique is employed, an aqueous solution of the agent first is emulsified in a solution of the polyetherester in an organic solvent such as methylene chloride. The emulsion then subsequently is emulsified in an aqueous solution of polyvinyl alcohol, which yields a water-in-oil-in-water emulsion. The organic solvent, such as methylene chloride, then is evaporated to yield the microspheres. When the spray drying technique is employed, an aqueous solution of the agent is emulsified in a solution of the polyetherester in an organic solvent such as methylene chloride, as described above. The water-in-oil emulsion then is spray-dried using a spray dryer.

The shaped administration forms may be administered by a variety of means known to those skilled in the art, including parenteral and mucosal administration, such as oral administration (such as, for example, buccal administration), subcutaneous administration, intravenous administration, intra-arterial administration, intraperitoneal administration intramuscular administration, vaginal administration, and rectal administration. Microspheres, blocks, films and tapes etc. may also be administered topically. The polyetheresters compatible with a wide variety of biologically active agents, and thus prevent denaturation of such agents until such agents are delivered to a desired cell, tissue, or organ. Examples of pharmaceutical carriers which may be employed include gels, including hyaluronic acid gels and macromolecular polysaccharide gels, creams, and ointments.

For example, microspheres may be contained in a gel, cream, or ointment, and may, if desired, be covered by a barrier, when administered topically. Thus, the compositions may contain one or more biologically active agents employed in the treatment of skin diseases, such as psoriasis, eczema, seborrhoea, and dermatitis. In another embodiment, the compositions may be contained in a gel such as a hyaluronic acid gel or a macromolecular polysaccharide gel. Such an embodiment is applicable particularly to parenteral applications, such as during and after surgery. When administered by injection, microspheres may be contained in a pharmaceutical carrier such as water, saline solution (for example, 0.9% saline), or a solution containing a surfactant in an amount of from 0.1% wt./vol. to 0.5% wt./vol. Examples of surfactants which may be employed include, but are not limited to, Tween 80 surfactant. In another embodiment, microspheres may be contained in a capsule coated with a gastric protective layer, such as, for example, Eudragit® methacrylate copolymers.

In general, microspheres have a size of up to 1,000 microns, preferably from 1 micron to 500 microns. The size of the microspheres may be dependent upon a variety of factors, including the method of forming the microspheres and the route of administration of the microspheres after their formation.

When administered orally, microspheres in general have a size from 0.1 micron to 1,000 microns. When one desires to deliver the microspheres to the Peyer's patches, the microspheres may have a diameter of from 1 micron to 10 microns, more preferably from 1 micron to 5 microns. The polyetherester copolymer protects the biologically active agent by preventing denaturation of the biologically active agent until the biologically active agent reaches the desired cell, tissue, or organ.

For example, this embodiment is applicable particularly to the oral delivery of peptide or protein molecules (including immunogenic peptides or proteins), whereby the microspheres including the peptide or protein travel through the gastrointestinal tract for delivery to the Peyer's patches located in the ileum or lower part of the intestine. Peptides and proteins, however, are highly susceptible to degradation or denaturation in the gastrointestinal tract. The polyetherester copolymer, which is compatible with peptide and protein molecules, protects the peptide or protein from degradation or denaturation in the gastrointestinal tract until the microspheres deliver the peptide or protein to the Peyer's patches. Thus, the microspheres of the present invention may be employed as part of a pharmaceutical composition for oral administration of biologically active peptides or proteins to the Peyer's patches.

In one embodiment, microspheres may include an immunogenic peptide or protein and thus may be employed in an orally administered vaccine. The polyetherester copolymer protects the immunogenic protein or peptide as it travels through the gastrointestinal tract, until the microspheres deliver the immunogenic protein or peptide to the Peyer's patches. Once the immunogenic peptide or protein is delivered to the Peyer's patches, an immune response is generated against the peptide or protein.

When administered via injection, microspheres have a size of from 1 micron to 30 microns, preferably from 10 microns to 20 microns. Such microspheres, when administered in combination with an acceptable pharmaceutical carrier, may be employed in the treatment of a variety of diseases or disorders, depending upon the biologically active material which is encapsulated. Thus, injectable formulations including the microspheres of the present invention may be employed in the treatment of locally confined diseases such as arthritis, rheumatism, inflammations, local pain processes, local infections, local skin diseases, tumours (or their sites after surgical removal as a post-operative treatment to destroy any tumour cells possibly remaining), and local brain diseases (*e.g.*, Parkinson's disease). Such injectable formulations also may be employed in long-term therapeutic treatments, such as, for example, treatments with corticosteroids, androgens, anti-androgens, estrogens, anti-estrogens, progestangenic agents or thyroid hormones, or with antituberculosis, anti-leprosy, or anti-malaria drugs.

Threads consisting of the polyetherester with active compounds may be prepared by known methods and can be used as surgical sutures containing active compounds. Blocks (porous or non-porous), particulates and granulates can be implanted, e.g. with bone-restoring or bone-improving treatments, and films or sheets can be used as e.g. implantable barriers, guided tissue regeneration membranes and also as plasters.

The compositions of the present invention surprisingly exhibit largely or completely zero-order release patterns, i.e. a release rate which is independent on time or on the amount released. The compositions provide for an exact adjustable and predictable release of the active agent of the composition as is illustrated in the examples. The present invention also provides a method for calculating the release rate of a PEGT/PBT composition as a function of the specific copolymer and the specific active agent (see Example 6).

**EXAMPLE 1**

In this example, poly (ethylene oxide) terephthalate/poly (1,4-butylene) terephthalate (PEGT/PBT) copolymers were synthesized, wherein such copolymers had PEGT/PBT weight ratios of 80/20, 70/30, and 60/40. Such copolymers also are sometimes hereinafter referred to as PolyActive, or PA. The polyethylene glycol component of the PA 70/30 and PA 60/40 copolymers had a molecular weight of 1,000. For the PA 80/20 copolymers, batches having a polyethylene glycol component with molecular weights of 1,000, 2,000, and 4,000 were prepared.

Diphtheria toxoid (Mw 62,000) was obtained from the Netherlands National Institute of Public Health and Environmental Protection, Bilthoven, NL (2025 LF/ml; 1 LF corresponds to about 4 μg protein). The term "LF" means limes flocculation, the International Unit for vaccines.

Polyvinyl alcohol (PVA, 87-89 % hydrolysed; Mw 13,000-23,000) was obtained from Aldrich, BE. Bovine serum albumin (BSA, fraction V) was obtained from Acros, Geel, BE. Lysozyme (chick egg white) was obtained from Sigma and IgG (immunoglobulin G, fraction II) was from ICN Biomedicals, Zoetermeer, NL.

Poly(DL-Lactide/glycolide) 50/50 w/w (batch number DL260AM), intrinsic viscosity 0.58 dl/g, $M_w$ relative to polystyrene was 50 kD, was obtained from Purac, Gorinchem, NL. This polymer is sometimes hereinafter referred to as "PLGA."

Preparation of PEGT/PBT microspheres

For preparation of the PEGT/PBT microspheres, two methods were used, namely, the solvent evaporation method and the spray-drying method. The preparation of PEGT/PBT microspheres using the solvent evaporation method was based on protocols used to prepare PLGA microspheres as disclosed in O'Hagan, et al., Intern. J. Pharmaceutics, Vol. 1033, 37-45 (1994). In general, PEGT/PBT was dissolved in 20 ml $CH_2Cl_2$ (concentration ranging from 5-20 % (w/v)). This solution was added to an aqueous solution of PVA (concentration 1-10 %). After stirring (mechanical stirrer, 1200 rpm) an oil-in-water emulsion was formed. Due to the evaporation of the $CH_2Cl_2$, PEGT/PBT precipitated from the solution to yield microspheres. After two hours (the time necessary for almost complete evaporation of the $CH_2Cl_2$), the formed PEGT/PBT microspheres were collected by centrifugation, washed with water (3 times) and finally air dried.

For the preparation of protein loaded microspheres, first a water-in-oil emulsion was created. The protein was dissolved in water to a concentration of about 170 mg/ml for BSA, 100 mg/ml for lysozyme and 2025 LF/ml for diphtheria toxoid and 0.5 ml of the protein solution was added to 20 ml of PolyActive in $CH_2Cl_2$. This mixture was homogenized using an Ultraturrax (Type 18/10, emulsification time: 10 seconds), yielding a water-in-oil emulsion which was added to 200 ml of an aqueous PVA solution and further treated as described above.

For the preparation of PEGT/PBT microspheres using the spray drying method, the polymer was dissolved in a suitable volatile organic solvent (1 gram PolyActive dissolved in 50 ml $CH_2Cl_2$). This solution was spray dried using a laboratory Buchi Spray Dryer at an inlet temperature of 42°C; an outlet temperature of 38°C; and a flow rate of 5 ml/min.

For the preparation of protein loaded microspheres, first a water-in-oil emulsion was formed as described hereinabove with respect to the solvent evaporation technique which was spray dried as described above.

Preparation of PLGA microspheres

PLGA microspheres were obtained by the solvent evaporation technique and the spray drying technique as described for the PEGT/PBT microspheres.

Microsphere characterization

The particle size ( number weight and volume weight) and the particle size distribution were determined by a laser light blocking technique (Accusizer®, model 770, Particle Sizing Systems, Santa Barbara, CA, USA). The shape and the surface characteristics (porosity) of the microspheres were established by scanning electron microscopy analysis.

Protein determinations

The protein concentrations (BSA, lysozyme) of the samples was measured with the Biorad protein assay using the micro assay procedure described in Bradford, Anal.Biochem., Vol. 72, pgs. 248-254 (1976). Diphtheria toxoid was determined using an enzyme-linked immunosorbent assay (ELISA).

Molecular weight determinations

Molecular weights ($M_n$ and $M_w$) of PEGT/PBT and PLGA were determined by gel permeation chromatography (GPC). For PLGA, Shodex columns were used and $CHCl_3$ was the mobile phase. The columns were calibrated using polystyrene standards of known molecular weights.

Determination of α-tocopherol content of the microspheres

The PEGT/PBT materials hereinabove described also contain 0.5 % of a suitable antioxidant (α-tocopherol). The amount of antioxidant present after the preparation of the microspheres was determined using HPLC analysis.

Protein encapsulation efficiency

Bovine serum albumin (BSA) encapsulation efficiency was determined by dissolving an aliquot of the protein loaded microspheres (±25 mg) in $CH_2Cl_2$ (0.5 ml). This solution was extracted 5 to 10 times with water (0.5 ml). The protein concentration in the extracts was determined using the Biorad assay. From the extracted amount of protein, the encapsulation efficiency was calculated. As a control, the protein concentration of the polyvinyl alcohol and the washing solutions also were determined.

Encapsulation efficiency of diphtheria toxoid was determined by determining the amount of protein in the polyvinyl alcohol solution and the different washing solutions.

In vitro release of proteins from microspheres

A known weight of the microspheres (±100 mg) was placed into an Eppendorf tube and a 1 ml buffer (PBS containing 0.02% $NaN_3$) was added. The tubes were closed and rotated at 37°C. At selected time intervals the microspheres were centrifuged, the supernatant removed, the pellet resuspended in 1 ml buffer and centrifuged again. The supernatant was added to the first supernatant and the concentration of the protein in the pooled sample was determined using the Biorad protein assay.

Aging of the PEGT/PBT and the PLGA microspheres

PEGT/PBT and the PLGA microspheres were aged at 37°C and at 0% and 100% humidity, respectively. The molecular weights of the polymers were determined by GPC and were established as a function of the aging time.

Results

Microsphere preparation via the solvent evaporation technique

33 batches of PolyActive (PEGT/PBT or PA) and PLGA microspheres were prepared via the solvent evaporation technique. The characteristics of such batches of microspheres are given in Table I below.

Table I

| Characteristics of PA/PLGA microspheres prepared by solvent evaporation technique | | | | | | |
|---|---|---|---|---|---|---|
| batch no. | polymer; concentration % (w/v) | PVA (%) | Yield (%) | protein and encapsulation efficiency | Size (µm) | |
| | | | | | number wt. | volume wt. |
| 1 | PA 80/20; 5% | 1 | 65 | DT; 2% | 13 | 37 |
| 2 | PA 80/20; 10% | 1 | 83 | DT; 24% | 13 | 88 |
| 3 | PA 80/20; 20% | 1 | 78 | DT; 30% | 13 | 101 |
| 4 | PA 70/30; 5% | 1 | 77 | NA | 14 | 38 |
| 5 | PA 70/30; 5% | 5 | 81 | NA | 12 | 25 |
| 6 | PA 70/30; 5% | 10 | 82 | NA | 6 | 9 |
| 7 | PA 70/30; 10% | 1 | 77 | NA | 15 | 112 |
| 8 | PA 70/30; 10% | 5 | 85 | NA | 8 | 47 |
| 9 | PA 70/30; 10% | 5 | 88 | NA | 8 | 41 |
| 10 | PA 70/30; 10% | 5 | 85 | NA | 8 | 38 |
| 11 | PA 70/30; 20% | 1 | 80 | NA | 13 | 456 |
| 12 | PA 70/30; 20% | 5 | 82 | NA | 13 | 36 |
| 13 | PA 70/30; 20% | 10 | 74 | NA | 9 | 21 |
| 14 | PA 60/40; 5% | 1 | 78 | NA | 14 | 48 |

Table I (continued)

| Characteristics of PA/PLGA microspheres prepared by solvent evaporation technique | | | | | | |
|---|---|---|---|---|---|---|
| batch no. | polymer; concentration % (w/v) | PVA (%) | Yield (%) | protein and encapsulation efficiency | Size (μm) | |
| | | | | | number wt. | volume wt. |
| 15 | PA 60/40; 10% | 1 | 60 | NA | 19 | 144 |
| 16 | PA 70/30; 5% | 1 | 78 | BSA; 104% | 14 | 57 |
| 17 | PA 70/30; 10% | 1 | 78 | BSA; 115% | 9 | 65 |
| 18 | PA 70/30; 20% | 1 | 54 | BSA; 75% | 19 | 369 |
| 19 | PA 60/40; 5% | 1 | 80 | BSA; 93% | 11 | 43 |
| 20 | PA 60/40; 10% | 1 | 53 | BSA; 55% | 17 | 444 |
| 21 | PLGA 5% | 1 | 83 | BSA; 82% | 8 | 29 |
| 22 | PLGA 10% | 1 | 77 | BSA; 85% | 11 | 71 |
| 23 | PA 70/30; 5% | 5 | 84 | DT; 85% | 5 | 21 |
| 24 | PA 70/30; 10% | 5 | 72 | DT; 94% | 9 | 70 |
| 25 | PA 70/30; 20% | 5 | 66 | DT; 88% | 12 | 202 |
| 26 | PA 70/30; 20% | 5 | 51 | DT; 98% | 9 | 145 |
| 27 | PA 60/40; 5% | 5 | 88 | DT; 94% | 6 | 28 |
| 28 | PA 60/40; 10% | 5 | 64 | DT; 10% | 13 | 51 |
| 29 | PLGA 5% | 5 | 78 | DT; 98% | 7 | 16 |
| 30 | PLGA 10% | 5 | 84 | DT; 27% | 8 | 41 |
| 31 | PA 80/20; 5% (PEG 1000) | 1 | 62 | lys | 15 | 52 |
| 32 | PA 80/20; 5% (PEG 2000) | 1 | 70 | lys | 20 | 80 |
| 33. | PA 80/20; 5% (PEG 4000) | 1 | 77 | lys | 22 | 89 |

The microsphere yield (defined as the mass of the microspheres/mass of polymer used for the preparation of microspheres) was normally about 80%. Figure 1 gives a representative example of the particle size distribution of the obtained microspheres (Batch 6, PA 70/30, 5 % in $CH_2Cl_2$, 10% PVA) as determined using the Accusizer.

The obtained size distribution is rather normal for microspheres prepared via the solvent evaporation technique. The batch shown in Figure 1 has a number weight mean of 6.0 μm and a volume weight mean of 9.4 μm; 95% of the particles has a diameter between 5 and 17 μm.

The effect of the PEGT/PBT concentration and the PVA concentration on particle size distribution was investigated. Figure 2 shows the effect of the PVA concentration on the size (distribution) of the 70/30 PEGT/PBT microspheres. From this figure it can be seen that an increasing PVA concentration yielded smaller particles with a smaller distribution. A higher viscosity (due to higher PVA concentration) of the external phase will yield smaller droplets of emulsified polymer and finally, after evaporation of the solvent, smaller sized PEGT/PBT particles. Interestingly, a higher concentration of PVA also yielded a more narrow particle size distribution (ratio of volume weight average and number weight average becomes smaller at higher PVA concentration).

Whereas PVA has a substantial effect on the particle size, the effect of the 70/30 PEGT/PBT concentration on particle size and particle size distribution is less pronounced (Figure 3). In addition, no large effect of the stirring rate on the particle size was observed.

It can be concluded that the best way to control and tailor the particle size of PEGT/PBT microspheres is the PVA concentration of the external phase.

By GPC measurements it was shown that the molecular weight of PEGT/PBT of the obtained microspheres was not significantly different from the polymer used for the preparation of the microspheres. This demonstrates that during the preparation, washing, and drying, no degradation (hydrolysis) of the polymer occurs.

The amount of antioxidant in two different microsphere batches was determined (Batch 7 (PEGT/PBT 70/30) and Batch 14 (PEGT/PBT 60/40)). No decrease in α-tocopherol was observed, which can be ascribed to the very low solubility of this compound in water. The antioxidant might inhibit the oxidative degradation of PA during in vitro release studies with hydrolysis being the dominant degradation route.

Also prepared by the solvent evaporation technique described hereinabove were the following microspheres:

Microsphere A

(i) 89 wt. % of a mixture of 99 wt. % of a 50/50 PEGT/PBT copolymer, wherein the polyethylene glycol has a molecular weight of 300, and 1 wt. % of α-tocopherol;
(ii) 2 wt. % of 1-ascorbic acid 6-palmitate;
(iii) 4 wt. % of cholesterol; and
(iv) 5 wt. % of enkephalin peptide.

Microsphere B

(i) 85 wt. % of a mixture of 98.5 wt. % of a 70/30 PEGT/PBT copolymer, wherein the polyethylene glycol has a molecular weight of 2,000, and 1.5 wt. % of the antioxidant Trolox-NH-PEG 1,000-$OCH_3$
(ii) 5 wt. % of polyethylene glycol having a molecular weight of 2,000; and
(iii) 10 wt. % of insulin.

The synthesis of Trolox-NH-PEG 1,000-$OCH_3$ is described in Example 3.

Microsphere preparation via the spray drying technique

Preparation of the PLGA microspheres using the spray drying technique has been published in the literature (Gander, et al., Microencapsulation, Vol. 12, 83-97 (1995)). It was shown that PolyActive microspheres could also be prepared by the spray drying technique. In contrast to the solvent evaporation technique, only low concentrations of PEGT/PBT in $CH_2Cl_2$ (2%) could be used to prepare microspheres, because at higher concentrations PEGT/PBT fibers were formed. The characteristics of the PEGT/PBT and PLGA microspheres prepared via the spray drying technique are given in Table II.

Table II

| Characteristics of Polyactive (PA)/PLGA microspheres prepared by the spray drying technique | | | | | |
|---|---|---|---|---|---|
| Batch No. | polymer; concentration % (w/v) | Yield (%) | protein and encapsulation efficiency | Size (µm) | |
| | | | | number wght | volume wght |
| 34 | PLGA; 5% | 30 | BSA; 48% | 3 | 6 |
| 35 | PA 70/30; 2% | 53 | DT | 7 | 16 |
| 36 | PA 60/40; 2% | 40 | DT | 16 | 84 |
| 37 | PA 70/30; 2% | 58 | BSA | 11 | 83 |
| 38 | PA 70/30; 2% | 43 | IgG | 10 | 259 |
| 39 | PA 70/30; 2% | 42 | lysozyme | 17 | 136 |

The particle size distribution for PEGT/PBT microspheres prepared via the spray drying technique is substantially broader as compared with the spray dried PLGA microspheres. This is especially the case for the protein loaded PEGT/PBT microspheres.

Protein encapsulation efficiency

The encapsulation efficiency of BSA could be determined by dissolving an aliquot of the microspheres in a suitable solvent ($CH_2Cl_2$) followed by extraction with water. Using this procedure BSA was extracted almost quantitatively. This procedure was validated by extracting a solution of PLGA in $CH_2Cl_2$ containing a known amount of BSA.
It appears that the encapsulation efficiency in PEGT/PBT microspheres was high (50-100%, Table I) and hardly dependent on the processing parameters. The concentration of protein in the PVA phase and the different washing solutions also was determined in order to establish the mass balance. Table III summarizes the results.

Table III

| BSA encapsulation efficiency in PolyActive and PLGA microspheres | | | | | | | |
|---|---|---|---|---|---|---|---|
| Batch | Polymer | PVA | Wash I | Wash II | Wash III | ms* | rec.**(%) |
| 16 | PA 70/30; 5% | 8.2 | 0.3 | 0 | 0 | 70.3 | 92 |
| 17 | PA 70/30; 10% | 2.0 | 0 | 0 | 0 | 76.5 | 92 |
| 18 | PA 70/30; 20% | 2.4 | 0.1 | 0 | 0 | 35.0 | 44 |
| 19 | PA 60/40; 5% | 3.1 | 0 | 0 | 0 | 74.3 | 91 |
| 20 | PA 60/40; 10% | 2.8 | 0.1 | 0 | 0 | 21.8 | 29 |
| 21 | PLGA; 5% | 14.7 | 1.1 | 0.9 | 1.0 | 57.8 | 88 |
| 22 | PLGA; 10% | 0.4 | 0 | 0 | 0 | 55.7 | 65 |

* BSA content (mg) of the microspheres as determined by the extraction method
** recovery: BSA detected in the microspheres, PVA and washing solutions/BSA offered for encapsulation (=85mg)

From this table it clear that the mass balance (% recovery) is good, except for 2 formulations. The low recovery for Batch 18 and Batch 20 can probably be ascribed to the low recovery of the microspheres (see Table I).
From the literature it is known that for encapsulation efficiency of proteins in PLGA (and other matrices) is highly dependent on the stability of the water-in-oil emulsion. Obviously, this is ensured by using the Ultraturrax as hereinabove described. The encapsulation efficiency of diphtheria toxoid therefore was determined from the diphtheria toxoid concentrations in the PVA external phase and the different washing solutions. It was shown that the amount of the diphtheria toxin in these solutions was less than 15% (encapsulation efficiency greater than 85%), except for the PEGT/PBT 70/30 (10%) and the PLGA (10%) microsphere batches.

Scanning electron microscopy analysis

SEM was used to evaluate the shape and surface characteristics (porosity) of the microspheres. The following trends were found:
Microspheres prepared using the solvent evaporation technique are perfectly spherical. This is especially true for microspheres which were prepared using a high concentration of PolyActive in $CH_2Cl_2$. Microspheres prepared using the spray drying technique did not have perfectly round shape as observed for the solvent evaporation particles. In the protein loaded microspheres pores sometimes can be observed. These pores might contribute to and be related to the sometimes observed burst release of the proteins.

Release of BSA from PA and PLGA microspheres

The release of BSA from PA and PLGA microspheres prepared by the solvent evaporation technique and from the one batch (PLGA) prepared by the spray drying technique was evaluated. The release of BSA from the microspheres of PA 60/40, PA 70/30 and PLGA over a period of 150 days in given in Figures 4, 5, and 6. The results are summarized in Table IV below.

Table IV

| Release of BSA from PA and PLGA microspheres | | | | | |
|---|---|---|---|---|---|
| Microsphere | Particle Size (µm) | | BSA Loading (mg BSA/g ms) | burst* µg/g; % | release** µg/g; day |
| | Number | Volume | | | |
| PA 70/30; 5% | 14 | 57 | 66.9 | 158; 2.3 | 21.3 |
| PA 70/30; 10% | 9 | 65 | 37.4 | 2.2; 0.1 | 8.7 |
| PA 70/30; 20% | 19 | 369 | 12.1 | 40; 3.5 | 21.3 |
| PA 60/40; 5% | 11 | 43 | 60.5 | 240; 3.8 | 4.9 |
| PA 60/40; 10% | 17 | 444 | 17.8 | 177; 9.1 | 4.9 |
| PLGA; 5% | 8 | 29 | 52.6 | 3500; 65 | 0 |
| PLGA; 10% | 9 | 65 | 27.1 | 100; 3.6 | 0 |
| PLGA; SD | 3 | 6 | 18500 | 18500; 100 | 0 |

* arbitrarily taken as the amount of protein released during the first 5 hours.
** calculated from the amount of protein released from day 1-150.

From Figures 4, 5, and 6 and Table IV, it appears that the release of BSA from PEGT/PBT microspheres is associated with a very small burst effect (maximum 10%). After the burst release, a sustained release of BSA is observed. The slow (sustained) release of BSA from the PEGT/PBT 70/30 and 60/40 microspheres is probably due to the diffusion of BSA through the (homogenous) matrix. Moreover, this release was more pronounced for the more hydrophilic microspheres. (PEGT/PBT 70/30; 9-21 µg BSA/g ms.day) as compared with the more hydrophobic 60/40 microspheres (5 µg BSA/g ms.day), indicating the diffusivity of BSA is greater in the PEGT/PBT 70/30 matrix than in the 60/40 matrix. This can be attributed to the higher equilibrium water content of the PEGT/PBT 70/30 microspheres as compared with the PEGT/PBT 60/40 microspheres. It also is noted that during the release time (150 days) probably no substantial degradation of the matrix occurs. Again, in oxidative environments the degradation is faster than in PBS, which might have a pronounced effect on the in vivo release of the BSA from PEGT/PBT microspheres.

In contrast to the PEGT/PBT microspheres, 2 out of 3 PLGA microsphere preparations showed a pronounced burst release (Figure 6). Especially the spray dried (abbreviated: SD) microspheres and the microspheres prepared using a low PLGA concentration (5%) showed this high outburst release, which can be ascribed to the porous character of the particles. On the other hand, the PLGA microspheres prepared using high PLGA concentration showed a marginal burst release. In addition, this batch showed a pulsed release of encapsulated BSA between 60 and 80 days. The pulse corresponds with the degradation (dissolution) time of these microspheres. However, it is remarkable that the percentage of BSA release did not reach 100%. Part of the protein may be denatured (precipitated) due to the low pH generated in the degrading microspheres.

Release of diphtheria toxoid from PA and PLGA microspheres

Figure 7 shows a representative example of the release of diphtheria toxoid from PEGT/PBT 70/30 and 60/40 microspheres. The release was followed for a period of up to 10 weeks. In general, a burst release was observed followed by a continuous release from day 1 to day 20, after which a very low release of diphtheria toxoid was observed for the next 50 days. Table V summarizes the results.

Table V

| Release of diphtheria toxoid from PEGT/PBT microspheres | | | | | |
|---|---|---|---|---|---|
| microsphere | particle size (µm) | | Release (LF/g) | | |
| | number | volume | burst* | day 1-21 | day 21-70 |
| PEGT/PBT 70/30; 5% | 5 | 21 | 7.5 | 1.4 | .24 |
| PEGT/PBT 70/30; 10% | 9 | 70 | .9 | .9 | .26 |

* arbitrarily taken as the amount of protein released during the first day.

Table V (continued)

| Release of diphtheria toxoid from PEGT/PBT microspheres | | | | | |
|---|---|---|---|---|---|
| microsphere | particle size (μm) | | Release (LF/g) | | |
| | number | volume | burst* | day 1-21 | day 21-70 |
| PEGT/PBT 70/30; 20% | 12 | 202 | 1.6 | .22 | .09 |
| PEGT/PBT 70/30; 20% | 9 | 145 | .5 | .15 | .03 |
| PEGT/PBT 70/30; SD | 7 | 16 | 123 | 1.9 | .46 |
| PEGT/PBT 60/40; 5% | 6 | 28 | 1.3 | .25 | .11 |
| PEGT/PBT 60/40; 10% | 13 | 51 | 1.8 | .55 | .07 |
| PEGT/PBT 60/40; SD | 16 | 84 | 88 | 5.1 | 3.0 |
| PLGA; 5% | 7 | 16 | 24 | 2.4 | .61 |
| PLGA; 10% | 8 | 41 | 2.0 | .15 | .043 |

* arbitrarily taken as the amount of protein released during the first day.

Taken into account that around 200 LF diphtheria toxoid per gram microsphere was encapsulated, it appears that the burst release of diphtheria toxoid from PEGT/PBT microspheres prepared by the solvent evaporation technique is relatively low. After the burst release, the PEGT/PBT particles showed a gradual release up to 70 days after the start of the release experiment.

In contrast to microspheres prepared by the solvent evaporation technique, the particles prepared by spray drying technique showed a very significant burst release (around 50%) which can probably be ascribed to the porous character of particles prepared via this technique. Like the "solvent evaporation" particles, the spray dried particles showed a gradual release after the burst release. The release rate (from day 1-21 and 21-70) of diphtheria toxoid was higher from the spray dried particles than for the "solvent evaporation" particles. Obviously, the release (after the burst) from the "solvent evaporation" particles is determined by the diffusion of diphtheria toxoid through the homogeneous polymer matrix, whereas the release from the "spray dried" particles is also determined by the diffusion of the protein through the pores present in the matrix. In contrast to PLGA microspheres which contained BSA (Figure 6), for the PLGA microspheres containing diphtheria toxoid, no pulsed release is observed between day 60 to 80 (dissolution time of the PLGA microspheres). This may be caused by the low stability of diphtheria toxoid at the low pH generated in the degrading matrix.

The release of diphtheria toxoid from PEGT/PBT (80/20) microspheres (prepared by solvent evaporation technique) was followed for a period of almost 600 days (Figure 8).

The microspheres prepared from a 5% PEGT/PBT 80/20 solution in $CH_2Cl_2$ showed a substantial burst release of around 50-60 % of the encapsulated diphtheria toxoid. Thereafter, a gradual release of the protein occurred within the following 20 days. The 10% PEGT/PBT microspheres showed a (smaller) burst release of around 10-20% of the encapsulated amount of protein, followed by a gradual release up to 200 days and by an additional release starting around 400 days and ending after 550 days. The 20% PEGT/PBT 80/20 microspheres showed a marginal burst release (1-2%), followed by a gradual release up to 400 days after which the remaining encapsulated diphtheria toxoid is released in a pulse. For the 5% PEGT/PBT 80/20 microspheres no pulse after 400 days was observed. This can be ascribed to the rather porous character of the microspheres and/or to the low encapsulation efficiency of diphtheria toxoid in these particles.

The observed release profiles can be explained as follows. The burst release is due to protein present at or near the surface of the microspheres and the protein present in pores. An increasing concentration of PEGT/PBT (5-20%) resulted in microspheres with a less porous character and consequently a reduced in a reduced burst release. The gradual release followed after the burst release is due to diffusion of the protein through the (non-porous) matrix. The release starting around 400 days can be ascribed to degradation of the matrix. The degradation was associated with increased swelling of the microspheres (visual observation).

The observed release profile of diphtheria toxoid from PA 80/20 microspheres is very interesting for the design of new vaccine formulations. It can be envisaged that by mixing diphtheria toxoid containing microspheres with a varying degradation (dissolution) time, diphtheria toxoid (antigens in general) can be released in multiple pulses which is considered necessary for obtaining a protective immune response.

When it is assumed that after 600 days all encapsulated diphtheria toxoid has been released, an estimation can be made on the DT encapsulation efficiency (Table VI).

Again it appears that the encapsulation efficiency is low when a diluted solution of PA (5%, w/v) is used for the encapsulation process.

Table VI

| Encapsulation efficiency of diphtheria toxoid in PolyActive microspheres | | | | |
|---|---|---|---|---|
| Batch | DT (in LF) released in 600 days | sample size (mg) | yield (mg) | DT encapsulated LF; %* |
| PA 80/20; 5% | 2.4 | 102 | 740 | 17; 2% |
| PA 80/20; 10% | 11.9 | 104 | 1700 | 195; 24% |
| PA 80/20; 20% | 8.0 | 107 | 3200 | 239; 30% |

* 810 LF were offered for encapsulation

Release of lysozyme from PEGT/PBT microspheres

The effect of the molecular weight of the PEG block of PolyActive was evaluated by studying the release of protein with a relatively low molecular weight (lysozyme, M-14.3 kD) from PA 80/20 microspheres with a varying PEG molecular weight (1,000, 2,000, and 4,000 g/mol). It is expected that a molecule of a higher weight of the PEG block will result in larger hydrophilic PEG domains in the PEGT/PBT matrix, which will facilitate the diffusion of the encapsulated protein. The initial release (up to 17 days) has been measured. Figure 9 shows the release of lysozyme from PA 80/20 microspheres.

A burst release of lysozyme from PEGT/PBT microspheres is observed followed by a more gradual release. Because the encapsulated amount of protein in the different microspheres is not known, the observed burst release cannot be expressed in % of the core loading. When it is assumed that the encapsulation efficiency was 100%, the burst release is about 10, 20, and 40 % for the PEGT/PBT 80/20 (1000), PEGT/PBT 80/20 (2000), and PEGT/PBT 80/20 (4000), respectively. The real burst release will be greater because the encapsulation efficiency will be less than assumed. This means that lysozyme shows a pronounced burst release from the 80/20 microspheres. This is probably caused by the low concentration of PEGT/PBT used (5%) for the preparation of the particles which provides a porous structure. After the burst release, all PEGT/PBT 80/20 particles showed a gradual release. This is shown in detail for one microsphere preparation in Figures 10A (release versus time) and 10B (release versus square root of time).

Figure 10B shows that the cumulative release (after the burst release) was proportional to the square root of time, which is indicative for a diffusion controlled release. The other microsphere also showed a square root of time dependency of the release (results not shown).

Aging of PA and PLGA microspheres

PEGT/PBT and PLGA microspheres were aged at 37°C and at 100 and 0 % relative humidity, respectively. Aging was followed by measuring the molecular weight of the polymer. Figure 11 shows the molecular weight of PA and PLGA as a function of the incubation (aging) time.

It appears that PLGA is degraded rapidly in a humid atmosphere. Within 8 weeks the particles were completely degraded. This is even faster than the degradation time of the same particles in a buffer solution of pH 7.2. (degradation time 10-12 weeks). The fast degradation of PLGA at 100% humidity can be explained as follows. Initially, the (dry) PLGA microspheres absorb some water. This results in hydrolysis of ester bonds, yielding carboxylic acid residues. These acidic groups catalyze a further hydrolysis of the polyester backbone, finally resulting in water soluble oligomers and monomers. In buffered solutions part of the formed acid residues are neutralized and/or diffused out of the microparticles resulting in a retarded degradation as compared with particles aged in a humid environment. On the other hand, both microspheres of PEGT/PBT 60/40 and PEGT/PBT 70/30 were stable at 100% humidity (no significant change in molecular weight over a period of 20 weeks). Figure 11 shows the results for PEGT/PBT 70/30. Comparable results were obtained for PEGT/PBT 60/40. This again demonstrates that PEGT/PBT degrades more slowly than PLGA in a humid environment. Further, it was shown that PEGT/PBT microspheres (Batch 12 70/30) absorbed water under the aging conditions. The equilibrium water content (Karl Fischer titration performed by Dr. J. Goedemoed) amounted to 16 and 25% (w/w) after 1 and 2 weeks aging respectively. Obviously the water present in the microspheres did not cause any significant hydrolysis (degradation) of the polymer. In a non-humid environment, both PLGA and PA were stable: no drop in molecular weight was observed.

**EXAMPLE 2**

Six polyethylene glycol terephthalate/polybutylene terephthalate (PEGT/PBT) copolymers were synthesized. In three of the copolymers, the polyethylene glycol had a molecular weight of 1,000. (PEG 1,000). These three copolymers had weight percentage ratios of PEGT/PBT of 40/60, 60/40, and 80/20. In the other three copolymers, the polyethylene glycol had a molecular weight of 600. (PEG 600). These three copolymers had weight percentage ratios of PEGT/PBT of 40/60, 60/40, and 77/23. Each of the six copolymers contains 0.4 wt.% of the antioxidant 1,3,5-trimethyl-2,4,6-tris(3,5-di-tert-butyl-4-hydroxybenzyl) benzene sold by Ciba-Geigy as Irganox 1330.

Disks were then made of each copolymer. Each disk included indomethacin in an amount of 5 wt.%. Indomethacin is 1-(p-chlorobenzoyl)-5-methoxy-2-methyl-3-indole-3-acetic acid, has a molecular weight of 357.8. Disks which included PEGT/PBT copolymer wherein the PEGT/PBT weight ratio was 40/60, and the molecular weight of polyethylene glycol in the polymer was 1,000, were made by forming a solution of 6.34 g PEGT/PBT, 73.36 g of chloroform, and 5 g of hexafluoroisopropanol (HFIP). This solution then was mixed with a solution of 0.333 g of indomethacin, 10 g of chloroform, and 50 µl HFIP in a Petri dish having a diameter of 9 cm. Prior to mixing, the polymer was allowed to dissolve overnight. A small amount of HFIP increases the dissolving rate of the drug considerably. Disks having a diameter of 5 mm then were made by punching the resulting film formed by the combination of the solution with a cork borer. Also, disks were made from small quantities of polymer/drug solution which was not poured into the Petri dish.

For the polymers in which the polyethylene glycol has a molecular weight of 1,000 and which have a PEGT/PBT weight ratio of 60/40 and 80/20, and for the polymers having a polyethylene glycol molecular weight of 600, a solution of 3.5 g of polymer and 35 g of chloroform, and a solution of 0.184 g of indomethacin, 5.53 g of chloroform, and 50 µl of HFIP were combined in a Petri dish having a diameter of 5 cm.

The PEG 600 40/60 copolymer showed an extensive shrink; the resulting layer possessed a thickness such that one 5 mm disk had a weight of 90-99 mg. For all other polymers, it was necessary to combine 3 to 5 disk layers.

*Flow-through elution*

Release studies were performed by use of flow cells. The flow cells were filled with tiny glass beads. 9 cells possessed an internal diameter of 22.6 mm and 9 other cells 12.0 mm. Because of the very low flow rate (1 ml/hr), it is very likely that cell dimension is not very critical in the release process.

The 18 flow cells were supplied with eluant with 2 Harvard Infusion syringe pumps (type Pump 22, microprocessor controlled). Each pump has been modified in such a way that 9 syringes of 100 ml can be placed on a holder. The extended plungers of the syringes were pushed continually in a horizontal direction by a moving bar.
Eluant composition: phosphate buffer 0.05 M, pH 7.4, isotonic

| | |
|---|---|
| $NaH_2PO_4.1H_2O$ | 3.65 g |
| $Na_2HPO_4$ (Anhydrous) | 5.7 g |
| NaCl | 9.2 g |
| $NaN_3$ | 0.5 g |
| purified water | 2.0 1 |

Samples were taken after 2, 4, 8 and 14 hours, and after 1, 2, 3, 7.25 and 8.25 days. During 30 min an Eppendorf cup was filled with 0.5 ml. Hereafter the cups were closed and frozen in liquid nitrogen.

*Analysis of indomethacin*

The liquid chromatographic system consisted of a Pharmacia analytical pump (Type 2248) with solvent conditioner, a Pharmacia variable wavelength monitor, a Pharmacia high sensitivity flow cell, a Marathon autosampler with 20 µl sample loop and a Pharmacia HPLC computing system with a Tandon 1 MB Ram computer with Star LC-20 printer.

A Zorbax RP8 Stable Bond (Rockland Technologies) column (150x4.6 mm) packed with 5 µm spherical particles was used. The wavelength was set at 254 nm. The mobile phase consisted of acetonitrile/1.3% v/v acetic acid in water 65/35. The flow rate was 1.0 ml/min. All separations were affected isocratically at ambient temperature.

Standards of indomethacin have been prepared, using the same flow through eluant, of 100, 50, 20, 10, 5, 2 and 1 µg/ml. Shortly after preparation the standards have been dispensed in 1.5 ml polypropylene Eppendorf cups and frozen in liquid nitrogen, in order to limit degradation phenomena prior to analysis. Samples from the flow-through system have been injected directly in the chromatographic system after melting.

Assay of indomethacin in PEGT/PBT disks

The dried disks were cut into tiny pieces, accurately weighed and dissolved in chloroform (40/60 in 50 ml, 60/40 and 80/20 in 25 ml) overnight. Standards have been prepared by dissolving indomethacin in chloroform and appropriately diluting to 50, 20, 10, 5 and 2 μg/ml.

Results

Preparation of devices

The indomethacin containing PEGT/PBT devices were yellow colored and had an opaque appearance. The evaporation process occurred over a period of 3 to 5 days. This slow rate was necessary for obtaining films with regular thickness. After 8.25 days elution, the cylindrical shape of all 18 devices were the original disk-layers separated.

After 8.25 days elution and evaporating/drying at 85°C, the 40/60 devices showed a tough constituence, the 60/40 devices a moderate, and the 80/20 devices a relatively soft constituence.

*Water uptake data*

Water uptake data for the disks with respect to flow-through elution at 8.25 days are given in Table VII. As a control, water uptake data for injection molded cylinders also are provided.

Table VII

| Water uptake of Polyactive devices after 8.25 days flow-through elution | | | |
|---|---|---|---|
| Polymer | weight* before start (mg) | % water uptake* after 8.25 days (w/w) | % water uptake** injection molded cylinder (w/w) |
| PEG 1000 40/60 | 107 | 7 | 15 (1 wk) |
| PEG 1000 60/40 | 113 | 43 | 37 (1 wk) |
| PEG 1000 80/20 | 97 | 78 | 75 (24 hrs) |
| PEG 600 40/60 | 93 | 14 | 18 (24 hrs) |
| PEG 600 60/40 | 102 | 21 | 25 (24 hrs) |
| PEG 600 77/23 | 129 | 23 | 42 (24 hrs) |

* average of 3 cylindrical devices containing 5% indomethacin

** cylinders d=10x20 mm

For PEG 600 77/23, considerably less water-uptake has been observed, possibly due to the extra hydrophobic influence of 5% w/w indomethacin. Also, the other two PEG 600 formulations had somewhat lower uptake percentages. In the PEG 1000 series, the 40/60 polymer showed a water uptake of half of that of the injection molded reference.

Flow-through release results

Indomethacin release profiles from disks made from each of the 6 PEGT/PBT polymers are shown in Figure 12. The ratio between the most rapid and the slowest indomethacin release is less than 2. After 8.25 days, 3.68 mg of indomethacin was released from the disks formed from the PEG 1000 80/20 polymer, and 2.05 mg of indomethacin was released from the disks formed from the 600 40/60 polymers.

*Indomethacin analysis*

With the applied chromatographic method, indomethacin showed a retention time of 3.3 min., with no interfering peaks in its proximity. After 8.25 days elution, a peak at 2.6 min slightly and a complex of peaks around 2 min. increases considerably. The chromatogram of an indomethacin standard in phosphate buffer (2 μg/ml) shows that at least one peak of the complex originates from indomethacin itself.

In the chromatogram resulting from the indomethacin assay of the PolyActive device remnants, an extra peak at a retention time of 2.8 min can be seen, which reflects the antioxidant Irganox 1330 or one or several oxidized forms of this compound.

The above analyses of indomethacin have been taken into consideration when determining the weight loss of polymer after 8.25 days elution. Weight losses for disks formed from each of the six polymers are given in Table VIII below.

Table VIII

| polymer | weight before start (mg) | weight w/out indomethacin | weight after drying | % polymer weight loss |
|---|---|---|---|---|
| PEG 1000 40/60 | 107 | 102 | 80 | 22 |
| PEG 1000 60/40 | 113 | 108 | 105 | 3 |
| PEG 1000 80/20 | 97 | 92 | 87 | 5 |
| PEG 600 40/60 | 93 | 90 | 85 | 6 |
| PEG 600 60/40 | 102 | 98 | 93 | 5 |
| PEG 600 77/23 | 129 | 123 | 107 | 13 |

Preparation of indomethacin-containing microspheres

Also prepared by the solvent evaporation technique hereinabove described in Example 1 were the following microspheres containing indomethacin.

Microsphere C

(i) 89 wt. % of a mixture of 99 wt. % of a 50/50 PEGT/PBT copolymer, wherein the polyethylene glycol has a molecular weight of 300, and 1 wt. % of α-tocopherol;
(ii) 1 wt. % of 1-ascorbic acid 6-palmitate;
(iii) 5 wt. % of cholic acid; and
(iv) 5 wt. % of indomethacin.

Microsphere D

(i) 94 wt. % of a mixture of 99 wt. % of a 50/50 PEGT/PBT copolymer, wherein the polyethylene glycol has a molecular weight of 300, and 1 wt. % α-tocopherol;
(ii) 1 wt. % 1-ascorbic acid 6-palmitate; and
(iii) 5 wt. % indomethacin.

Microsphere E

(i) 95 wt. % of a mixture of 99.5 wt. % of a 50/50 PEGT/PBT copolymer, wherein the polyethylene glycol has a molecular weight of 300, and 0.5 wt. % α-tocopherol; and
(ii) 5 wt. % indomethacin.

Microsphere F

Same as Microsphere E, except that the polyethylene glycol of the PEGT/PBT copolymer has a molecular weight of 400.

Microsphere G

Same as Microsphere E, except that the PEGT/PBT ratio of the PEGT/PBT copolymer is 55/45.

**EXAMPLE 3**

The polymeric materials used in this example were (i) a PEGT/PBT copolymer having a PEGT/PBT ratio of 70/30 and wherein the polyethylene glycol had a molecular weight of 1,000, and wherein such material included 0.5 wt.% of the antioxidant d,l $\alpha$-tocopherol; and (ii) a PEGT/PBT copolymer having a PEGT/PBT ratio of 70/30, and a polyethylene glycol molecular weight of 1,000, wherein such material also included the antioxidant Trolox-NH-PEG 1,000 -$OCH_3$. The Trolox-derived antioxidant was synthesized by dissolving 5.00 g of Trolox, which is 6-hydroxy-2,5,7,8-tetramethyl-chroman-2-carboxylic acid, in a mixture of 100 ml dioxane and 100 ml dichloromethane and 5 ml (1.5 eq) triethylamine at room temperature. The clear solution was cooled to -15°C, and 2.01 ml (19.5 mmole) of ethyl chloroformate in 20 ml dichloromethane was added dropwise and the resulting suspension was stirred for 10 minutes at -15°C. A solution of 10.0 g (10 mmole) of $(CH_2\text{-}CH_2\text{-}O)_{21}\text{-}NH_2$ in 60 ml dioxane (with 1 ml triethylamine) was added and the cooling bath was removed. The reaction mixture was allowed to warm to room temperature in about 1 hour. The solvent was removed *in vacuo* and the residue was dissolved in 100 ml of 1 M HCl solution and extracted twice with 50 ml of ethyl acetate. The aqueous layer was neutralized to pH 12 with solid $K_2CO_3$ and extracted three times with 50 ml of ethyl acetate. (The product forms an intermediate layer. This layer was combined with the ethyl acetate layer.) 50 ml of 2-propanol then was added to the combined organic layers, and the layers then were dried with $Na_2SO_4$ and concentrated *in vacuo* to give 10.61 g (86%) of crude product.

The crude product was purified by chromatography (Silicagel 60, with methanol as eluant). The ninhydrin-negative fractions with an Rf value of 0.3-0.1 were combined and concentrated *in vacuo* to give an almost colorless oil (8.5 g, 69% yield), which solidified on standing. Several batches of the Trolox-PEG 1,000 derivative were prepared. In this example, 14.5 g of the Trolox-PEG 1,000 derivative are employed, whereby the Trolox-PEG 1,000 derivative is present in the matrix in an amount of 1.45 wt. %.

The particles of each polymer were melt fused to form porous cylindrical rods having a diameter of 12 mm and a length of 60 mm.

For both types of porous rods, 12x60 mm, partially gamma-irradiated in its usual packaging, disks of approximately 0.4 g were prepared using a cutting device. The preparations then were weighed and placed in 20.0 ml of testing medium.

Testing media employed in this example were as follows:

1. Nanopure water (having an electrical conductivity below 1 nano-Siemens per cm)
2. Physiological saline (0.9% NaCl)
3. Phosphate buffer 20 mM, pH 7.4
4. Phosphate buffer 50 mM, pH 7.4
5. Phosphate buffer 0.5 M, pH 7.4
6. Physiological saline with $H_2O_2$ (1mM)
7. Nanopure water with Fe (100 $\mu$M)/$H_2O_2$ (1 mM) to generate hydroxyl radicals
8. Physiological saline with Fe (100 $\mu$M)/$H_2O_2$ (1mM) to generate hydroxyl radicals.
9. Phosphate buffer, 20 mM, with Fe (100$\mu$M), EDTA 100 $\mu$M/$H_2O_2$ (1mM) to generate hydroxyl radicals, pH 7.4

In order to examine the influence of gamma-radiation on the non-irradiated PEGT 1,000/PBT 70/30 rods, the rods were incubated in the following media:

10. Physiological saline (0.9% NaCl)
11. Physiological saline with Fe (100 $\mu$M)/$H_2O_2$ (1mM) to generate hydroxyl radicals.

Also, gamma-irradiated and non-irradiated including the Trolox antioxidant were incubated in the following media:

12. Gamma-radiation and physiological saline (0.9% NaCl)
13. Gamma-radiation and physiological saline (0.9% NaCl) with Fe (100 $\mu$M/$H_2O_2$ (1mM)
14. No radiation, physiological saline (0.9% NaCl)
15. No radiation, physiological saline (0.9% NaCl) with Fe (100 $\mu$M)/$H_2O_2$ (1mM)

During the testing of these polymers, the following conditions also were employed.

Mass/Volume ratio: 1:50. 400 mg of polymer was added to the liquid phase of 20 ml.
Bottle: 40 ml cylindrical flasks, closed with plastic screw caps (chemical grade glassware).
Temperature: The incubation temperature was 37°C ± 0.5°C.

Pressure: Atmospheric Pressure.
Testing Periods: Polymer samples were tested at 0, 1, 2, 4, 8, 12, 26, 39 and 52 weeks.
Medium change: All test media were changed once a week.
Agitation: No agitation.
Handling prior to testing: No drying procedure to constant weight of the test specimens was performed in order to prevent antioxidant content alterations.
Handling after testing: At the end of the test period the device remnants were dried in an oven at 45°C for 3 hours and weighed. It was expected that with decreasing PEGT-content of partially degraded devices the water content is a minor issue (<< 1% w/w) after 3 hours drying at 45°C. Special care was taken during the change of the media at the stage of device fragmentation (approximately when $M_w$ has reached values below 40,000).
All test period resulting solutions were stored in the freezer below 0°C.
Number tests: All tests were performed in triplicate.

A summary of media and number of specimens tested in each medium is given in Table IX below.

Table IX

| | 70/30 rod gamma-irradiated | | 70/30 rod non-irradiated | |
|---|---|---|---|---|
| | medium no. | test spec. no. | medium no. | test spec. no. |
| water | 1. | 24 | | |
| 0.9% NaCl | 2. | 24 | 10. | 24 |
| 20mM phosphate | 3. | 24 | | |
| 50mM phosphate | 4. | 24 | | |
| 0.5M phosphate | 5. | 24 | | |
| 0.9% NaCl/1mM H2O2 | 6. | 24 | | |
| 100 µM Fe/1mM H2O2 | 7. | 24 | | |
| 0.9% NaCl/100µM Fe/ 1mM H2O2 | 8. | 24 | 11 | 24 |
| 20 mM phosphate/ 100µM Fe/1mM H2O2/100µM EDTA | 9. | 24 | | |
| total no. test spec./ bottles | | 216 | | 48 |
| | 70/30 rod gamma-irradiated Trolox-der. (sec. batch) | | 70/30 rod non-irradiated Trolox-der. (sec. batch) | |
| | medium no. | test spec. no. | medium no. | test spec. no. |
| 0.9% NaCl | 12. | 24 | 14. | 24 |
| 0.9% NaCl/100 µM Fe/ 1µM H2O2 | 13. | 24 | 15. | 24 |
| total no. test specimens/ bottles | | 48 | | 48 |

The following analyses were conducted on each of the polymer samples.
The intact devices were weighed after the drying procedure hereinabove described. When a device became fragmented, the tarrated glass bottle, after careful removal of the testing medium, was weighed as a whole after the same drying process.
pH measurements were performed in one of the triplicate test media just after the weekly medium change (accuracy within 0.01 pH unit).
GPC analysis was performed on all samples. Calibration was conducted with 12 standards ranging from 580

Daltons to 2,000,000 Daltons.

HPLC analysis of a α-tocopherol was performed until no more α-tocopherol could be measured from device remnants. The method was conducted by refluxing the device remnants with methanol, followed by HPLC reverse phase analysis using a Zorbax SB C8 column and 100% acetonitrile as an eluant.

HPLC analysis of aqueous solutions of terephthalic acid and its derivatives was conducted by HPLC reverse phase analysis using a Zorbax SB CN column with 30% acetonitrile/70% diluted acetic acid, 1.3% in water as the eluant.

UV spectrophotometric analyses for formic acid and acetic acid were conducted by applying enzymatic test combinations (formic acid: Boehringer Mannheim No. 979732; acetic acid: Boehringer Mannheim No. 148261). Testing solutions which have been shown to be acidified based on measured pH values are selected.

Results

Analysis of Trolox antioxidant

Chromatographic analyses determined the purity of the Trolox derivative and indicated that no coupling occurred via the phenolic hydroxy group. HPLC detected free Trolox and its derivative. A free Trolox content in the derivative was determined to be about 1.2%. HPLC analysis of free $H_2N$-PEG 1,000-$OCH_3$, the other starting material, employing pre-column derivitization with F-moc-Cl, showed a free $H_2N$-PEG 1,000-$OCH_3$ content of about 0.35 wt.%. GPC analysis showed a monodisperse peak.

The following data in Table X below are given for two PEGT/PBT 70/30 batches synthesized on a 1 kg scale. Each polymer also includes α-tocopherol or Trolox derived antioxidant. The batch including the α-tocopherol antioxidant gave a synthesis yield of 870g, and the batch including the Trolox-derived antioxidant gave a synthesis yield of 835 g. Molecular weight data were obtained by GPC using polystyrenes as calibrants, and % PEGT was calculated using H-NMR data.

Table X

| | $M_w$ | $M_w/M_n$ | % PEGT |
|---|---|---|---|
| α-tocopherol yield: 870 g | 107,222 | 2.144 | 70.1% |
| Trolox-derivative yield: 835 g | 103,285 | 2.065 | 71.1% |
| Mw - Weight average molecular weight<br>Mn - Number average molecular weight | | | |

Before testing, several processing steps were conducted, including chopping of the granulate, sieving, drying, melt fusing, packaging, and in some cases, gamma-radiation.

In Table XI below, molecular weight data are given which represent three stages for each batch: (i) after synthesis as granulate; (ii) as a non-irradiated porous rod; and (iii) as a gamma-radiated porous rod.

Table XI

| **α-tocopherol batch** | **Trolox derivative batch** |
|---|---|
| granulate-Mw=107,222<br>non-irradiated porous rod Mw=107,127<br>gamma-radiated porous rod Mw=102,294 | granulate-Mw=103,285<br>non-irradiated porous rod Mw=104,162<br>gamma-radiated porous rod Mw=97,665 |

The presented data for both batches are comparable; it shows that with both antioxidants completely comparable copolymer materials, with respect to molecular weight and PEGT/PBT ratio, can be obtained. Also, the Trolox antioxidant effectively protects the developing copolymer material during the polycondensation process a temperature of up to 245°C. The Trolox antioxidant has a higher boiling point than α-tocopherol; therefore, it is only necessary to add such antioxidant at the start of the synthesis in contrast to the situation with α-tocopherol.

**TGA-analysis of PolyActive granulate with both types of antioxidants**

Also, the Trolox antioxidant protects the PolyActive 70/30 material effectively. Thermogravimetric analysis (TGA) is an appropriate method for detecting weight losses of the material during exposure to thermal stress.

Weight loss could only be monitored for both formulations after a severe heat-pretreatment of the material for 17 days in a heat chamber at 100°C. The necessity of this pretreatment already indicates the stability and resistance

to thermal influences of the new formulation. In Figures 13A and 13B it can be seen that after this pretreatment the formulation with the Trolox antioxidant degrades faster than the one with α-tocopherol under these conditions. The shown resistance of the new formulation however, to the applied thermal stress suggests a satisfactory shelf-life behavior under normal conditions.

**In vitro degradation results of 70/30 rods containing α-tocopherol**

*** gamma-radiated rods**

In Figures 14A and 14B molecular weight $M_w$ - time profiles are depicted for the testing media numbers 2 through 9. (The profile for medium no. 1 is almost identical with that of medium no. 2 and for reasons of clarity is not depicted.)

It can be seen that there is almost no $M_w$ decrease in the water and 0.9% NaCl media. Up to 4 weeks $M_w$ -decreasing rates in the phosphate buffered media and the $Fe/H_2O_2$ media are comparable. After 4 weeks the degrading influence of the $Fe/H_2O_2$ media on the 70/30 α-tocopherol rods is far more pronounced than that of the phosphate buffered media.

In the phosphate buffered media (nos. 3,4,5), after 8 weeks (including 7 medium changes), still more than half of the α-tocopherol quantity of timepoint zero is present in the device. In medium no. 5, half of the initial quantity is present after 26 weeks. In the oxidative $Fe/H_2O_2$ media (nos. 7,8) α-tocopherol has disappeared (i.e., reacted).

Thus, comparable $M_w$ decreases can be established with and without the consumption of α-tocopherol, for instance nos. 3,4,5,7,8,9 after 2 or 4 weeks.

Regarding the 3 phosphate buffers, it can be observed that the higher the phosphate concentration, the more α-tocopherol will remain; this suggests a specific "phosphate effect," such as iron-phosphate complexation or OH radical scavenging ability, rather than a nonspecific pH-effect of the buffer on the $Fe/H_2O_2$ system.

The more pronounced influence of the $Fe/H_2O_2$ media after 4 weeks compared with the phosphate buffered media could be explained as follows. For oxidative chain-scission the concentration of available PEG remains relatively constant as is the case in media nos. 7 and 8. An almost linear $M_w$-decrease in the time can be observed. In contrast, the concentration available, hydrolysis-sensitive ester-bonds diminishes (nos. 3,4,5). All phosphate medium $M_w$-time curves level off.

The stabilizing influence of the phosphate buffers on the oxidative degradation of the PEGT/PBT material appears by comparison of the results from media nos. 3 and 9. The 20 mM phosphate buffer in medium no. 9 is able to prevent completely the extensive oxidative degradation ($M_w$-decrease) as occurred in medium no. 7. The profile of media nos. 3 and 9 are almost identical.

**Non-irradiated rods**

In Figures 15 and 16, the $M_w$-time profiles of gamma-irradiated and non-irradiated α-tocopherol containing rods are depicted, in the 0.9% $NaCl/Fe/H_2O_2$ medium and 0.9% NaCl medium, respectively.

Although gamma-radiation decreases the α-tocopherol content of 70/30 rods from 0.78% w/w to 0.51% w/w, the radiation does not influence the $M_w$ degradation behavior. $M_w$ decreasing profiles are almost identical.

**In vitro degradation results of 70/30 rods containing the Trolox-derivative antioxidant.**

**gamma-radiated rods**

The degradation rate of the rods containing this type of antioxidant proceeds faster in the 0.9% $NaCl/Fe/H_2O_2$ medium than the rates of α-tocopherol rods (see Figure 15).

In the 0.9% NaCl medium the gamma-irradiated rods with the hydrophilic antioxidant starts to degrade after 4 weeks, resulting in a 20% $M_w$-decrease after 8 weeks, 31% after 12 weeks, and 42% after 26 weeks.

**Non-irradiated rods**

In the 0.9% NaCl medium there is only slight degradation (no. 14) - a 16% $M_w$ decrease after 26 weeks.

The difference in $M_w$ degradation between both types of antioxidant formulations is extremely interesting for protein delivery applications. Larger proteins (e.g. vaccines) are restrained in the polymer matrix by their molecular size. Assuming that a release only starts at a (chain) degradation level corresponding with an $M_w$ of about 40,000, then it follows that in the 0.9% $NaCl/Fe/H_2O_2$ medium the "Trolox-derivative" matrix starts to release 4 weeks earlier than the "α-tocopherol" matrix. Considering the rigid oxidative stress of this medium, it is quite likely that the *in vivo*

release starting times deviate more than 4 weeks. Another indication for a large difference in release times *in vivo* between the PEGT/PBT copolymers containing the different antioxidants is the large difference in $M_w$ loss between the two gamma-radiated matrices after 26 weeks. There was an 8% $M_w$ loss for the matrix containing α-tocopherol versus a 42% $M_w$ loss for the matrix including the Trolox derivative. Knowing these extremes, intermediate release starting times may be obtained by varying the ratio of the Trolox-derivative and α-tocopherol *within the same matrix*. By combining various matrices, for instance in the form of microspheres, with varying protein starting times, pulsatile release systems can be developed.

Mass loss

In Figure 14B both % original mass and $M_w$-time profiles are depicted for 5 selected media showing varying degradation rates.

The small percentage losses (ca. 0.5 - 1.5%) in the water and 0.9% NaCl media are caused by dissolution of the water-soluble PEG-butylene terephthalate (PEGT-BT) oligomers during the first week. Hereafter the weight remained fairly constant. Small variations are caused by deviations on the drying procedure or in humidity conditions.

This oligomer, with a chemical structure comprising $(BT)_x$ units, wherein x=1-20, with 1 or 2 pending PEG 1,000 chains, is the chemically non-incorporated oligomer from the copolymer-synthesis; through the hydrophilic PEG 1,000 and hydrophobic $(BT)_x$ moieties, it has the characteristics of a surfactant. Shaking of the solutions results in foaming.

In case of the Trolox-derivative rods mass losses in 0.9% NaCl are slightly larger than those of the α-tocopherol rods (nos. 12 and 14 versus nos. 2 and 10). This can be explained by loss of the water-soluble antioxidant itself (concentration approx. between 0.8 and 1.2 % w/w)

Considering this, it is reasonable to assume that the percentages of remaining PEG-BT oligomer after synthesis using both types of antioxidant are comparable. This is confirmed by the observation of comparable peak surface areas of the PEG-BT oligomer resulting from HPLC terephthalate analysis.

It is expected that trends in mass loss are slower than those in $M_w$-decrease (Figure 14B). Initially, only chain-scission occurs, at a later stage loss of degradation products proceeds. Some examples of varying $M_w$-decreases and mass losses can be found in the Table XII below.

It can be seen in this table and in Figure 14B, that mass losses for the Trolox-derivative rods in the 0.9% NaCl/Fe/$H_2O_2$ medium are considerably larger than those of the α-tocopherol rods (nos. 8 and 11 versus nos. 13 and 15).

Table XII

| | | **8 weeks** | | **12 weeks** | | **26 weeks** | |
|---|---|---|---|---|---|---|---|
| type of antioxidant | medium | % mass loss | % Mw loss | % mass loss | % Mw loss | % mass loss | % Mw loss |
| α-tocopherol | no. 3 | 4.8 | 40.5 | 6.5 | 46.6 | 11.6 | 40.5 |
| α-tocopherol | no.7 | 9.4 | 90.4 | 27.7 | 88.1 | 58.1 | 96.1 |
| α-tocopherol | no.8 | 7.5 | 68.8 | 18.7 | 85.8 | 47.7 | 95.8 |
| α-tocopherol | no.11 | 7.9 | 72.5 | 18.6 | 89.8 | 50.7 | 96.1 |
| Trolox | no.13 | 17.8 | 93.4 | 39.8 | 91.8 | 62.7 | 96.1 |
| Trolox | no.15 | 17.4 | 94.4 | 35.4 | 92.7 | 60.1 | 96.2 |

**$^1$H-NMR measurements**

By determining $^1$H-NMR changes with respect to the PEGT/PBT ratio, changes within the PEGT segment can be monitored. Only at the starting time can the PEGT segment be calculated because it can be assumed at that time that the PEG chain has a molecular weight of 1000 Daltons. As soon as chain scission occurs such calculations are not possible due to varying PEG molecular weights. Instead of % PEGT calculations, characteristic signal ratios will be used which can show a decrease in the PEG-segment length:

signal a = inner butylene protons (O-$CH_2$-C**H**$_2$-C**H**$_2$-$CH_2$-O)

signal c = inner oxyethylene protons $(CH_2\text{-}O\text{-}C\mathbf{H}_2\text{-}C\mathbf{H}_2\text{-}O\text{-}CH_2)$
signal c' = oxyethylene protons $(CO\text{-}O\text{-}CH_2\text{-}C\mathbf{H}_2\text{-}O\text{-}CH_2)$
signal c" = oxyethylene protons $(CO\text{-}O\text{-}C\mathbf{H}_2\text{-}CH_2\text{-}O\text{-}CH_2)$

Ratio a/c reflects the ratio between the available quantity of the inner butylene protons and that of the inner oxethylene protons, and, therefore, is the measure for the ratio between the PEGT and PBT segment.

Using 70% PEGT, Mw PEGT 1130, 30% PBT, Mw PBT 220: a/c is 0.1084
PEG: 1 + 20.3 + 1 oxyethylene units

Ratio (c'+c')/c reflects the ratio between the available quantity of the outer and inner oxyethylene units. In the case of progressing chain scission, this ratio will increase. Using (1+20.3+1) oxyethylene units for PEG, (c"+c')/c is 0.0985.
In Tables XIII and XIV below, these ratios are given after analysis of the device remnants for no. 4 (50 mM phosphate), no. 8 ($NaCl/Fe/H_2O_2$), and no. 13 ($NaCl/Fe/H_2O_2$ hydrophilic antioxidant) after 0, 4, 8, and 12 weeks.
It can be seen that the fast degrading device no. 13 shows the largest a/c ratios. The largest difference with no. 8 ($\alpha$-tocopherol devices) is observed after 8 weeks; after 12 weeks the difference is smaller.
Regarding the (c"+c')/c ratio, no. 13 also reveals the largest values; however, there is a slight decrease after 8 weeks.
Possibly some measurement errors are made due to the fact that only dissolved copolymer remnant is measured by NMR; the higher the PBT content, the more difficult the dissolution behavior will be in the deuterated chloroform.
The general trend is clear, however, that the faster the degradation of a device is taking place is taking place (Mw, mass loss), the higher the resulting PBT-content present in the device remnant will be.

Table XIII

| a/c ratios of proton NMR signals | | | |
|---|---|---|---|
| | no. 4 | no. 8 | no. 13 |
| 0 weeks | 0.106 | 0.106 | 0.103<br>0.103 |
| 4 weeks | 0.110 | 0.114 | 0.117<br>0.124<br>0.119 |
| 8 weeks | 0.114<br>0.113<br>0.113 | 0.113<br>0.126<br>0.125 | 0.162<br>0.162<br>0.167<br>0.167 |
| 12 weeks | 0.117<br>0.117 | 0.161<br>0.163 | 0.179<br>0.179 |

Table XIV

| (c"+c')/c ratios of proton NMR signals | | | |
|---|---|---|---|
| | no. 4 | no. 8 | no. 13 |
| 0 weeks | 0.098 | 0.098 | 0.095<br>0.095 |

Table XIV (continued)

| (c"+c')/c ratios of proton NMR signals | | | |
|---|---|---|---|
| | no. 4 | no. 8 | no. 13 |
| 4 weeks | 0.097 | 0.105 | 0.104<br>0.104<br>0.102 |
| 8 weeks | 0.105<br>0.099<br>0.096 | 0.105<br>0.097<br>0.109 | 0.148<br>0.149<br>0.138<br>0.135 |
| 12 weeks | 0.097<br>0.94 | 0.119<br>0.110 | 0.128<br>0.127 |

**Formation of acid degradation products**

In the weak-acid oxidative (NaCl)/Fe/$H_2O_2$ media, an additional acidificatino occurs. The start pH value of medium no. 7, 3.47, is within 5 weeks decreased to the fluctuating range of 2.77-3.13 (with one sample of medium no. 7 having a pH value of 3.35). The start pH value of medium no. 8, 3.65, is within 5 weeks decreased to 3.04-3.25, and for medium no. 13, about the same reduction is observed.

This acidification can only be explained by the formation of acids like formic acid and acetic acid (known oxidation products arising from PEG), rather than terephthalic acid, considering the fact that terephthalic acid (or a mono-functional derivative) is not able to establish pH values below 4 due to its high $pK_a$ value.

To confirm this, concentrations of formic acid and acetic acid in selected degradation solutions were measured by applying specific, enzymatic UV-tests commercially available for these carboxylic acids.

In Table XV below, concentrations of formic acid in µg/ml in selected solutions of media nos. 7, 8 and 11 are listed, as measured by a UV enzymatic test combination.

Table XV

| | **medium no. 7** | **medium no. 8** | **medium no. 11** |
|---|---|---|---|
| week 2 | 37.51 | 9.29 | 10.40 |
| week 4 | 61.28 | 16.34 | 29.71 |
| week 6 | 78.37 | 30.08 | 34.17 |
| week 9 | 139.65 | 53.85 | 66.11 |
| week 12 | 137.05 | 81.71 | 97.31 |
| week 15 | 98.05 | 71.65 | 80.97 |

Table XVI

| | **medium no. 7** | **medium no. 8** | **medium no. 11** |
|---|---|---|---|
| week 2 | 0.31 | 0.08 | 0.07 |
| week 4 | 0.50 | 0.13 | 0.23 |
| week 6 | 0.61 | 0.23 | 0.26 |
| week 9 | 1.08 | 0.43 | 0.46 |
| week 12 | 1.09 | 0.66 | 0.68 |
| week 15 | 0.73 | 0.53 | 0.60 |

In Table XVI, these concentrations of formic acid are used to calculate the mole percent formation of formic acid from the available oxyethylene units, assuming 1 molecule of formic acid originates from 1 oxyethylene unit.

In addition, cumulative percentages during the period of 15 weeks are given, as shown in Table XVII below.

For the most rapidly degrading medium, no. 7, this cumulative percentage, for 15 weeks, was about 11% (See Table XVII below). This predicts a long period during which this formic acid production will be continued. The resulting low pH value was measured up to 26 weeks.

Table XVII

| Cumulative Molar Percentages of Formic Acid from Available Oxyethylene Units | | | |
|---|---|---|---|
| | **no. 7** | **no. 8** | **no. 11** |
| cumul. % week 0-15 | 11.08 | 5.24 | 5.86 |

In Figure 17, the formic acid formation percentages for nos. 7, 8, 11 and 13 are depicted. It can be seen that in medium no. 7, $Fe/H_2O_2$ without NaCl, after 6 weeks, higher percentages of formic acid are built up in comparison with the other media. Again, there is no influence of gamma-irradiation (no. 8 versus no. 11).

During 0-6 weeks the formic acid concentrations produced in no. 13 (hydrophilic antioxidant) are somewhat higher than those in no. 8 (α-tocopherol); however, the cumulative percentages after 15 weeks are comparable.

Apparently the faster degradation rate observed in no. 13, as reflected in $M_w$-decrease and mass loss, is not accompanied by a faster oxidative breakdown of PEG-units resulting in an increased acid production.

Table XVIII

| | **start pH** | **week 5** | **week 10** | **week 15** | **week 20** | **week 26** |
|---|---|---|---|---|---|---|
| α-toc, rods | | | | | | |
| no. 8 | 3.65 | 3.25 | 3.14 | 3.14 | 2.75 | 3.07 |
| no. 11 | 3.65 | 3.26 | 3.15 | 3.13 | 3.03 | 3.08 |
| Trolox-derived rods | | | | | | |
| no. 13 | 3.47 | 3.09 | 3.13 | 3.19 | 3.05 | 3.15 |
| no. 15 | 3.47 | 3.06 | 3.14 | 3.20 | 3.06 | 3.17 |

With the hydrophilic antioxidant, about the same pH values were found in the $NaCl/Fe/H_2O_2$ medium. After 26 weeks even slightly higher pH values were found, as shown in Table XVIII.

In Table XIX, concentrations of acetic acid (μg/ml) in selected solutions of media nos. 7, 8 and 11 are given.

On a molar basis the measured concentrations of acetic acid are 20-100x lower than the formic acid concentrations. The concentrations are measured by a UV enzymatic test combination.

Table XIX

| | **medium no. 7** | **medium no. 8** | **medium no. 11** |
|---|---|---|---|
| week 2 | 0.03 | 0.72 | 0.09 |
| week 4 | 0.40 | 0.99 | 0.38 |
| week 6 | 0.72 | 0.75 | 0.50 |
| week 9 | 1.14 | 2.00 | 1.47 |
| week 12 | 1.01 | 1.80 | 1.88 |
| week 15 | 1.25 | 1.83 | 1.75 |
| week 20 | 1.25 | 1.29 | 1.80 |

In Figure 18, concentrations of acetic acid for media nos. 7, 8, 11, 13 and 15 are presented.

It can be concluded that the observed acidification in the strong oxidative media $(NaCl)/Fe/H_2O_2$ can be mainly ascribed to the formation of formic acid.

Acidification was only observed in the rigid oxidative $(NaCl)/Fe/H_2O_2$ environment. In all other media no acidification occurred (most likely) or was dominated by present buffer systems.

It should be stated, however, that this acidification is not to be expected *in vivo*. A "bulk-oxidation" as is the case in the rigid $(NaCl)/Fe/H_2O_2$ medium will not occur; *in vivo* the generation of reactive OH readicals takes only place in especially adapted structures within the cell of phagocytosing macrophages.

Therefore, fragmentation and particle size reduction of the copolymer material, and phagocytosis (a surface mediated process) are rate limiting steps *in vivo*. By this spreading in space and time of the degradation process the (reactive) aldehyde precursors of formic acid and acetic acid are effectively metabolized by enzyme systems from the cell. Most likely this will prevent any arising of free acids in the cytoplasm.

**HPLC terephthalate analyses**

**70/30 rods containing α-tocopherol (nos. 1-11)**

In water and 0.9% NaCl (nos. 1, 2, 10) a characteristic pattern of peaks can be observed:

| | Retention time |
|---|---|
| terephthalic acid | 2.3 min. |
| "dimeric" terephthalate | 3 min. |
| PEG-BT oligomer | 4-8 min. |
| unknown peak | 12-14 min. |

After one week all these compounds largely have been released. The chromatograms resulting from Week 2 solutions are almost empty.

The phosphate media (nos. 3, 4, 5, 9) and (NaCl)/Fe/$H_2O_2$ media (nos. 7, 8, 11) have other characteristic patterns. There are extra peaks and assemblies of peaks are present at the shorter retention times (immediately after terephthalic acid). The surface area of the PEGT-BT oligomer is usually smaller than that resulting from the water media, suggesting a partial degradation of the PEGT-BT oligomer into more hydrophilic (incorporation of oxygen) or into lower molecular weight products both resulting in shorter retention times.

Gamma-radiation does not influence any of the peak patterns. The chromatogram resulting from the water and 0.9% NaCl solutions remain almost empty during 2-12 weeks; no degradation is taking place.

The peak assembly areas in the chromatogram resulting from the phosphate- and oxidative media diminish in size during 2 and 4 weeks. However, from Week 6 the surface area of the "oxidative" peak pattern increases again.

**70/30 rods containing the Trolox-derivative antioxidant**

After Week 1, the peak patterns in the chromatogram from the Trolox-derivative rods in the 0.9% NaCl media (nos. 12, 14) are almost identical with those from the α-tocopherol rods (nos. 2, 10)

Again, no influence of gamma-radiation could be observed.

The chromatograms showed that in synthesis using both α-tocopherol as well as the Trolox-derivative, the portion of the non-incorporated PEG-BT oligomer is formed in the same degree. This indicated that the type of antioxidant does not influence the polymerization process.

Also, the peak patterns resulting from the phosphate and (NaCl)/Fe/$H_2O_2$ media are almost identical after Week 1.

From Week 2 the surface area of the "oxidative" peak patterns increases. From Week 4 the enlarged surface area of the peak assembly remains constant regarding shape and magnitude. The surface area of the "oxidative" peak pattern of the Trolox-derivative rods (nos. 13, 15) are always considerably larger than those of the corresponding α-tocopherol rods (nos. 8, 11).

Comparing the terephthalate chromatograms in time resulting from the α-tocopherol and the Trolox-derivative rods, i.e., nos. 8 versus nos. 13 and nos. 11 versus nos. 15, it was found that only quantitative differences exist. The released terephthalates should explain at least part, and possibly the largest part, of the difference in mass loss between both types of antioxidant formulations.

Considering the almost identical pH values, it may be concluded that the more extensive degradation with the hydrophilic antioxidant is caused predominantly by more extensive PEG-chain scissions, resulting in higher concentrations of terephthalate derivatives, lower $M_w$ values and larger mass losses. These increased PEG-chain scissions, however, are not accompanied by increased oxidative consumption of oxyethylene units leading to acid products.

**EXAMPLE 4:** CONTINUOUS RELEASE OF PROTEINS FROM PEGT/PBT FILMS

*Materials*

In this example, poly(ethylene oxide)terephthalate/poly(1,4-butylene)terephthalate (PEGT/PBT) copolymers were synthesized, wherein such copolymers had PEGT/PBT weight ratios of 80/20, 77/23, 70/30, 60/40 and 40/60. The polyethylene glycol component of the polymers having PEGT/PBT weight ratios of 70/30, 60/40 and 40/60 had a molecular weight of 1,000. For the copolymer having a PEGT/PBT weight ratio of 77/23, the molecular weight of the polyethylene glycol component was 600 and for the polymer having a PEGT/PBT weight ratio of 80/20 the molecular weight of the polyethylene glycol component was 4,000. Such copolymers are indicated as aPEGbPBTc, in which a is the PEG molecular weight, **b** the wt% PEG-terephthalate and c the wt% PBT.

Phosphate Buffered Saline (PBS), pH 7.4 was purchased from NPBI (Emmer-compascuum, NL). Bovine serum albumin (BSA), Immunoglobulin G (IgG), Lysozyme from chicken egg white (3x crystallized, dialysed and lyophilized) and lyophilized cells of *Micrococcus Lysodeikticus* were purchased from Sigma Chem Corp. (St. Louis, USA). Chloroform ($CHCl_3$) and methanol, obtained from Merck (Darmstadt, DE) were of analytical grade.

*Preparation of (lysozyme loaded) PEGT/PBT films*

A lysozyme solution (0.6 ml, 55 mg/ml) in PBS (pH 7.4) was emulsified in a polymer solution in chloroform (7 ml, 9 wt% PEGT/PBT) using ultraturrax mixing (30 s at 20.5 krpm). The resulting water-in-oil emulsion was cast onto a glass plate using a 0.75 mm casting knife. After slow evaporation of the chloroform, films with a thickness of about 50 µm were stripped from the glass plate and stored under desiccation at 4°C. Unloaded copolymer films were cast from 9 wt% solutions in chloroform using the same conditions. To study the influence of the copolymer composition, different polymers were used, with the PBT content varying from 20 to 60 wt% and the PEG molecular weight of 600, 1000 and 4000. To evaluate the effect of polymer molecular weight on the protein release rate, 1000PEG70PBT30 powder (2 g) was degraded in PBS (25 ml) at 37°C and used as matrix material for the protein loaded films. The incubation time was 3, 11 or 21 days and buffer was refreshed weekly. After incubation, buffer was removed, polymers were washed with methanol and dried for 2 days in a vacuum oven at 50°C. Polymer molecular weight was estimated from figure 21.

*Preparation of (BSA and IgG loaded) PEGT/PBT films*

To prepare BSA loaded films, 0.5 - 2.5 ml of an aqueous BSA solution (55 mg/ml PBS) was emulsified in a polymer solution in chloroform (7 ml, 9 wt% 1000PEG70PBT30) using ultraturrax-mixing (30 s at 20.5 krpm), cast onto a glass plate and further processed as described for lysozyme loaded films. To prepare IgG loaded films, 0.6 or 2.5 ml of an aqueous IgG solution (25 mg/ml PBS) was emulsified in a polymer solution in chloroform (7 ml, 9 wt% 1000PEG70PBT30) using ultraturrax-mixing (30 s at 20.5 krpm), cast onto a glass plate and further processed as described for lysozyme loaded films.

*Scanning Electron Microscopy (SEM)*

The internal structure of the lysozyme loaded matrices was evaluated using scanning electron microscopy. Dry films were freeze-fractured in liquid nitrogen and mounted on a substrate holder. The surface was sputter-coated with a thin gold layer and studied using a Hitachi S-800 field emission SEM.

*water uptake*

The swelling behaviour of the films was evaluated by periodically measuring the weight of films after blotting the surface with a tissue. The water content was calculated by:

$$\text{water uptake (\%)} = 100 \times (W_1 - W_0)/W_0. \qquad (1)$$

where $W_0$ and $W_1$ are the weights of the dried and the hydrated films, respectively.

*Polymer degradation*

To determine degradation of polymer matrices, dry films were weighted and incubated in PBS at 37°C in a shaking bath. After certain time intervals, samples were taken and weighted after drying. The weight loss was calculated by:

$$\text{weight loss (\%)} = 100 \times (W_0 - W_1)/W_0. \qquad (2)$$

where $W_0$ and $W_1$ are the weights of the films before and after incubation, respectively. The change in molecular weight of the polymers was determined using Gel Permeation Chromatography (GPC). Samples were eluted in chloroform containing hexafluoroisopropanol and acetonitrile through a Waters Styragel Guard precolumn and 2 x 5 µm Mixed-C 30 cm columns. Flow rate was 1 mL/min and a UV detector set at 245 nm was used. Column temperature was 35½C. Sample concentration was 0.03%. The molecular weight was determined relative to polystyrene standards and a PEGT/PBT standard.

*Protein release*

Protein loaded films (50 µm x 2.25 $cm^2$) were incubated in 1.5 ml PBS (pH 7.4). Vials were shaken at 37°C and samples were taken at various time points. Protein content was determined using a standard Coomassie Blue assay (Pierce). Buffer was refreshed after sampling. The activity of released lysozyme was evaluated using a *Micrococcus lysodeikticus* assay. Briefly, 150 µl of the lysozyme samples or standards (0-25 µg/ml) were added to a 96-wells microplate. A suspension of *M. lysodeikticus* cells (100 µl, 2.3 mg/ml) was added and the decrease in turbidity at 450 nm was measured at 15 seconds intervals for 4 minutes at 37°C. Results were expressed as the initial kinetic rate (slope of the OD vs. time at t=0). The lysozyme activity for the corresponding initial rate of a sample was calculated from a standard curve of the log concentration vs. initial rate.

*Results*

A variety of thin (about 50 µm) protein loaded PEGT/PBT matrices were prepared. Scanning electron microscopy showed that dried matrices had a dense internal structure, irrespective of the type of copolymer or the protein content of the matrices. Fully hydrated protein loaded matrices turned opaque, indicating that pores were formed upon swelling. However, these pores could not be detected using SEM, since the structure of hydrated films was not retained during freeze drying.

The swelling behaviour of the matrices was strongly dependent on the composition of the copolymers. Water-uptake reached equilibrium within three days and decreased with increasing PBT wt% of the copolymer or decreasing molecular weight of the PEG component (figure 19). As a result of protein incorporation, water uptake increased. The kinetics of swelling of protein containing matrices was comparable to unloaded matrices. Water uptake was fast during the first hours, and reached equilibrium after three days (data not shown).

Besides the swelling, polymer degradation may influence release characteristics. To study the polymer degradation, films were incubated in PBS at 37°C. The weight loss of copolymer films is shown in figure 20. Matrices containing increasing PEG weight percentages exhibited a faster weight loss during incubation time. Fragmentation of 1000PEG70PBT30 films was observed, whereas 1000PEG60PBT40 and 1000PEG40PBT60 films remained intact. However, from figure 20 it is obvious that weight loss of the PEGT/PBT films was small, and will not contribute significantly to the release of incorporated molecules. The weight average molecular weight decreased about 50% over the time period of 54 days. A plot of the reciprocal number average molecular weight, $M_n$, as a function of incubation time in PBS is a straight line (figure 21), indicating that *in vitro* degradation is caused by a non-catalysed hydrolysis. However, after a certain time period a plateau value of the molecular weight is reached.

Release of lysozyme from five different PEGT/PBT copolymers was studied. For all copolymer compositions, no initial protein burst was observed, indicating that proteins are effectively entrapped within the core of the films. The release characteristics of lysozyme from PEGT/PBT films was strongly dependent on the composition of the copolymers. Lysozyme was released with an almost constant rate (zero-order release) from films prepared from copolymers 1000PEG70PBT30, 1000PEG60PBT40, 1000PEG40PBT60 and 600PEG77PBT23 (figure 22A). The ability to sustain a constant release rate is an often desirable feature of a controlled release device, but difficult to achieve with a matrix type device. For release systems based PEGT/PBT copolymers the constant release rate is obtained by a precise balance of the degradation rate of the polymer and the diffusion coeffient of the protein through the matrix. Polymer degradation will result in an increase of the diffusion coefficient, which may compensate for the reduced drug concentration in the matrix, to yield an almost constant release rate.

The release rate could be controlled by the composition of the copolymers. Release rates of 1.5 up to 7 %/day were obtained for these particular polymers. An increase of the PBT weight percentage, or a decrease in the molecular weight of the PEG segments, resulted in a decrease of the release rate. This decrease can be explained by the difference in degree of swelling of the various copolymers. The higher the equilibrium water content of the films, the greater the lysozyme diffusivity will be.

Release of lysozyme from 4000PEG80PBT20, a copolymer having a high equilibrium water content (220%) was fast as compared to release from the other matrices (figure 22B). Release was completed within two hours, and the amount of released lysozyme was proportional to the square root of time, which indicates that the release follows Fickian diffusion instead of a zero-order profile. This can be attributed to the fact that diffusion of lysozyme through 4000PEG80PBT20 is fast. For short release times no effect of polymer degradation on the release can be expected.

Another way to control the release rate is to vary the molecular weight of the PEGT/PBT polymers. A decrease of the molecular weight results in an increase of the release rate (figure 23), which can be attributed to a reduction in resistance against diffusion with decreasing molecular weight.

An important characteristic of a protein or peptide release system is the stability of the proteins. Protein aggregation may occur during formulation, storage and release periods. For example, there is a considerable concern using polylactides and copolymers as matrices for the delivery of proteins. Degradation of polylactides and copolymers generates a pH drop in the polymer matrices, which may cause protein instability problems. It may be expected that PEGT/PBT copolymers will cause less protein stability problems as compared to the polylactide-based systems, since PEGT/PBT degradation is slower and generates less acid end-groups. To investigate protein stability problems with respect to PEGT/PBT systems, the enzymatic activity of released lysozyme was determined (figure 24). No decrease in activity was found, indicating that protein was not damaged during the formulation, storage and release periods.

Protein release from 1000PEG70PBT30 films depended on the molecular weight of the proteins (figure 25): at low loading levels (3.3 wt% protein) release of high molecular weight proteins (BSA, 67 kD; IgG, 160 kD) was less than 10%, whereas lysozyme (14.4 kD) release was 100% after 20 days. This can be attributed to the low diffusivity of the HMW proteins. The rate of diffusion can be increased by increasing the porosity of a matrix. The porosity of the protein loaded PEGT/PBT films is dependent on the amount of non-solvent (the protein solution) which is mixed with the polymer solution during the preparation of the water-in-oil emulsion. An increase in the fraction non-solvent (the water/polymer ratio) will cause an increase in porosity. In figure 26 the influence of the water/polymer ratio of the initial water-in-oil emulsion on the water uptake of BSA loaded matrices is shown. It was found that the water uptake increased with increasing water/polymer ratio, indicating that matrices with increasing porosity were produced. The release characteristics of BSA and IgG from 1000PEG70PBT30 films were strongly dependent on the water/polymer ratio of the initial water-in-oil emulsion and thus on the porosity of the films (figure 27 and 10). An increasing porosity caused a increase in the release rate. Films prepared from emulsions with a high water/polymer ratio provided a burst release, whereas release of BSA from films with a lower water/polymer ratio was almost constant for at least 40 days.

*Conclusion*

Thin (about 50 µm) polyetherester films, loaded with different proteins were successfully prepared using a water-in-oil technique. The release rate of proteins from PEGT/PBT films could be manipulated by several parameters. Release rates decreased with decreasing PEGT/PBT ratio and decreasing molecular weight of the PEG component. Furthermore, an increase in molecular weight caused a decrease in release rate. Release of lysozyme followed zero-order kinetics, without a burst release of protein. By changing the porosity of the films, PEGT/PBT polymers could be applied as matrix for the release of HMW proteins such as BSA and IgG. Finally, determination of the activity of lysozyme showed that the proteins were not damaged during the formulation, storage and release periods.

**EXAMPLE 5:** Continuous release of proteins from PEGT/PBT microspheres

*Materials*

In this example, poly(ethylene oxide)terephthalate/poly(1,4-butylene)terephthalate (PEGT/PBT) copolymers were synthesized, wherein such copolymers had PEGT/PBT weight ratios of 77/23, 70/30, 60/40, 55/45 and 40/60. The polyethylene glycol component of the polymers having PEGT/PBT weight ratios of 70/30, 60/40 and 40/60 had a molecular weight of 1,000. For the copolymers having a PEGT/PBT weight ratio of 77/23 and 55/45, the molecular weight of the polyethylene glycol component was 600. Such copolymers are indicated as **a**PEG**b**PBT**c**, in which **a** is the PEG molecular weight, **b** the wt% PEG-terephthalate and **c** the wt% PBT.

Phosphate Buffered Saline (PBS), pH 7.4 was purchased from NPBI (Emmer-compascuum, NL). Polyvinyl alcohol (PVA, $M_w$ = 22,000) was obtained from ICN Biomedical Inc., Lysozyme from chicken egg white (3x crystallized,

dialysed and lyophilized) was purchased from Sigma Chem Corp. (St. Louis, USA). 1M 2,3,6-trinitrobenzenesulphonic acid (TNBS) from Fluka Chemika, $NaHCO_3$, 1M NaOH, HCl (37%), Chloroform ($CHCl_3$) and methanol (MeOH), obtained from Merck (Darmstadt, DE) were of analytical grade.

*Preparation of protein loaded PEGT/PBT microspheres*

A lysozyme solution (0.6 ml, 55 mg/ml) in PBS (pH 7.4) was emulsified in a solution of 1 g PEGT/PBT in chloroform using ultraturrax mixing (30 s at 20.5 krpm). The resulting water-in-oil emulsion was slowly added to 50 ml PBS containing 4 wt% PVA ($M_w$=22000) and after 5 minutes stirring at 400 rpm 200 ml PBS was added. This was kept at a constant stirring rate of 800 rpm for 2 hours. The microcapsules were collected by filtration, washed with PBS and lyophilized. To prepare microspheres of different sizes, the concentration of the polymer solution was varied from 0.14 to 0.048 g/ml. The viscosity of the polymer solutions was determined using a Ubbelohde (Canon 55, L117) at 20°C. To study the influence of the copolymer composition, different polymers were used, with the PBT content varying from 30 to 60 wt% and the PEG molecular weight of 600 and 1000. Microspheres were prepared from 0.6 ml lysozyme solution (55 mg/ml PBS, pH 7.4) and a solution of 1 g PEGT/PBT in 7 ml chloroform as described above.

*Determination of lysozyme loading*

Microcapsules (20-30 mg) or 50 µl of a lysozyme standard solution (0-1 mg/ml) was incubated with 1 ml 6M HCl for 24 hours at 37°C. Thereafter 6 ml 1M NaOH was added and the suspension was incubated for another 24 hours at 37°C. The protein concentration in the solution was determined using a TNBS assay. In short, 50 µl of the samples were added to a 96-wells microplate, whereafter 125 µl of a 4% (w/v) $NaHCO_3$ (pH=9) and 50µl of a 0.5% (w/v) TNBS solution was added to each well. After 2 hours incubation at room temperature, the extinction at 450 nm was measured using a SLT 340 AC microelisa reader. The extinction was related to the lysozyme concentration using the lysozyme standards.

*Determination of microsphere size*

The size of (swollen) microspheres (number weight and volume weight) was determined using a Microtrac (X100, Leeds & Northrup).

*Scanning Electron Microscopy (SEM)*

A Hitachi S-800 field emission SEM was used to evaluate the surface characteristics of the microspheres. The internal structure was observed by fracturing the microspheres in liquid nitrogen.

*Protein release*

Protein loaded microspheres (15 mg) were incubated in 1.5 ml PBS (pH 7.4) and shaken at 37°C. At various time points the suspension was centrifuged and samples of the supernatant were taken. Protein content was determined using a standard Coomassie Blue assay (Pierce).
Buffer was refreshed after sampling.

*Results*

PEGT/PBT microspheres containing lysozyme were prepared using a water -in-oil-in-water emulsion method. The microsphere yield (mass of the microspheres/mass of polymer used for preparation) was about 85%. SEM showed that the microspheres were perfectly spherical. The internal of the spheres was dense, with some small pores (about 100 nm), except for 1000PEG40PBT60 microspheres, which were hollow and very porous. The characteristics of the different batches are given in table XX and table XXI.

Table XX:

| Characteristics of lysozyme loaded 600PEG77PBT23 microspheres prepared from polymer solutions of different concentrations | | | | |
|---|---|---|---|---|
| Polymer concentration (g/ml) | reduced viscosity polymer solution (ml/mg) | $d_n$ (μm) | $d_v$ (μm) | encapsulation efficiency |
| 0.143 | 0.3827 | 24 | 108 | 86 ± 2 |
| 0.111 | 0.3161 | 41 | 81 | 67 ± 1 |
| 0.091 | 0.2737 | 31 | 63 | 77 ± 4 |
| 0.071 | 0.2327 | 26 | 50 | 71 ± 8 |
| 0.048 | 0.1828 | 15 | 29 | 75 ± 10 |

The influence of the polymer concentration on the size of the microspheres was investigated. Table XX shows that an decreasing polymer concentration resulted in smaller particles and a smaller size distribution. It is known that the size of emulsion droplets depends on a number of variables, given in formula (3):

$$d \propto K\,\frac{D_v R \nu_d \gamma}{D_s N \nu_c C_v} \qquad (3)$$

in which $D_v$ = diameter beaker, $D_s$ = diameter stirrer, R = volume ratio dispersed phase/continuous phase, $\nu_d$ = viscosity dispersed phase, $\nu_c$ = viscosity continuous phase, N = stirring rate, $\gamma$ = surface tension between dispersed and continuous phase and $C_s$ = concentration of the stabiliser. This formula predicts a linear relationship between the microsphere size and the viscosity of the polymer solution, which was confirmed by figure 29.

Table XXI:

| characteristics of lysozyme loaded PEGT/PBT microspheres prepared from polymers of different copolymer composition (concentration of the polymer solution is 0.143 g/ml chloroform). | | | |
|---|---|---|---|
| Polymer | $d_n$ (μm) | $d_v$ (μm) | encapsulation efficiency |
| 1000PEG40PBT60 | 48 | 215 | 56 ± 5 |
| 1000PEG60PBT40 | 38 | 128 | 103 ± 13 |
| 1000PEG70PBT30 | 34 | 150 | 64 ± 5 |
| 600PEG77PBT23 | 24 | 109 | 86 ± 2 |
| 600PEG55PBT45 | 64 | 153 | 102 ± 5 |

The encapsulation efficiency of lysozyme in PEGT/PBT microspheres varied from about 60 up to 100%. In general, the amount of encapsulated protein decreased with increasing hydrophilicity of the polymers and with decreasing size. The may be the result of release of lysozyme during the two hours solvent evaporation in PBS. This release will increase with increasing equilibrium water content (greater diffusivity) and greater surface area of the spheres. The low encapsulation efficiency of 1000PEG40PBT60 can be explained by the porous structure of these microspheres.

Release of lysozyme was studied as a function of the size of the microspheres and the composition of the copolymers. Figure 30 shows that lysozyme release from 600PEG77PBT23 microspheres prepared from solutions with decreasing polymer concentration resulted in an increase in the release rate. As explained above, a decreasing polymer concentration caused a decreasing microsphere size, and thus a higher surface area per volume, which results in a faster diffusion. Figure 31 shows that the release rate (%/day) of the first 50% of released lysozyme is proportional to the reciprocal sphere-diameter, which is consistent with the literature.

Lysozyme was released with an almost constant rate (zero-order release) from 600PEG77PBT23 microspheres, without an initial burst release of proteins. As discussed in example 4, the constant release rate is most likely the result of a precise balance between the rate of diffusion of the protein through the matrix and the rate of degradation of the polymers.

Another parameter which can be used to manipulate release rates is the copolymer composition. Figure 32 shows the release from microspheres of five different copolymer compositions. No initial protein burst was observed, indicating that proteins are effectively entrapped within the microspheres. Furthermore, release rates were almost

constant, except for 1000PEG40PBT60. In general, an increase of the PBT weight percentage, or a decrease in the molecular weight of the PEG segments, resulted in a decrease of the release rate. As explained in example 4, this decrease can be explained by the difference in degree of swelling of the various copolymers. The release 1000PEG40PBT60 was faster than expected, which can be explained by the porous structure of these microspheres.

*Conclusion*

Polyetherester microspheres, containing lysozyme, were successfully prepared using a water-in-oil-in-water emulsion technique. The encapsulation efficiency was high (60 - 100%). The size of the PEGT/PBT microspheres could be tailored by the concentration of the polymer solution which was used in the emulsion procedure. Release of lysozyme from the polyetherester spheres followed zero-order kinetics, without a burst release of protein, and could be manipulated by the copolymer composition and the microsphere size.

**EXAMPLE 6:** Prediction of protein release from PEGT/PBT matrices

*Materials*

In this example, five poly(ethylene oxide) terephthalate/poly(1,4-butylene terephthalate) (PEGT/PBT) copolymers were synthesized, wherein such copolymers had PEGT/PBT weight ratios of 80/20, 77/23, 70/30, 60/40 and 40/60. The polyethylene glycol component of the polymers having PEGT/PBT weight ratios of 70/30, 60/40 and 40/60 had a molecular weight of 1,000. For the copolymer having a PEGT/PBT weight ratio of 77/23, the molecular weight of the polyethylene glycol component was 600 and for the polymer having a PEGT/PBT weight ratio of 80/20 the molecular weight of the polyethylene glycol component was 4,000. Such copolymers are indicated as aPEGbPBTc, in which a is the PEG molecular weight, b the wt% PEG-terephthalate and c the wt% PBT.

Phosphate Buffered Saline (PBS), pH 7.4 was purchased from NPBI (Emmer-compascuum, NL). Polyvinyl alcohol (PVA, $M_w$ = 22,000) was obtained from ICN Biomedical Inc., Lysozyme from chicken egg white (3x crystallized, dialysed and lyophilized) was purchased from Sigma Chem Corp. (St. Louis, USA). 1M 2,3,6-trinitrobenzenesulphonic acid (TNBS) from Fluka Chemika, $NaHCO_3$, 1M NaOH, HCl (37%), Chloroform ($CHCl_3$) and methanol (MeOH), obtained from Merck (Darmstadt, DE) were of analytical grade.

*Preparation and characterization of (lysozyme containing) microspheres and films*

Preparation and characterization of lysozyme containing films and microspheres was carried out as described in example 4 and 5.

*Determination of initial diffusion coefficient*

The initial diffusion coefficient of lysozyme through PEGT/PBT films was determined as a function of copolymer composition. Lysozyme release was determined as described in example 1. Diffusion coefficients were determined by plotting the fractional lysozyme release $M_t/M_\infty$ versus $(time)^{1/2}$. From the first part of the curves, which is a straight line, the initial diffusion coefficient $D_0$ was estimated using equation (4):

$$M_t/M_\infty = 4\,(D_0 t/\pi l^2)^{1/2} \qquad (4)$$

in which l is the thickness of the film and t the time.

Figure 33 shows the release of lysozyme from PEGT/PBT films of different composition as a function of $(time)^{1/2}$. The diffusion coefficients calculated from the first part of the curves were very small as compared to the diffusion of lysozyme in water ($10^{-6}$ $cm^2/s$) (table XXII). The diffusion coefficients were strongly dependent on the swelling of the copolymers (figure 34). An increase in water uptake of the matrices from 40% up to 220% caused an almost 5,000 times increase in the diffusion coefficient. This shows again that a wide range of release rates can be obtained using the PEGT/PBT system as matrices for drug delivery.

Table XXII:

| Water-uptake of PEGT/PBT copolymers and diffusion coefficient of lysozyme as function of the polymer composition. Protein loaded films were prepared from water-in-oil emulsions containing 1.2 ml lysozyme solution (55 mg/ml PBS) and 2 g polymer (9 wt% in $CHCl_3$). | | |
|---|---|---|
| Polymer | water uptake (%) | diffusion coefficient (x$10^{11}$ $cm^2/s$) |
| 1000PEG40PBT60 | 40 | 0.0079 |
| 1000PEG60PBT40 | 61 | 0.03 |
| 1000PEG70PBT30 | 80 | 0.54 |
| 600PEG77PBT23 | 55 | 0.021 |
| 4000PEG80PBT20 | 220 | 390 |

*Modelation of protein release*

As described in example one, the constant release from PEGT/PBT matrices is most likely the result of the influence of polymer degradation on the diffusion through the polymer matrix. To quantify the effect of polymer degradation on the diffusion of lysozyme through the polymer matrix, the relation between the diffusion coefficient and the molecular weight of the polymers must be found. Polymers of different molecular weight were obtained by incubating 1000PEG70PBT30 powder (2 g) in PBS (25 ml) at 37¡C. The incubation time was 3, 5, 7, 11, 14 or 21 days and buffer was refreshed weekly. After incubation, buffer was removed, polymers were washed with methanol and dried for 2 days in a vacuum oven at 50¡C. Polymer molecular weight was estimated from figure 21. Protein containing films, lysozyme release and diffusion coefficients were determined as described above.

Figure 35 shows the release of lysozyme from PEGT/PBT films of different molecular weight as a function of $(time)^{1/2}$. A decrease in release rate was observed with increasing molecular weight. The diffusion coefficients, calculated from the first part of the curves were plotted against $(1/M_n)^2$ to yield a straight line (figure 36):

$$D = k_1 \times (1/M_n)^2 + k_2 \qquad (5)$$

From figure 21 it is known that

$$(1/M_n) = k_3 \times t + (1/M_{n,0}) \qquad (6)$$

in which D = diffusion coefficient, $M_n$ = number average molecular weight, t = time, $M_{n,0}$ = number average molecular weight at t = 0, and $k_1$ ...$k_3$ are constants. Combining (5) and (6) and substitution of D = $D_0$ at t = 0, results in an equation (7) for a time dependent diffusion coefficient:

$$D(t) = D_0 \times (1 + a\,t + b\,t^2) \qquad (7)$$

The value of a and b can be calculated from figures 21 and 36, and $D_0$ for lysozyme diffusion through the different PEGT/PBT copolymers can be determined from figure 34. To describe the diffusion of lysozyme through PEGT/PBT matrices, the time dependent diffusion coefficient can be incorporated in the equations for diffusion by a simple substitution (Crank, The mathematics of diffusion):

$$T = \int_0^t D(t')dt'$$

$$T = D_0\,(t + 0.5at^2 + bt^3) \qquad (8)$$

Diffusion from spheres can be described by

$$\frac{M_t}{M_\infty}=1-\frac{6}{\pi^2}\exp(-\frac{\pi^2 T}{r^2})$$

$$M_t/M_\infty > 0,6 \qquad (9)$$

$$\frac{M_t}{M_\infty}= \sqrt[6]{\frac{T}{\pi r^2}}$$

$$M_t/M_\infty < 0,4 \qquad (10)$$

in which r is the radius of the sphere.

Diffusion from films can be described by

$$\frac{M_t}{M_\infty}=1-\frac{8}{\pi^2}\exp(-\frac{\pi^2 T}{r^2})$$

$$M_t/M_\infty > 0,6 \qquad (11)$$

$$\frac{M_t}{M\infty}=\sqrt[4]{\frac{T}{\pi r^2}}$$

$$M_t/M_\infty < 0,4 \qquad (12)$$

in which r is the thickness of the film.

Figure 37 shows the results of the calculation of the lysozyme release from 1000PEG70PBT30, 1000PEG60PBT40 and 1000PEG40PBT60 films and 1000PEG70PBT30 and 1000PEG60PBT40 microspheres using the above presented model. The constants used in the calculation are given in table XXIII. It is clear that the model is successful - the calculated release curve closely follows the experimental curve.

Table XXIII:

| Constants used in equations (8)-(12) to calculate lysozyme release from PEGT/PBT matrices. a: $10^{-5}s^{-1}$; b: 1.3 x $10^{-12}s^{-2}$ | | | |
|---|---|---|---|
| Polymer | form of device | r (μm) | $D_0$ (x $10^{12}$ $cm^2/s$) |
| 1000PEG40PBT60 | film | 96 | 0.08 |
| 1000PEG60PBT40 | film | 96 | 0.3 |
| 1000PEG70PBT30 | film | 109 | 5 |
| 1000PEG60PBT40 | microsphere | 128 | 0.3 |
| 1000PEG70PBT30 | microsphere | 150 | 5 |

*Conclusion*

It was shown that release can be described by a combination of diffusion and polymer degradation. For moderately swollen matrices, polymer degradation will result in an increase of the diffusion coefficient, which may compensate for the reduced drug concentration in the matrix, to yield an almost constant release rate. However, for highly swollen matrices, diffusion is fast as compared to degradation. resulting in first order release. A mathematical model of the release mechanism was developed which was consistent with the experimental observations. This offers the possibility to tailor release rates of drugs from PEGT/PBT matrices precisely.

**Description of the figures**

Fig. 1 shows a typical size distribution of a PolyActive microsphere batch in μm.

Fig. 2 shows the effect of the PVA concentration (%) on the volume weight average diameter (●) and the number weight average diameter (■) in μm at 5% PolyActive (w/v) with a PEGT/PBT ratio of 70/30; stirring rate : 1200 rpm.

Fig. 3 shows the effect of the PolyActive (PA) concentration (%) on the volume weight average diameter (■) and the number weight average diameter (●) in μm at 5% PVA (w/v) with a PEGT/PBT ratio of 70/30; stirring rate : 1200 rpm.

Fig. 4 shows the release of BSA from PA 60/40 microspheres; '10%' refers to the concentration of PA in $CH_2Cl_2$.

Fig. 5 shows the release of BSA from PA 70/30 microspheres; '10%' refers to the concentration of PA in $CH_2Cl_2$.

Fig. 6 shows the release of BSA from PLGA microspheres; '10%' refers to the concentration of PLGA in $CH_2Cl_2$; SD means spray-dried particles.

Fig. 7 shows the release of diphtheria toxoid from PA 60/40 and 70/30 microspheres; '10%' refers to the concentration of PA in $CH_2Cl_2$.

Fig. 8 shows the release of diphtheria toxoid from PA 80/20 microspheres; '10%' refers to the concentration of PA in $CH_2Cl_2$.

Fig. 9 shows the release of lysozyme from PA 80/20 microspheres.

Fig. 10A and 10B show the release of lysozyme from PA 80/20 microspheres as a function of time and as a function of square root of time, respectively.

Fig. 11 shows the weight average molecular weight ($M_w$) of PLGA and PA in microspheres as a function of the aging time.

Fig. 12 shows indomethacin release profiles from PolyActive devices: + (·-·-·) PEG 1000 40/60; □ (----) PEG 1000 60/40; o (----) PEG 1000 80/20; + (·····) PEG 600 40/60; ▲ (______) PEG 600 60/40; • (---) PEG 600 77/23.

Fig. 13A and 13B show the weight loss of Polyactive granulate with α-tocopherol and with Trolox, respectively.

Fig. 14A and 14B show average $M_w$ values (n=3) for gamma-irradiated rods of PEGT/PBT 70/30 rods, with percentage of original mass in fig. 14B.

Fig. 15 shows average $M_w$ values (n=3) for gamma-irradiated and non-irradiated PEGT/PBT 70/30 rods with antioxidants: -o- α-tocopherol γ-irradiated; -Δ- α-tocopherol non-irradiated; -●- Trolox γ-irradiated; -◆- Trolox non-irradiated.

Fig. 16 shows average $M_w$ values (n=3) for gamma-irradiated and non-irradiated PEGT/PBT 70/30 rods in 0.9% NaCl with antioxidants: -o- α-tocopherol γ-irradiated; -Δ-α-tocopherol non-irradiated; -●- Trolox γ-irradiated; -◆- Trolox non-irradiated.

Fig. 17 shows formic acid formation as % $OCH_2CH_2$ for PEGT/PBT 70/30 rods nos. 7 (-o-), 8 (-Δ-), 11 (-●-) and 13 (-◆-).

Fig. 18 shows acetic acid concentration in mg/l in medium for PEGT/PBT 70/30 rods nos. 7 (-o-), 8 (-Δ-), 11 (-●-), 13 (-◆-) and 15 (-o-).

Fig. 19 shows water uptake of PEGT/PBT copolymer films as a function of time; (□) 4000PEG80PBT20; (o) 1000PEG70PBT30; (◊) 1000PEG60PBT40; (Δ) 1000PEG40PBT60; (▽) 600PEG77PBT23; n = 3 ± S.D.

Fig. 20 shows degradation of: (□) 1000PEG70PBT30; (◊) 1000PEG60PBT40; (◊) 1000PEG40PBT60 films in PBS at 37°C. Filled (lower) symbols indicate weight loss (n=2 ± S.D.), open (upper) symbols represent $M_w$.

Fig. 21 shows the change of the reciprocal $M_n$ as a function of incubation time in PBS at 37°C of (□) 1000PEG70PBT30; (◊) 1000PEG60PBT40; (o) 1000PEG40PBT60 films.

Fig. 22A and 22B show cumulative release of lysozyme from PEGT/PBT films as a function of time and as a function of square root of time for 4000PEG80PBT20, respectively.

Fig. 23 shows the effect of polymer molecular weight on cumulative release of lysozyme from 1000PEG70PBT30 films. $M_n$ = 45,100 (□), 42,000 (◊), 36,600 (Δ) or 28,700 (o) g/mol.

Fig. 24 shows the activity of lysozyme released from (o) 1000PEG60PBT40; (◊) 1000PEG-40PBT60 matrices. (n=3 ±S.D.).

Fig. 25 shows cumulative protein release (%) from 1000PEG70PBT30 matrices. Release of lysozyme (◊), BSA (□) and IgG (Δ). Films were prepared from water-in-oil emulsions containing 0.6 ml lysozyme solution (55 mg/ml PBS) and 1 g polymer (9 wt.% in $CHCl_3$). (n=3 ± S.D.).

Fig. 26 shows the influence of the water/polymer ratio of the initial water-in-oil emulsion on the water uptake of BSA 1000PEG70PBT30 films. (n=3 ± S.D.).

Fig. 27 shows the effect of 1000PEG70PBT30 on the cumulative release of BSA. Water uptake (w.u.) is taken as a measure of porosity. (n=3 ± S.D.).

Fig. 28 shows the effect of the water/polymer ratio of the initial water-in-oil emulsion (w/p) and water uptake on

the cumulative release of IgG from 1000PEG70PBT30 matrices. (n=3 ± S.D.).

Fig. 29 shows the mean diameter (volume) of lysozyme-loaded 600PEG77PBT23 microspheres as a function of the viscosity of polymer solutions.

Fig. 30 shows the lysozyme release from 600PEG77PBT23 microspheres prepared from polymer solutions with different concentrations.

Fig. 31 shows the initial lysozyme release rate from 600PEG77PBT23 microspheres as a function of reciprocal microsphere size.

Fig. 32 shows the lysozyme release from PEGT/PBT microspheres as a function of copolymer composition.

Fig. 33 is a plot of $M_t/M_\infty$ versus $(time)^{1/2}$ for the release of lysozyme from PEGT/PBT matrices for different copolymers.

Fig. 34 shows the initial diffusion coefficient of lysozyme through PEGT/PBT matrices as a function of the water uptake.

Fig. 35 is a plot of $M_t/M_\infty$ versus $(time)^{1/2}$ for the release of lysozyme from 1000PEG70PBT30 matrices with different molecular weight.

Fig. 36 shows the initial diffusion coefficient of lysozyme through 1000PEG70PBT30 matrices as a function of the molecular weight.

Fig. 37A-E show a prediction of protein release from (A) 1000PEG70PBT30 films; (B) 1000PEG60PBT40 films; (C) 1000PEG40PBT60 films; (D) 1000PEG70PBT30 microspheres and (E) 1000PEG60PBT40 microspheres; ————: calculated; o: experimental.

## Claims

1. A composition for delivering a biologically active agent to a host comprising a biologically active agent encapsulated in a matrix comprising a copolymer of a polyalkylene glycol and poly(butylene terephthalate).

2. A composition according to claim 1, wherein said polyalkylene glycol is selected from the group consisting of polyethylene glycol, polypropylene glycol and polybutylene glycol and copolymers thereof.

3. A composition according to claim 2 wherein said copolymer is a polyethylene glycol/poly(butylene terephthalate) copolymer.

4. A composition according to any of the preceding claims, wherein said polyalkylene glycol component constitutes 30 wt.% to 90 wt.% of the weight of said copolymer.

5. A composition according to any of the preceding claims, wherein said biologically active agent is a non-peptide, non-protein drug.

6. A composition according to claim 5, wherein the polyalkylene glycol component of said copolymer has a molecular weight of from 200 to 400.

7. A composition according to claim 5 or 6, wherein said matrix further includes 0.5 wt.% to 2 wt.% of at least one hydrophobic antioxidant, based on the total weight of the matrix.

8. A composition according to any one of the claims 5-7, wherein 0.1 mole% to 20 mole% of said poly(butylene terephthalate) is replaced by an aliphatic polyester such as poly(alkylene succinate) and/or by an aromatic polyester of a dialkylene glycol, in an amount of from 0.1 mole% to 5 mole%.

9. A composition according to any of the preceding claims wherein 0.1 mole% to 20 mole% of said poly(butylene terephthalate) is replaced by a poly(diethyleneglycol terephthalate).

10. A composition according to any of the claims 1-4, wherein said biologically active agent is a biologically active protein having a molecular weight of greater than 10,000.

11. A composition according to claim 10, wherein the polyalkylene glycol component of said copolymer has a molecular weight of from 1,000 to 20,000.

12. A composition according to claim 10 or 11, wherein said matrix further includes up to 10 wt.%, based on the weight

of said copolymer, of polyethylene glycol having a molecular weight of from 4,000 to 10,000.

**13.** A composition according to any of the claims 1-4, wherein said biologically active agent is a biologically active peptide or a biologically active protein having a molecular weight of less than 10,000.

**14.** A composition according to claim 13, wherein said polyethylene glycol has a molecular weight of from 200 to 6,000.

**15.** A composition according to any of the claims 10-14, wherein said matrix further comprises 1.0 wt.% to 5.0 wt.% of a hydrophilic antioxidant, based on the total weight of the matrix.

**16.** A composition according to any of the preceding claims, wherein said matrix has the form of microspheres, rods, porous or non-porous blocks, sponges, particulate, membrane, plasters, films, tapes or threads.

**Patentansprüche**

**1.** Zusammensetzung für die Übermittlung eines biologisch aktiven Mittels an einen Wirt, umfassend ein biologisch aktives Mittel, verkapselt in einer Matrix, die ein Copolymer aus einem Polyalkylenglykol und Poly(butylenterephthalat) umfasst.

**2.** Zusammensetzung gemäß Anspruch 1, worin der Polyalkylenglykol ausgewählt ist aus der Gruppe, bestehend aus Polyethylenglykol, Polypropylenglykol und Polybutylenglykol und Copolymeren derselben.

**3.** Zusammensetzung gemäß Anspruch 2, worin das Copolymer ein Polyethylenglykol/Poly(butylenterephthalat)-Copolymer ist.

**4.** Zusammensetzung gemäß einem der vorstehenden Ansprüche, worin die Polyalkylenglykol-Komponente 30 Gew.-% bis 90 Gew.-% des Gewichts des Copolymeren ausmacht.

**5.** Zusammensetzung gemäß einem der vorstehenden Ansprüche, worin das biologisch aktive Mittel ein nicht peptidischer, nicht proteinöser Wirkstoff ist.

**6.** Zusammensetzung gemäß Anspruch 5, worin die Polyethylenglykol-Komponente des Copolymeren ein Molekulargewicht von 200 bis 400 aufweist.

**7.** Zusammensetzung gemäß Anspruch 5 oder 6, worin die Matrix zusätzlich 0,5 Gew.-% bis 2 Gew.-% an mindestens einem hydrophoben Antioxidans umfasst, basierend auf dem Gesamtgewicht der Matrix.

**8.** Zusammensetzung gemäß einem der Ansprüche 5-7, worin 0,1 Mol-% bis 20 Mol-% des Poly(ethylenterephthalats) durch einen aliphatischen Polyester wie Poly(alkylensuccinat) und/oder einen aromatischen Polyester eines Dialkylenglykols in einer Menge von 0,1 Mol-% bis 5 Mol-% ersetzt sind.

**9.** Zusammensetzung gemäß einem der vorstehenden Ansprüche, worin 0,1 Mol-% bis 20 Mol-% des Poly(butylenterephthalats) durch ein Poly(diethylenglykolterephthalat) ersetzt sind.

**10.** Zusammensetzung gemäß einem der Ansprüche 1-4, worin das biologisch aktive Mittel ein biologisch aktives Protein mit einem Molekulargewicht von über 10.000 ist.

**11.** Zusammensetzung gemäß Anspruch 10, worin die Polyalkylenglykol-Komponente des Copolymeren ein Molekulargewicht von 1.000 bis 20.000 aufweist.

**12.** Zusammensetzung gemäß Anspruch 10 oder 11, worin die Matrix zusätzlich bis zu 10 Gew.-%, basierend auf dem Gewicht des Copolymeren, an Polyethylenglykol mit einem Molekulargewicht von 4.000 bis 10.000 aufweist.

**13.** Zusammensetzung gemäß einem der Ansprüche 1-4, worin das biologisch aktive Mittel ein biologisch aktives Peptid oder ein biologisch aktives Protein mit einem Molekulargewicht von weniger als 10.000 ist.

**14.** Zusammensetzung gemäß Anspruch 13, worin das Polyethylenglykol ein Molekulargewicht von 200 bis 6.000

aufweist.

15. Zusammensetzung gemäß einem der Ansprüche 10-14, worin die Matrix zusätzlich 1,0 Gew.-% bis 5,0 Gew.-% eines hydrophilen Antioxidans umfasst, basierend auf dem Gesamtgewicht der Matrix.

16. Zusammensetzung gemäß einem der vorstehenden Ansprüche, worin die Matrix die Form von Mikrokügelchen, Stäbchen, porösen oder nicht porösen Blöcken, Schwämmen, Teilchen, einer Membran, Pflastern, Filmen, (Klebe-) Bändern oder Fäden aufweist.

**Revendications**

1. Composition pour la distribution à un hôte d'un agent actif du point de vue biologique, comprenant un agent actif du point de vue biologique encapsulé dans une matrice comprenant un copolymère d'un polyalkylène glycol et d'un poly(téréphtalate de butylène).

2. Composition selon la revendication 1, dans laquelle ledit polyalkylène glycol est choisi dans le groupe consistant en polyéthylène glycol, polypropylène glycol et polybutylène glycol et leurs copolymères.

3. Composition selon la revendication 2, dans laquelle ledit copolymère est un copolymère de polyéthylène glycol et de poly(téréphtalate de butylène).

4. Composition selon l'une quelconque des revendications précédentes, dans laquelle ledit composant polyalkylène glycol constitue 30% en poids à 90% en poids du poids dudit copolymère.

5. Composition selon l'une quelconque des revendications précédentes, dans laquelle ledit agent actif du point de vue biologique est un médicament non peptidique, non protéinique.

6. Composition selon la revendication 5, dans laquelle le composant polyalkylène glycol dudit copolymère a un poids moléculaire de 200 à 400.

7. Composition selon les revendications 5 ou 6, dans laquelle ladite matrice comprend de plus 0,5% en poids à 2% en poids d'au moins un anti-oxydant hydrophobe, par rapport au poids total de la matrice.

8. Composition selon l'une quelconque des revendications 5 à 7, dans laquelle 0,1% en moles à 20% en moles dudit poly(téréphtalate de butylène) sont remplacés par un polyester aliphatique comme un poly(succinate d'alkylène) et/ou par un polyester aromatique d'un dialkylène glycol en une quantité de 0,1 % en moles à 5% en moles.

9. Composition selon l'une quelconque des revendications précédentes, dans laquelle 0,1% en moles à 20% en moles dudit poly(téréphtalate de butylène) sont remplacés par un poly(téréphtalate de diéthylène glycol).

10. Composition selon l'une quelconque des revendications 1 à 4, dans laquelle ledit agent actif du point de vue biologique est une protéine active du point de vue biologique ayant un poids moléculaire supérieur à 10.000.

11. Composition selon la revendication 10, dans laquelle le composant polyalkylène glycol dudit copolymère a un poids moléculaire de 1.000 à 20.000.

12. Composition selon les revendications 10 ou 11, dans laquelle ladite matrice comprend de plus jusqu'à 10% en poids par rapport au poids dudit copolymère, de polyéthylène glycol ayant un poids moléculaire de 4.000 à 10.000.

13. Composition selon l'une quelconque des revendications 1 à 4, dans laquelle ledit agent actif du point de vue biologique est un peptide actif du point de vue biologique ou une protéine active du point de vue biologique ayant un poids moléculaire inférieur à 10.000.

14. Composition selon la revendication 13, dans laquelle ledit polyéthylène glycol a un poids moléculaire de 200 à 6.000.

15. Composition selon l'une quelconque des revendications 10 à 14, dans laquelle ladite matrice comprend de plus

1,0% en poids à 5,0% en poids d'un anti-oxydant hydrophile par rapport au poids total de la matrice.

**16.** Composition selon l'une quelconque des revendications précédentes, dans laquelle ladite matrice a la forme de microsphères, de bâtonnets, de blocs poreux ou non poreux, d'éponges, de particules, de membrane, d'emplâtres, de films, de rubans ou de fils.

FIG.1

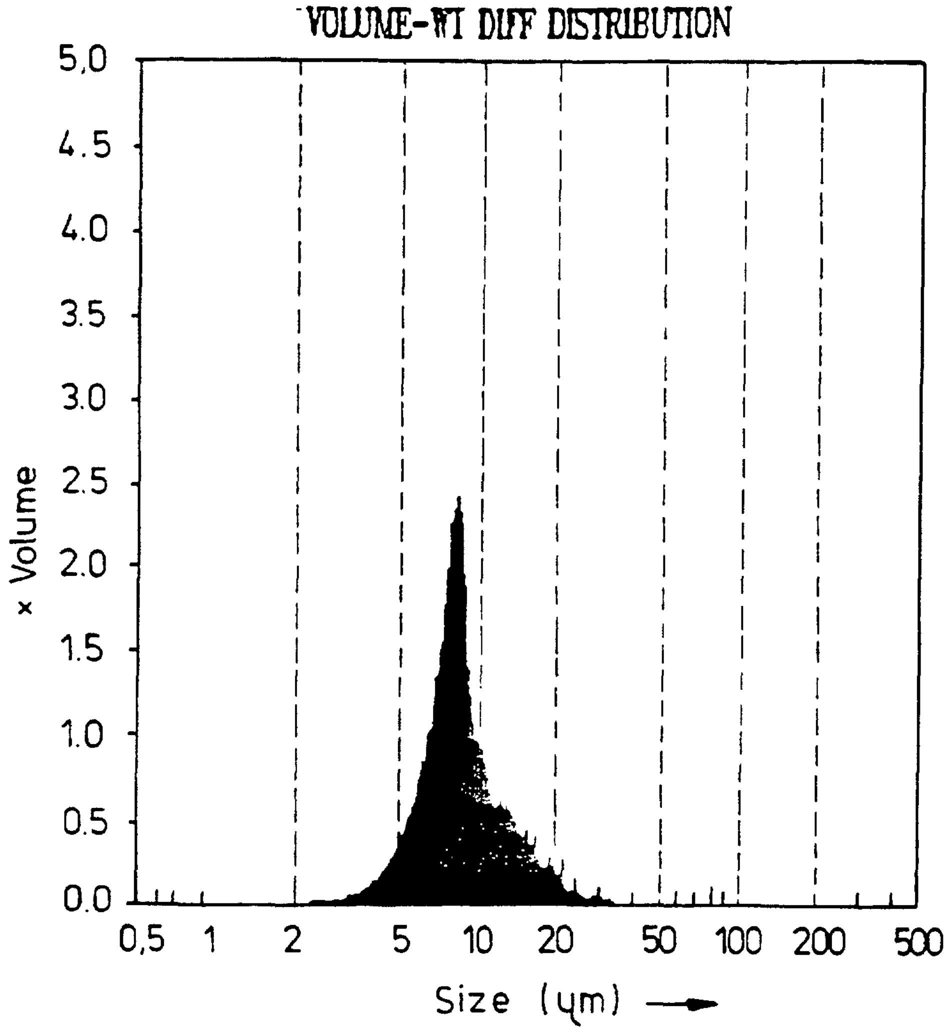


a typical size distribution of a PolyActive microsphere batch in µm.

FIG. 2

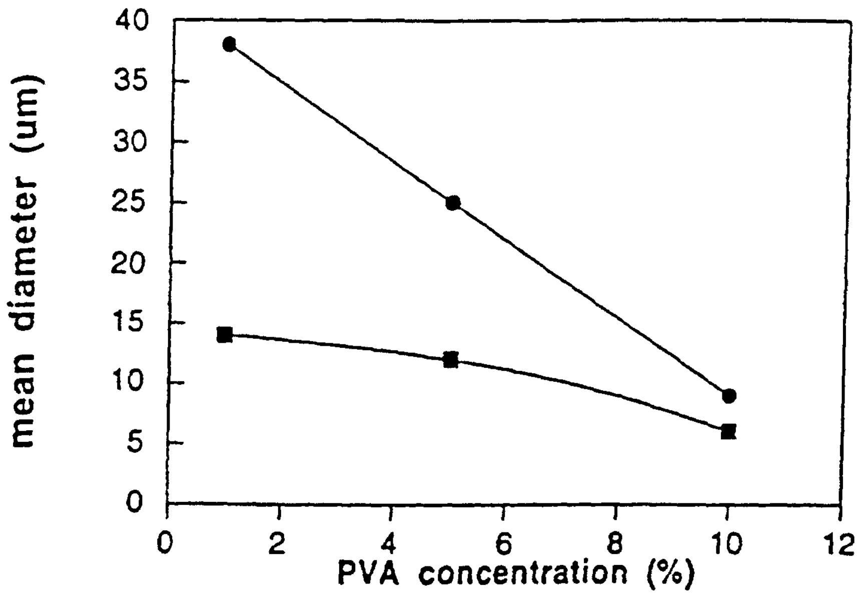


PolyActive concentration 5 % (w/v); stirring rate: 1200 rpm.
(•) volume weight average diameter; (■) number weight average diameter.
PEGT/PBT ratio of 70/30

FIG. 3

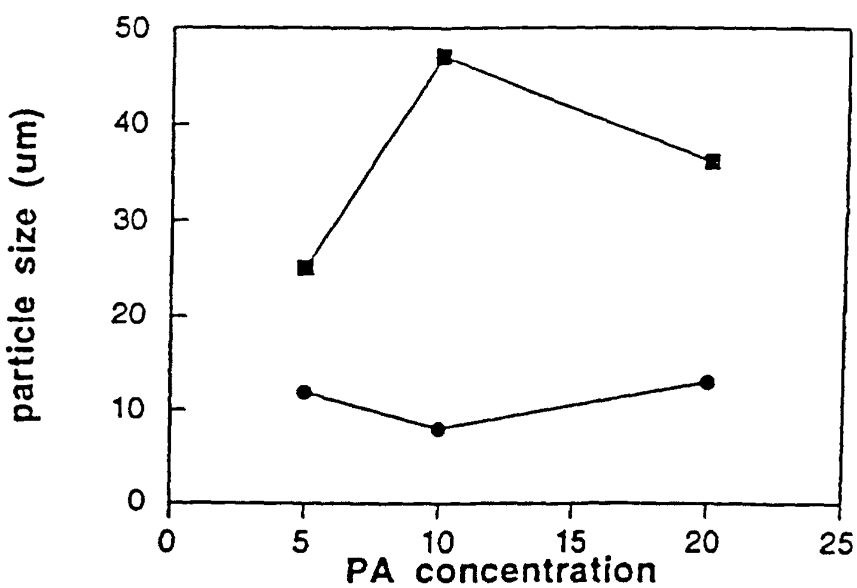


PVA concentration 5 %; stirring rate: 1200 rpm. (■) volume weight average diameter;
(•) number weight average diameter.
PEGT/PBT ratio of 70/30

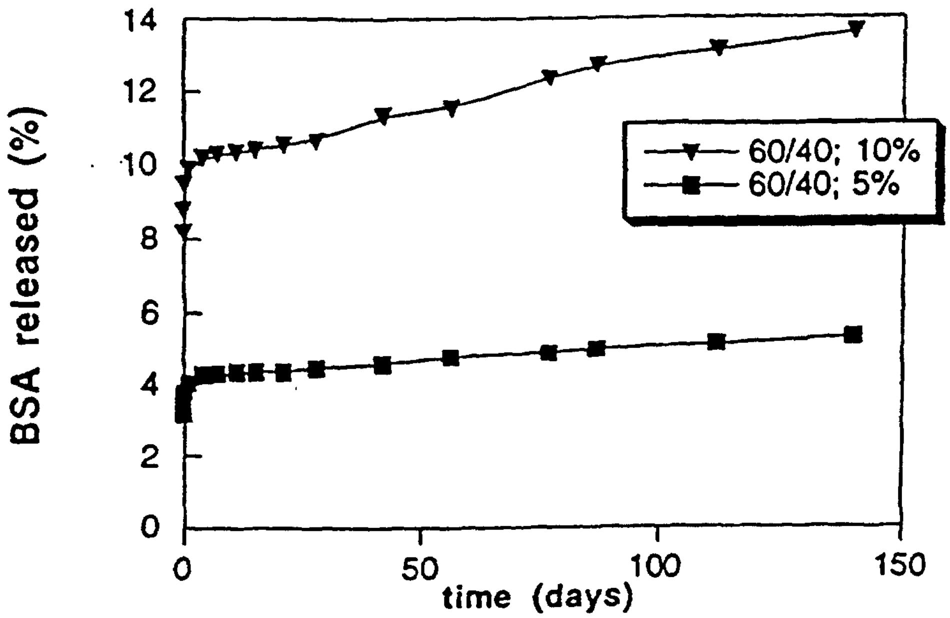


The release of BSA from PA 60/40 microspheres. '10 %' refers to the concentration of PA in $CH_2Cl_2$.

Fig. 4

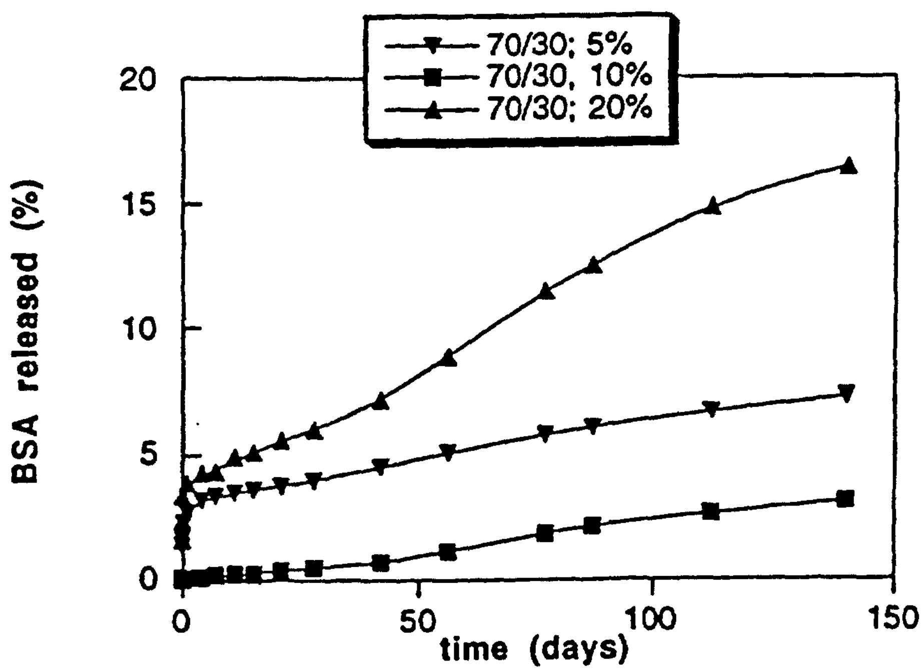


. The release of BSA from PA 70/30 microspheres; '10 %' refers to the concentration of PA in $CH_2Cl_2$.

Fig. 5

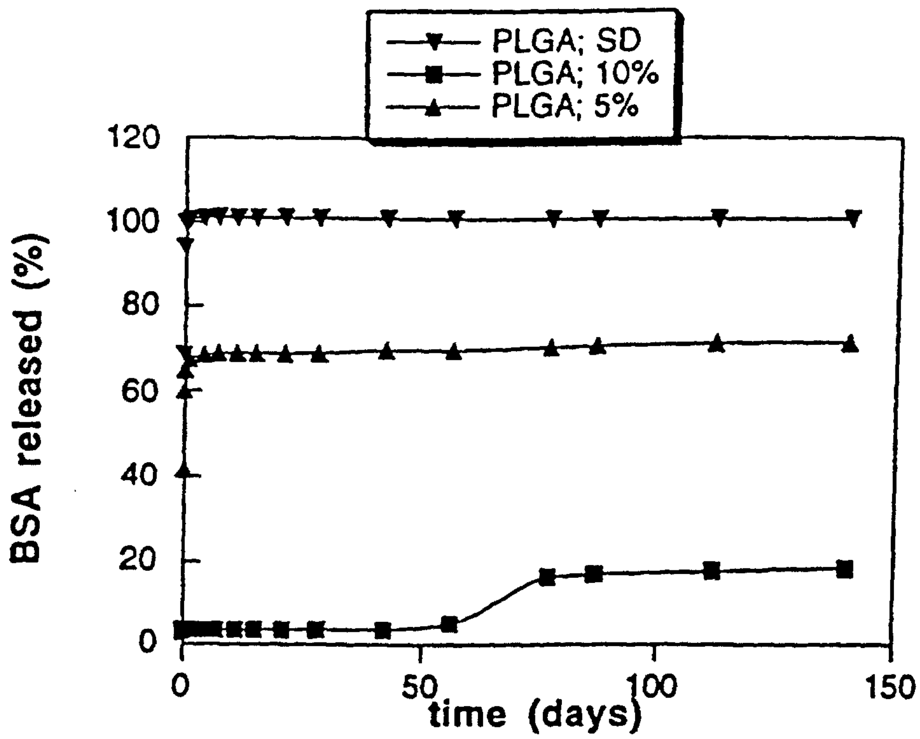


The release of BSA from PLGA microspheres; '10 %' refers to the concentration of PLGA in $CH_2Cl_2$. SD means spray dried particles.

Fig. 6

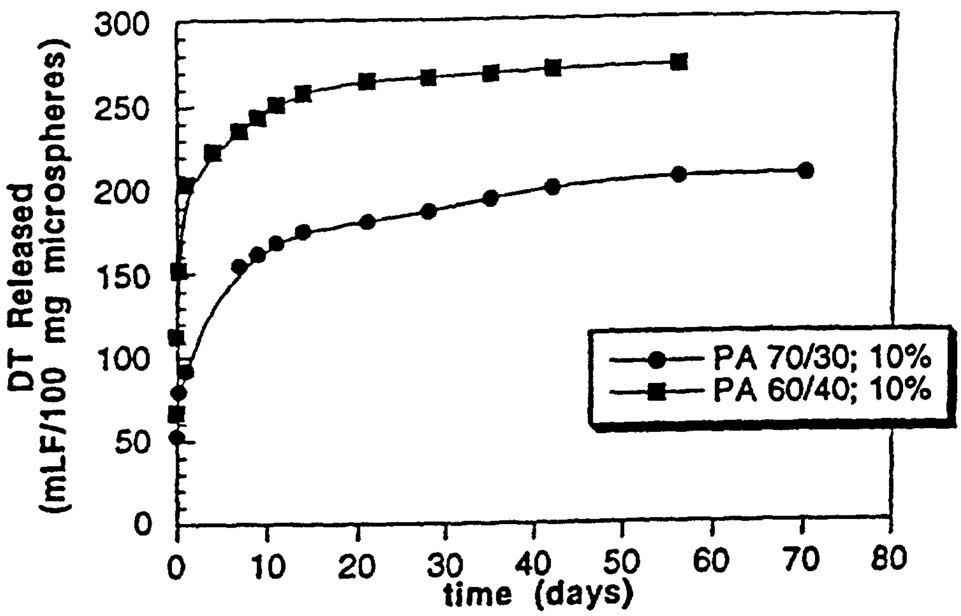


The release of diphtheria toxoid from PA microspheres, 10 % refers to the concentration of PA in $CH_2Cl_2$.

Fig. 7

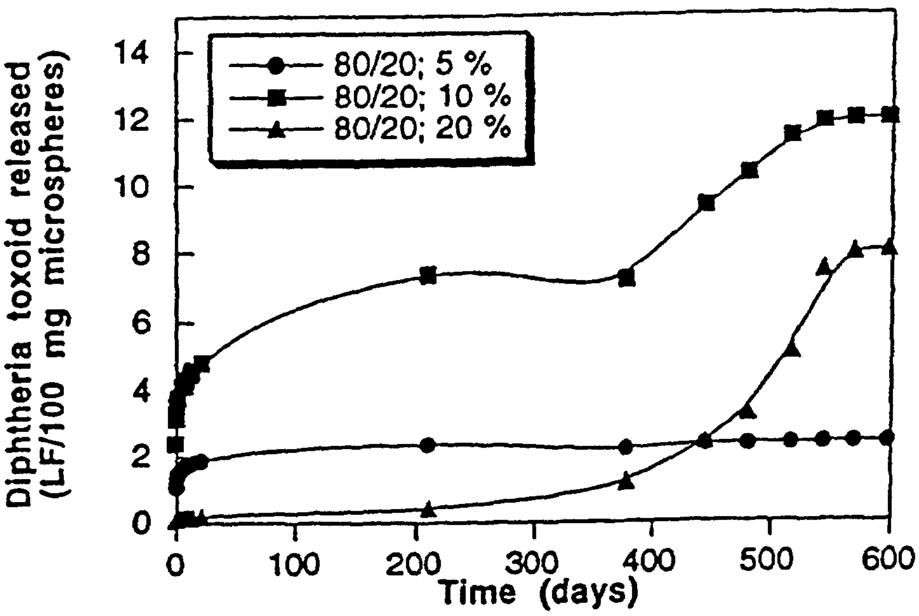


. The release of diphtheria toxoid from PA 80/20 microspheres; 10 % refers to the concentration of PA in $CH_2Cl_2$.

Fig. 8

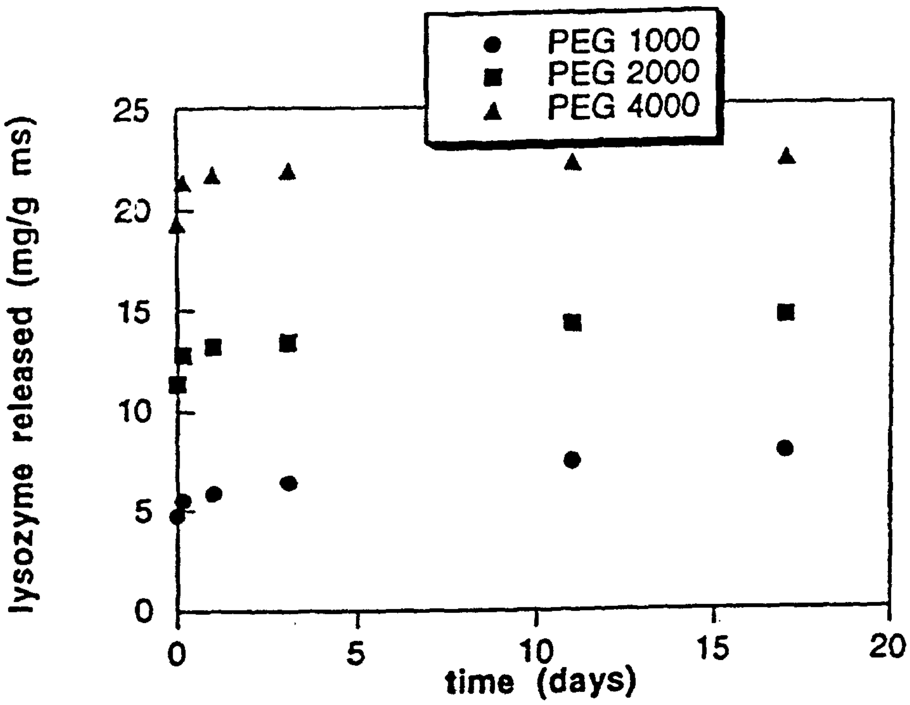


. The release of lysozyme from PA 80/20 microspheres.

Fig. 9

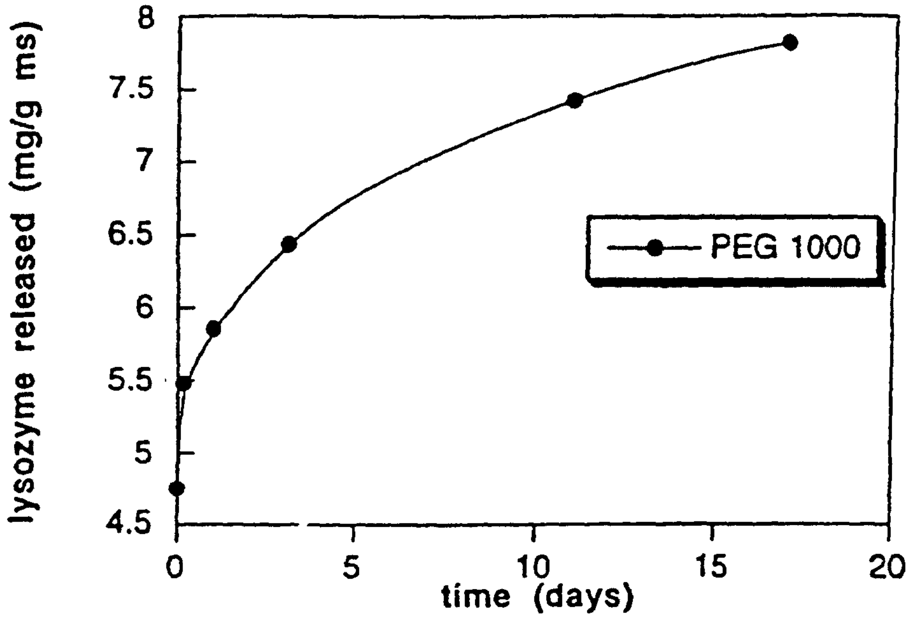


The release of lysozyme from PA 80/20 microspheres as a function of time.

Fig.10A

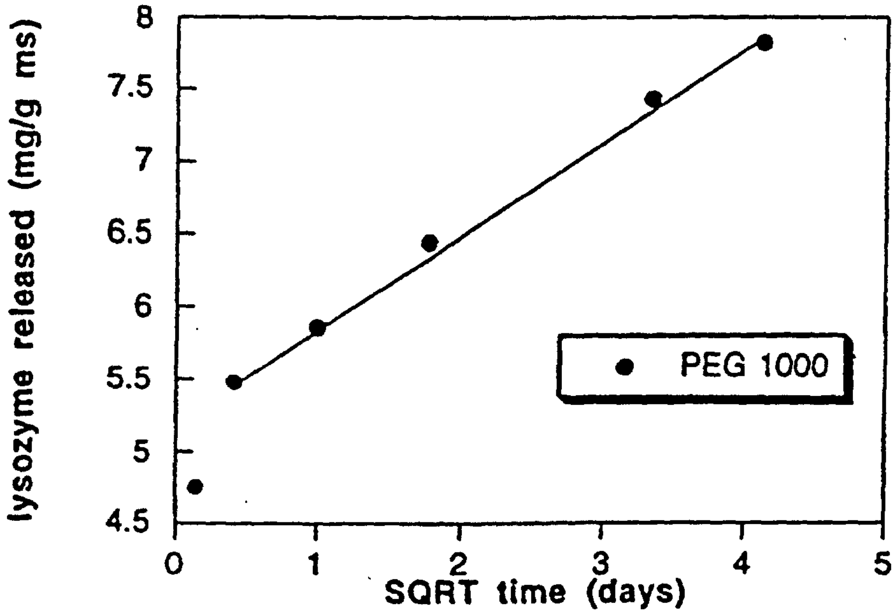


The release of lysozyme from PA 80/20 microspheres as a function of square root of time.

Fig. 10B

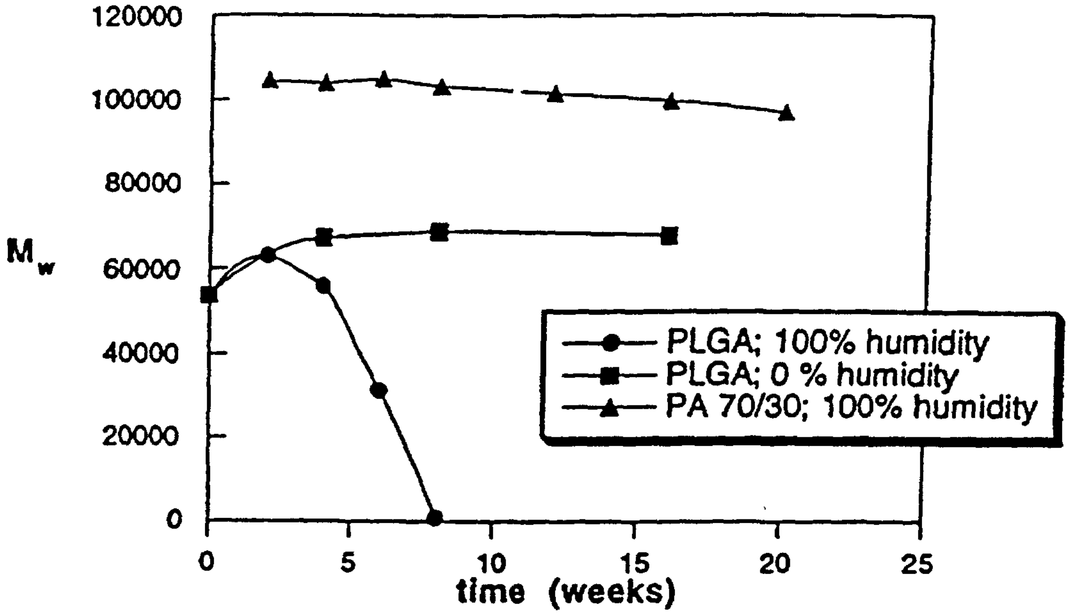


. Weight average molecular weight ($M_W$) of PLGA and PA in microspheres as a function of the ageing time.

Fig. 11

Fig. 12

indomethacin release profiles from PolyActive devices: + (–·–·) PEG 1000 40/60; □ (– – – –) PEG 1000 60/40; o (– – – –) PEG 1000 80/20; + (·······) PEG 600 40/60; ▲ (———) PEG 600 60/40; • (— — —) PEG 600 77/23.

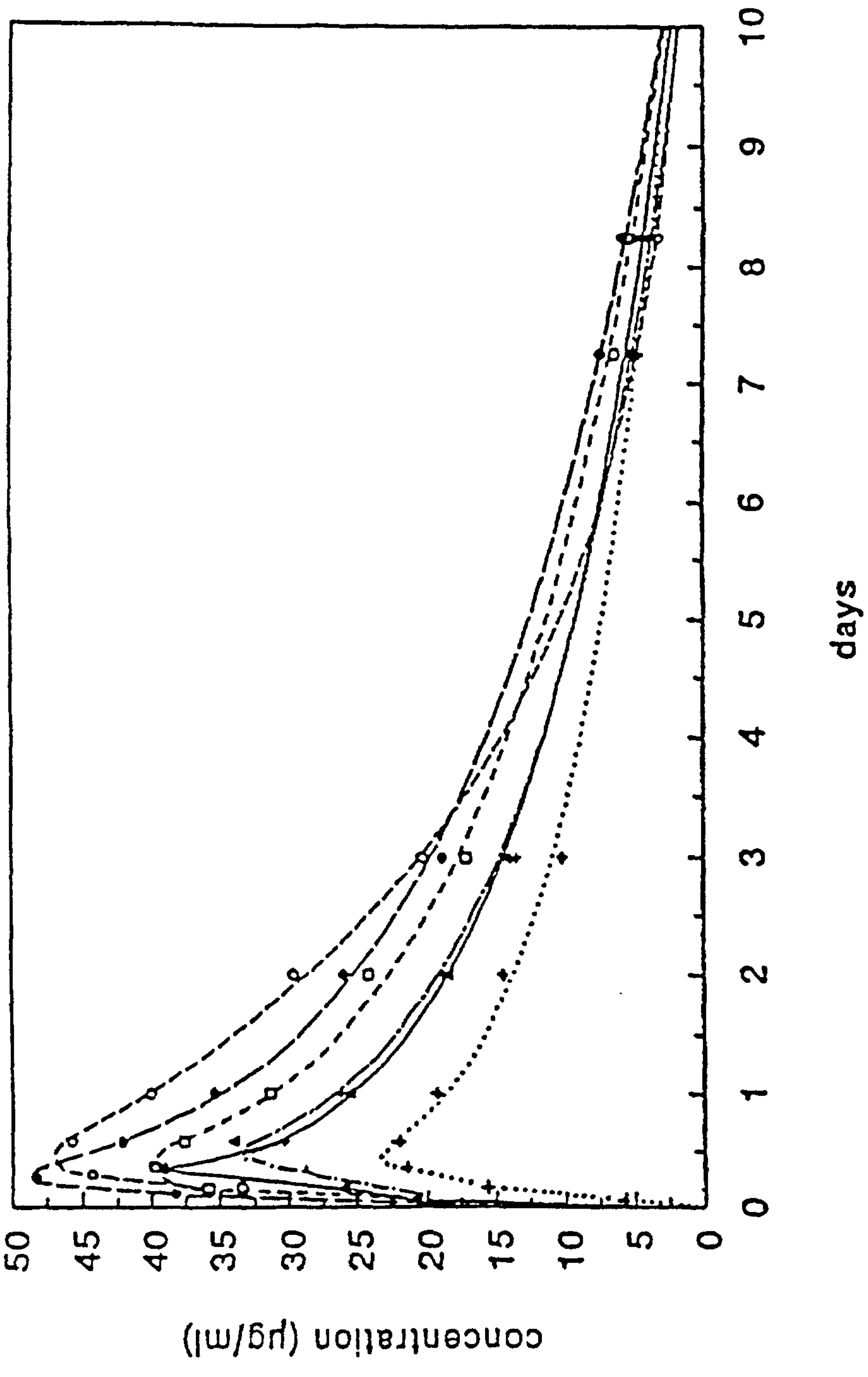

FIG. 13A

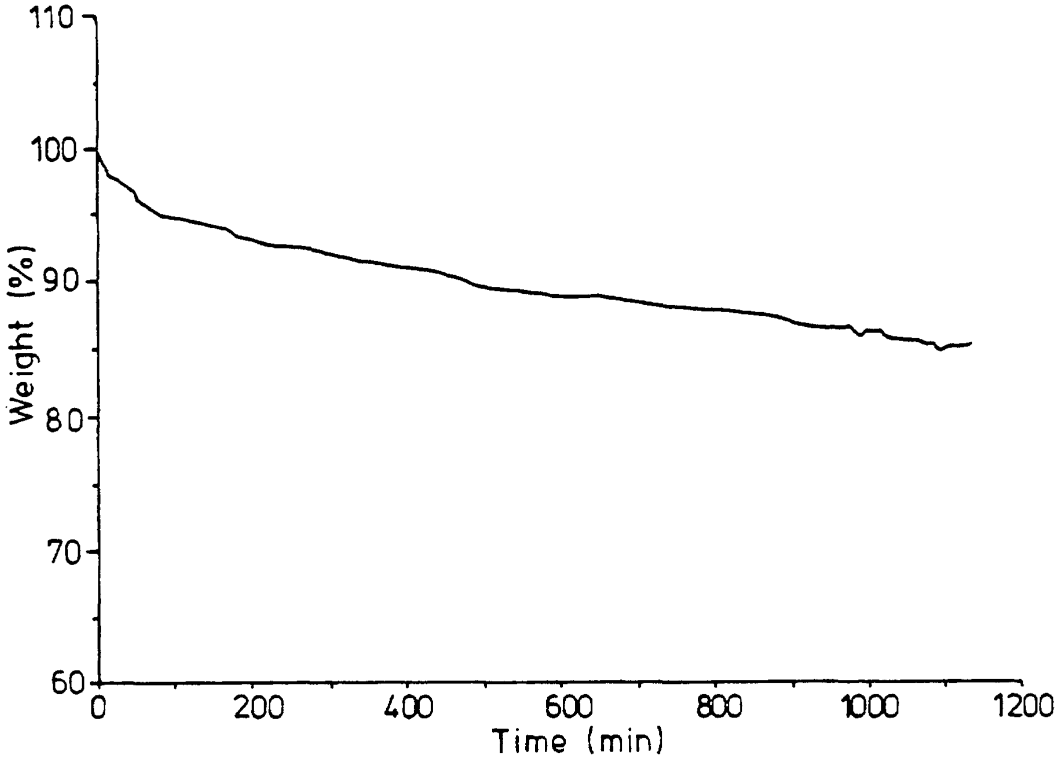


FIG. 13B

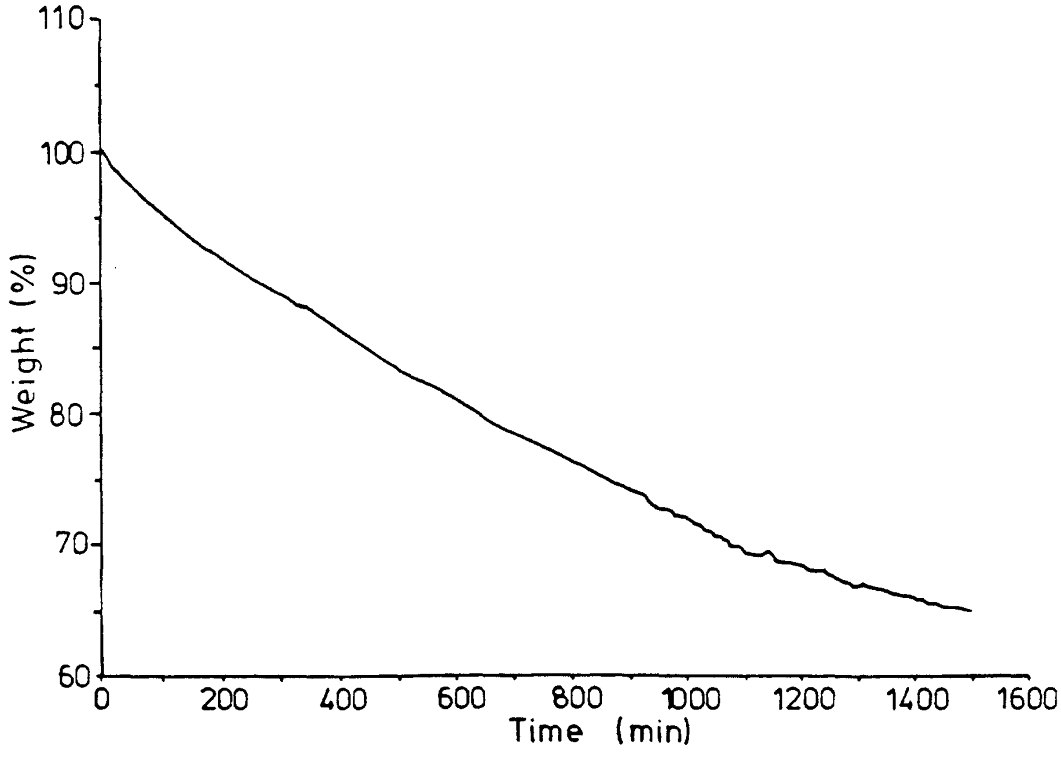

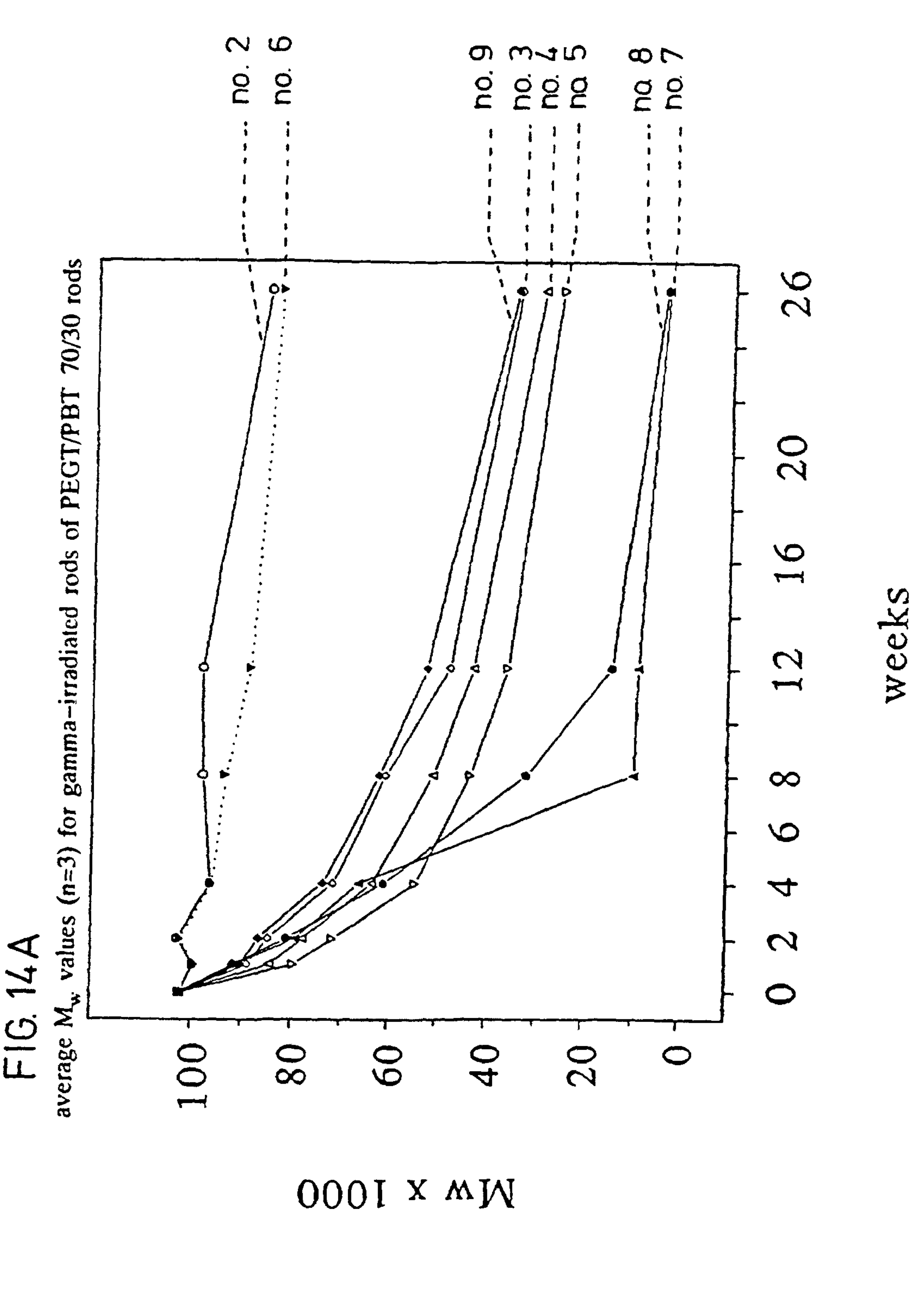
FIG. 14A
average $M_w$ values (n=3) for gamma-irradiated rods of PEGT/PBT 70/30 rods
Mw x 1000
100
80
60
40
20
0
0 2 4 6 8 12 16 20 26
weeks
no. 2
no. 6
no. 9
no. 3
no. 4
no. 5
no. 8
no. 7

FIG. 14 B

average $M_w$ values (n=3) for gamma-irradiated rods of PEGT/PBT 70/30 rods, with percentage of original mass

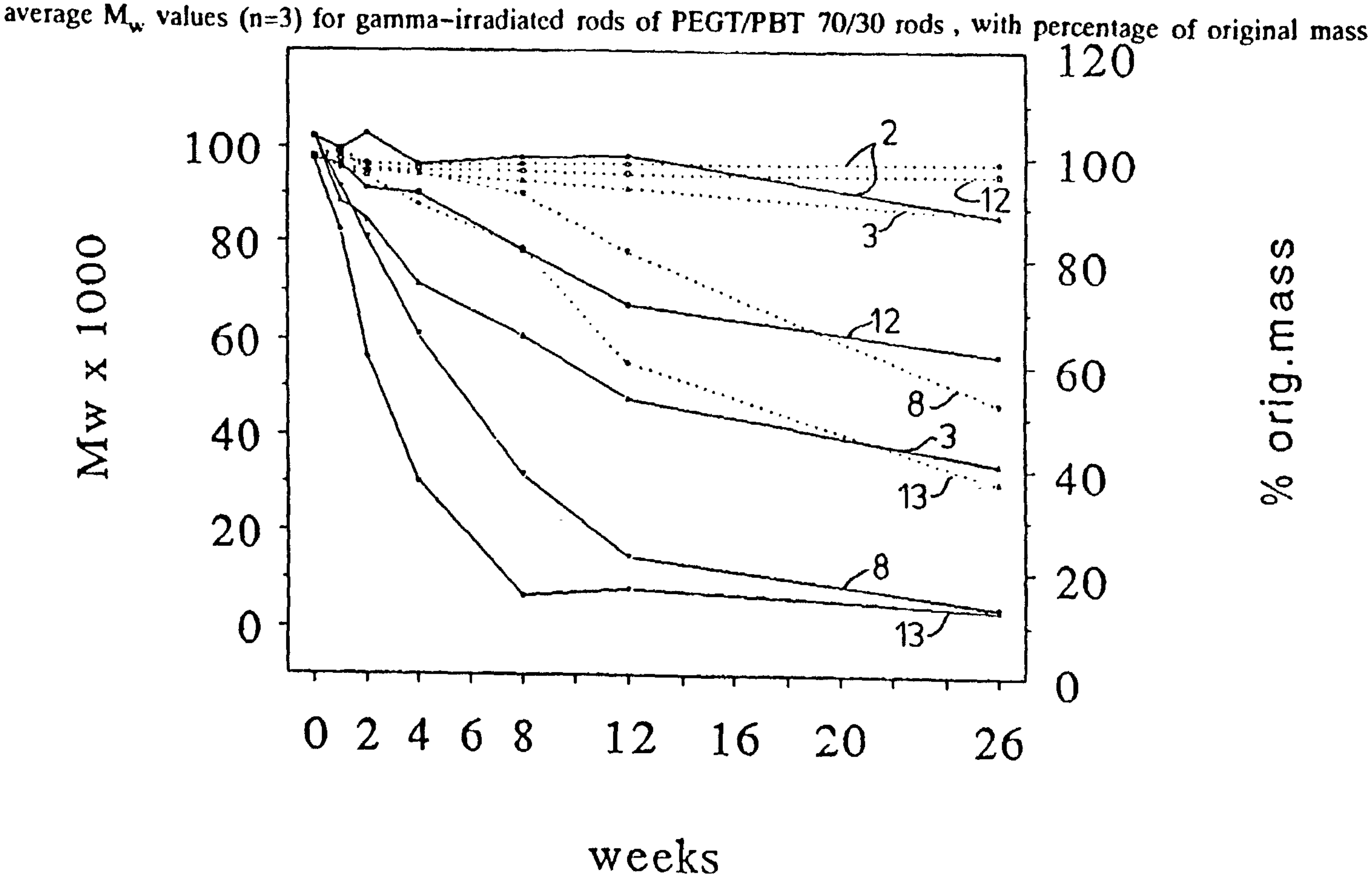

FIG. 15

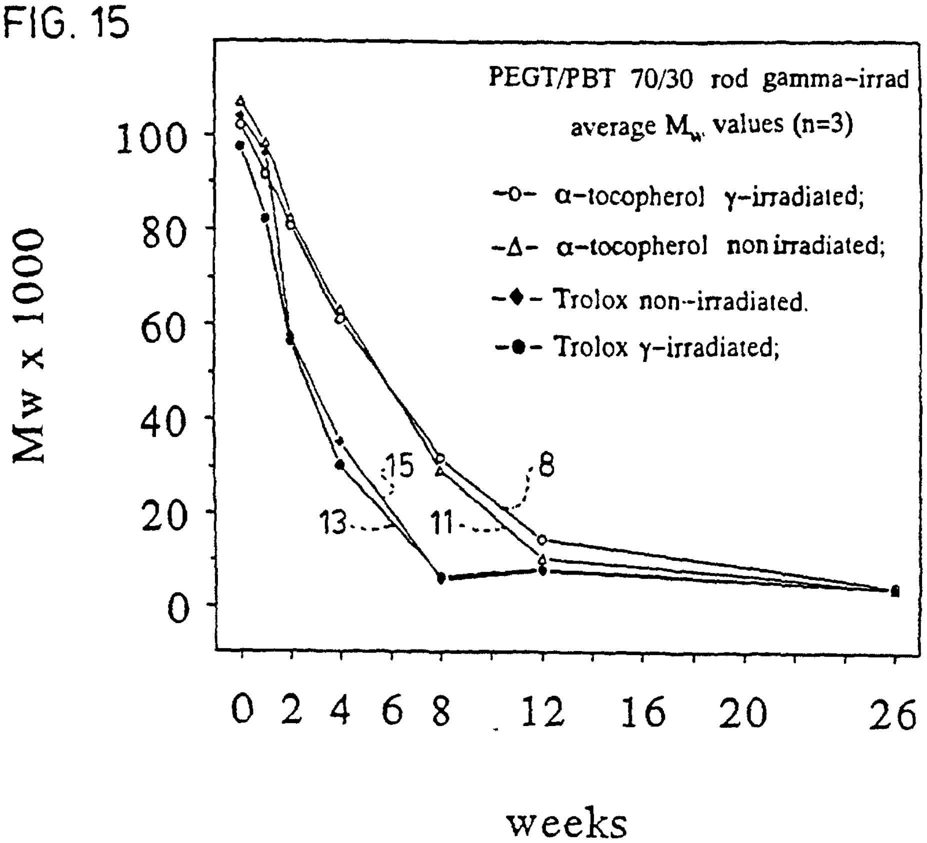
PEGT/PBT 70/30 rod gamma-irrad
average $M_w$ values (n=3)
-o- α-tocopherol γ-irradiated;
-Δ- α-tocopherol nonirradiated;
-♦- Trolox non-irradiated.
-●- Trolox γ-irradiated;
Mw x 1000
100
80
60
40
20
0
15
8
13
11
0 2 4 6 8 12 16 20 26
weeks

FIG. 16

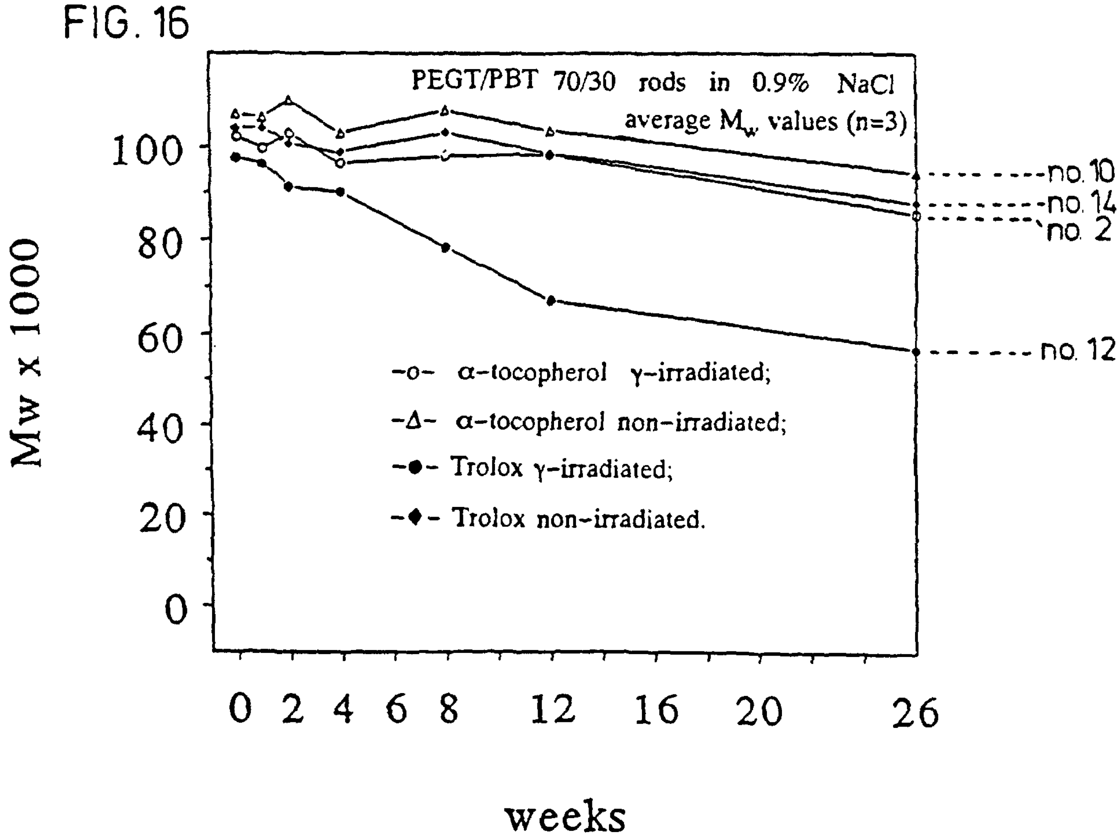
PEGT/PBT 70/30 rods in 0.9% NaCl
average $M_w$ values (n=3)
-o- α-tocopherol γ-irradiated;
-Δ- α-tocopherol non-irradiated;
-●- Trolox γ-irradiated;
-♦- Trolox non-irradiated.
Mw x 1000
100
80
60
40
20
0
no. 10
no. 14
no. 2
no. 12
0 2 4 6 8 12 16 20 26
weeks

FIG. 17

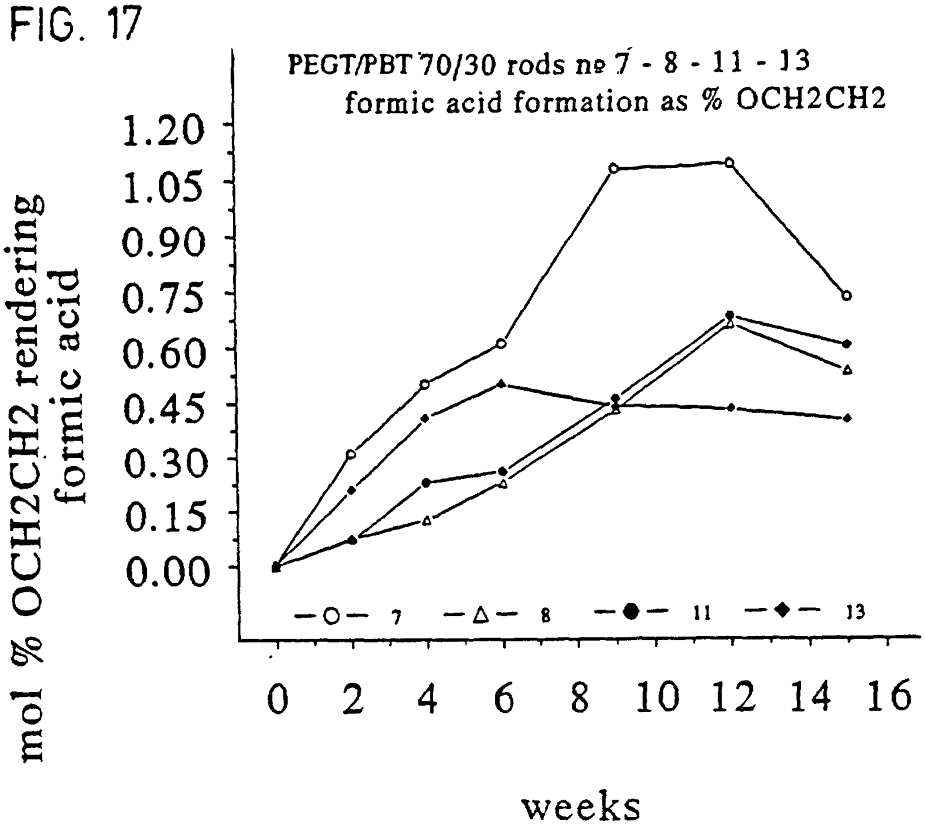
PEGT/PBT 70/30 rods nº 7 - 8 - 11 - 13
formic acid formation as % OCH2CH2
mol % OCH2CH2 rendering formic acid
1.20
1.05
0.90
0.75
0.60
0.45
0.30
0.15
0.00
7
8
11
13
0 2 4 6 8 10 12 14 16
weeks

FIG. 18

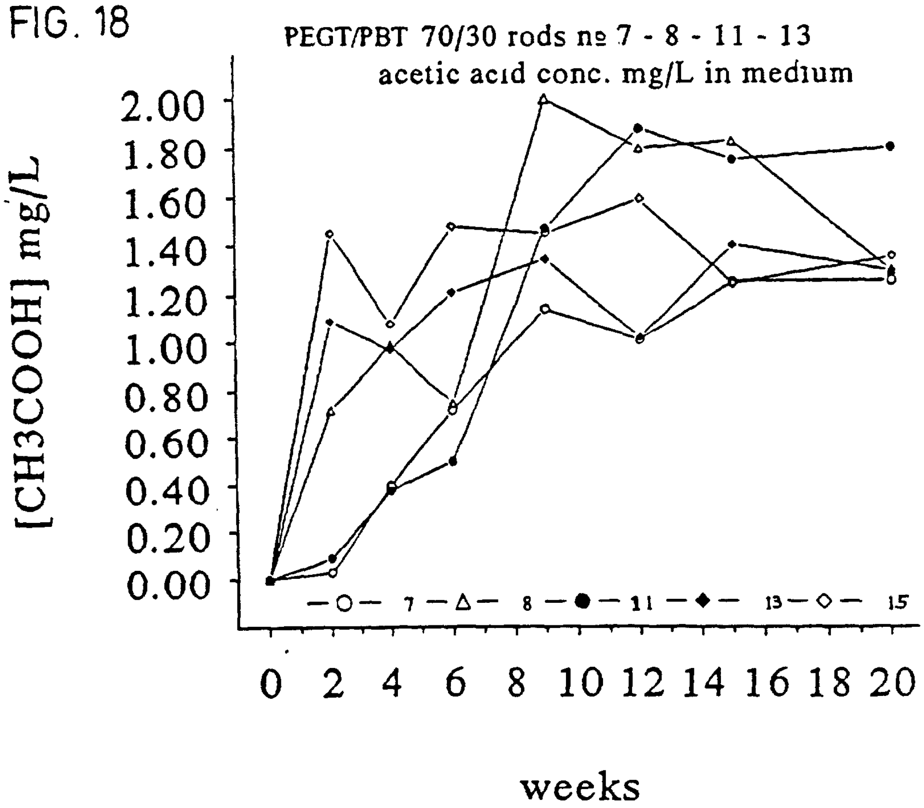
PEGT/PBT 70/30 rods nº 7 - 8 - 11 - 13
acetic acid conc. mg/L in medium
[CH3COOH] mg/L
2.00
1.80
1.60
1.40
1.20
1.00
0.80
0.60
0.40
0.20
0.00
7
8
11
13
15
0 2 4 6 8 10 12 14 16 18 20
weeks

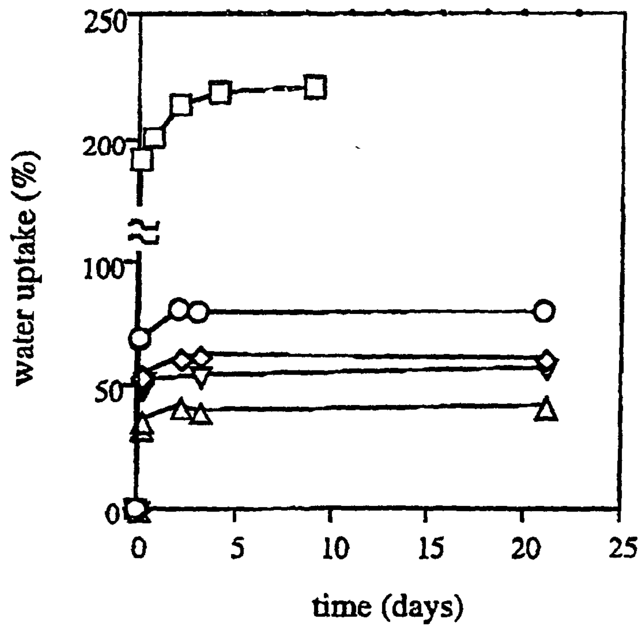


**Figure 19.** Water uptake of PEGT/PBT copolymer films as a function of time. (□) 4000PEG80PBT20; (o) 1000PEG70PBT30; (◊) 1000PEG60PBT40; (Δ) 1000PEG40PBT60; (∇) 600PEG77PBT23. $n = 3 \pm$ S.D.

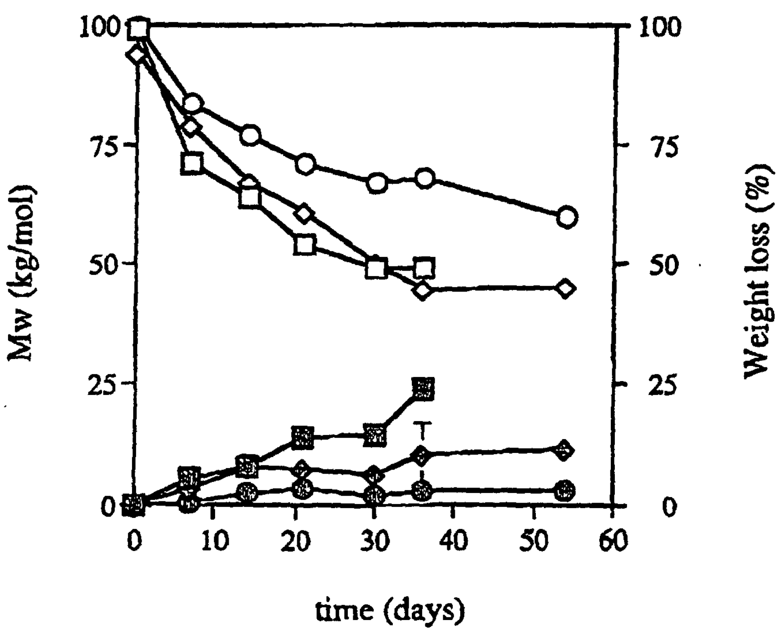


**Figure 20.** Degradation of 1000PEG70PBT30 (□), 1000PEG60PBT40; (◊) and 1000PEG60PBT40 (o) films in PBS at 37°C. Filled symbols indicate weight loss ($n$=2 ± S.D.), open symbols represent $M_w$.

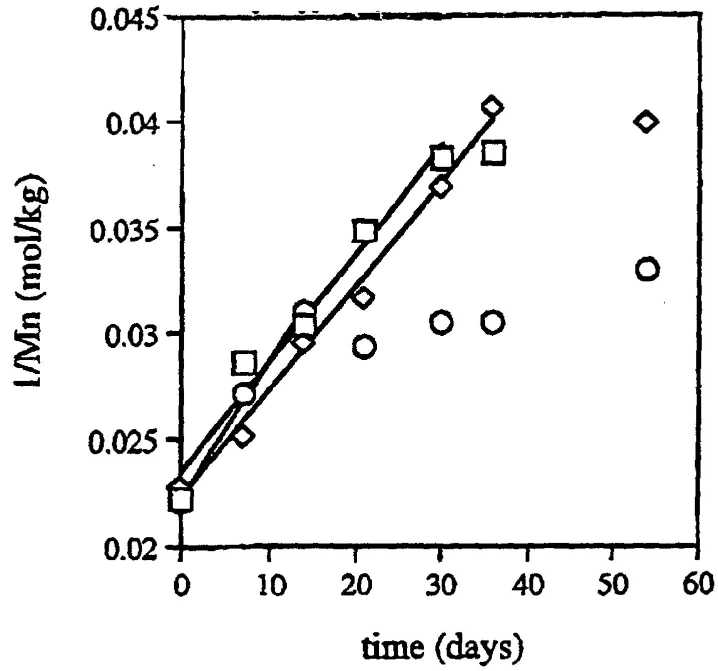


**Figure 21.** Change of the reciprocal $M_n$ as a function of incubation time in PBS at 37°C of 1000PEG70PBT30 (□), 1000PEG60PBT40; (◊) and 1000PEG60PBT40 (o) films.

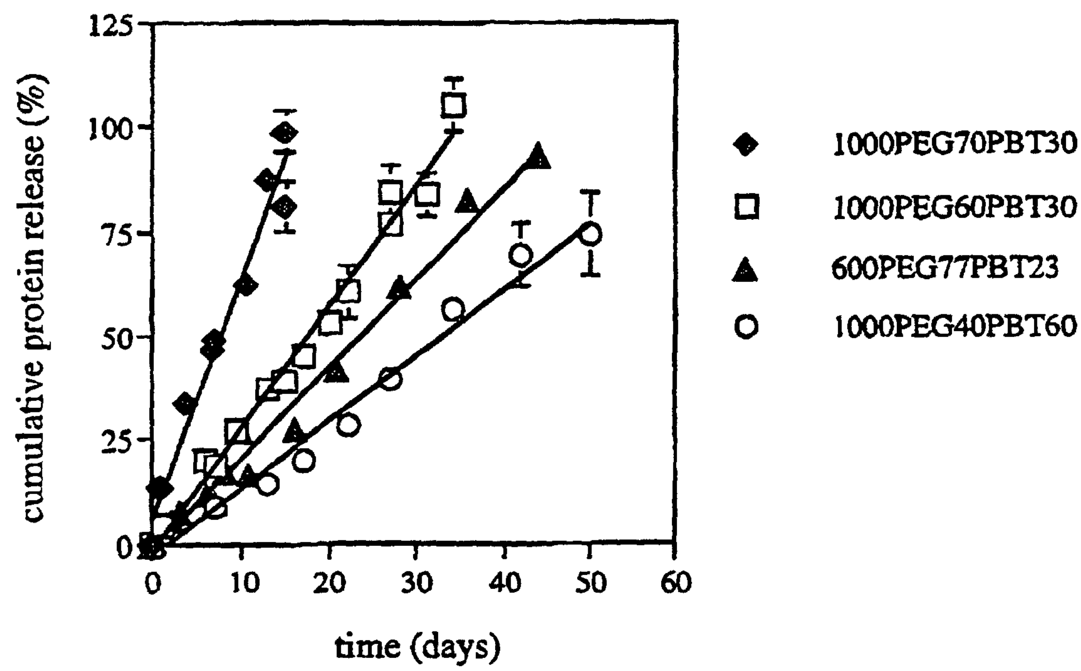


**Figure 22A** Cumulative release of lysozyme from PEGT/PBT films. $n = 3 \pm$ S.D.

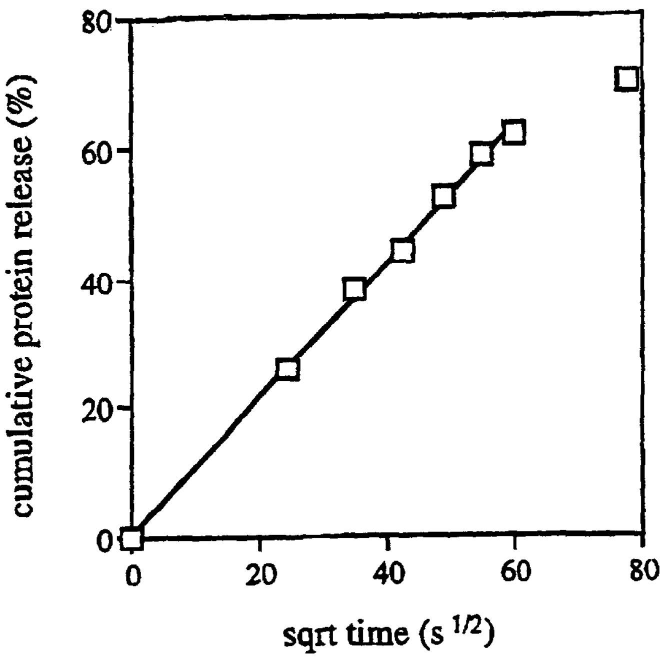


**Fig'ire 22B.** Cumulative release of lysozyme from 4000PEG80PBT20 films. $n = 3 \pm$ S.D.

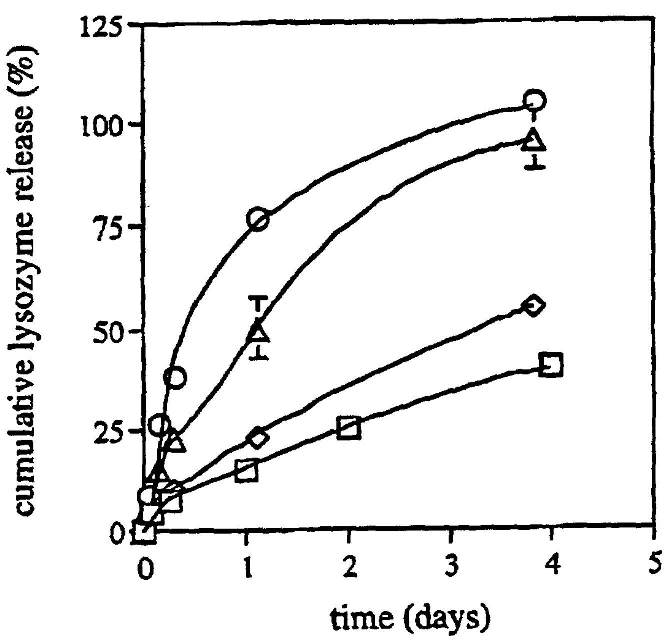


**Figure 23.** Effect of polymer molecular weight on cumulative lysozyme release from 1000PEG70PBT30 films. $M_n$ = 45100 (□), 42000 (◇), 36600 (△) or 28700 g/mol (o).

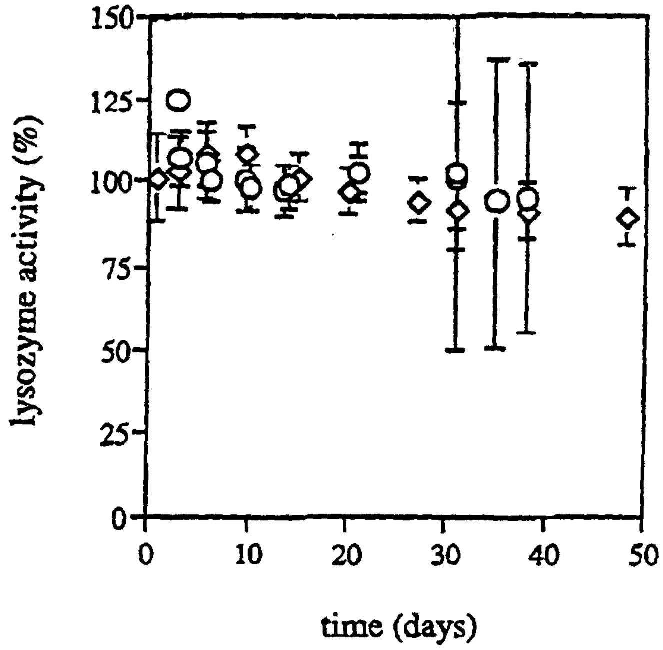


**Figure 24.** Activity of lysozyme released from 1000PEG60PBT40 (o) and 1000PEG40PBT60 (◊) matrices. ($n$=3 ± S.D.).

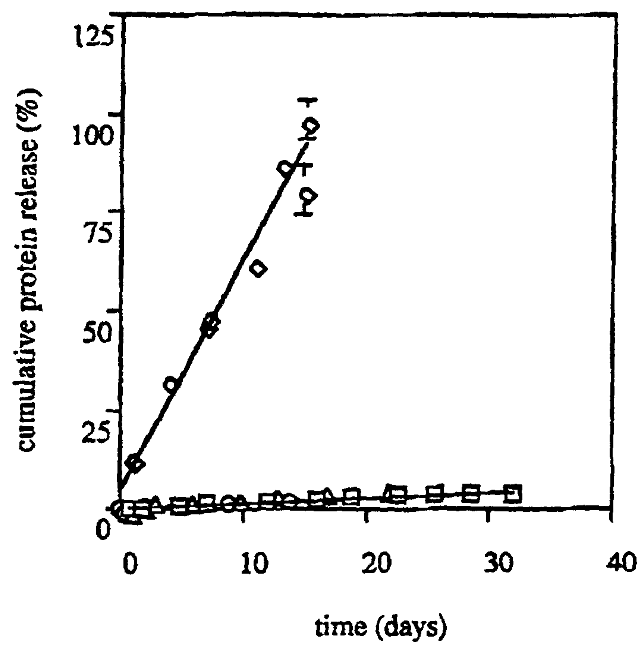


**Figure 25.** Cumulative protein release (%) from 1000PEG70PBT30 matrices. Release of lysozyme (◊), BSA (□) and IgG (Δ). Films were prepared from water-in-oil emulsions containing 0.6 ml lysozyme solution (55 mg/ml PBS) and 1 g polymer (9 wt% in $CHCl_3$). (n=3 ± s.d.).

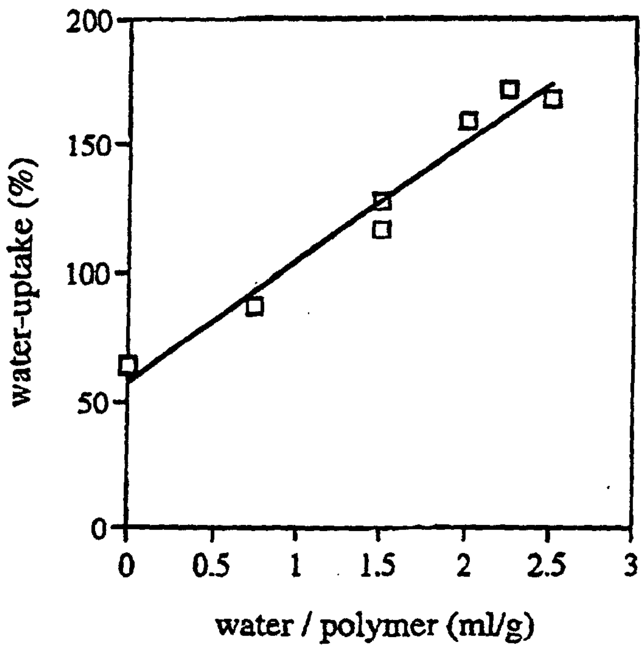


**Figure 26.** Influence of the water/polymer ratio of the initial water-in-oil emulsion on the water uptake of BSA 1000PEG70PBT30 films. (n=3 ± s.d.).

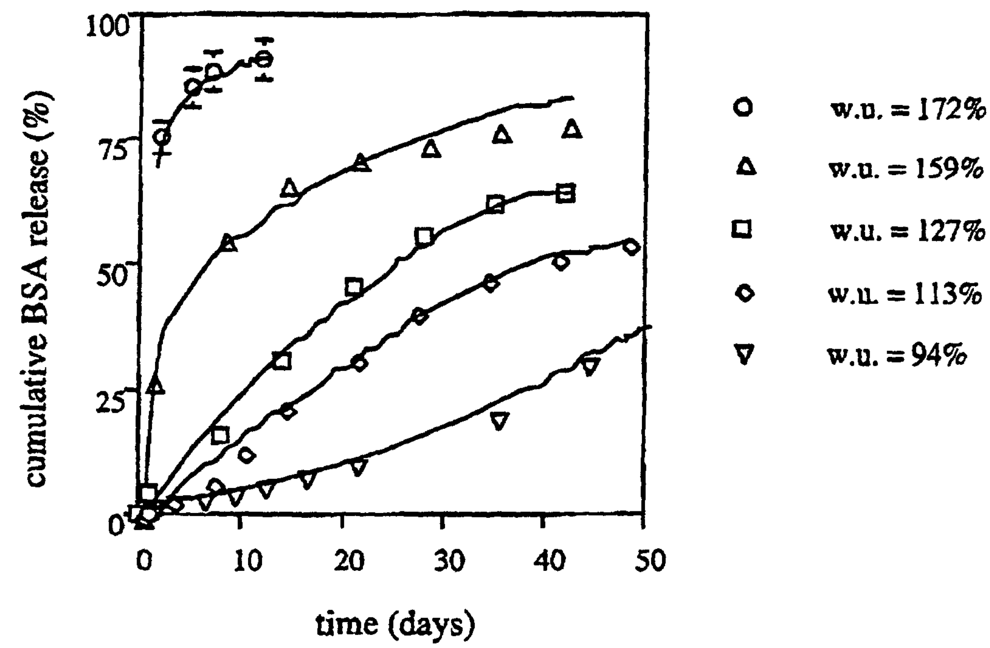


**Figure 27.** Effect of the porosity of 1000PEG70PBT30 on the cumulative release of BSA. Water uptake (w.u.) is taken as a measure of the porosity. (n=3 ± s.d.).

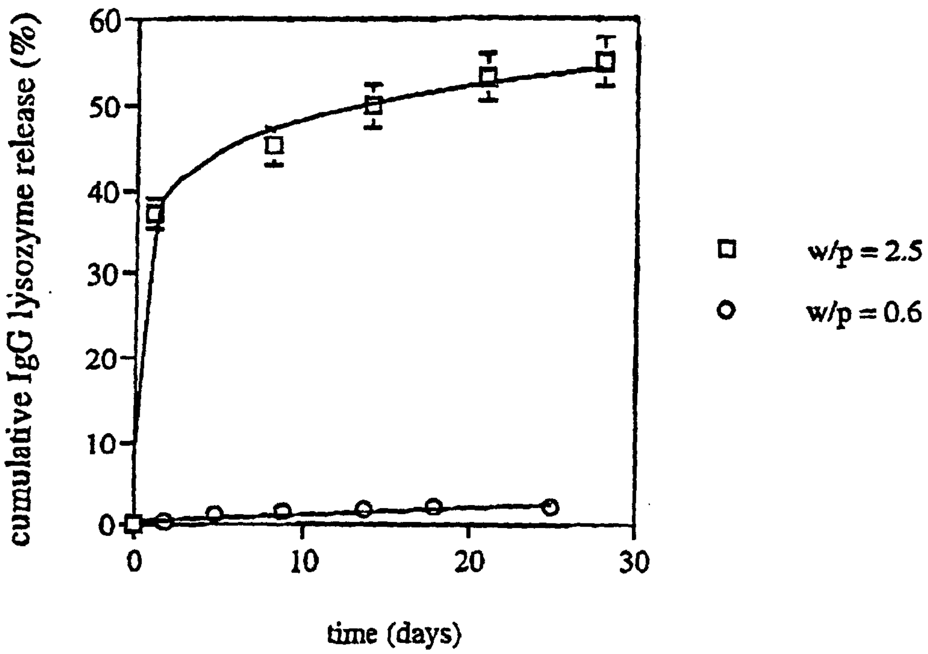


**Figure 28**. Effect of the water/polymer ratio of the initial water-in-oil emulsion (W/P) and water uptake on the cumulative release of IgG from 1000PEG70PBT30 matrices. (n=3 ± s.d.).

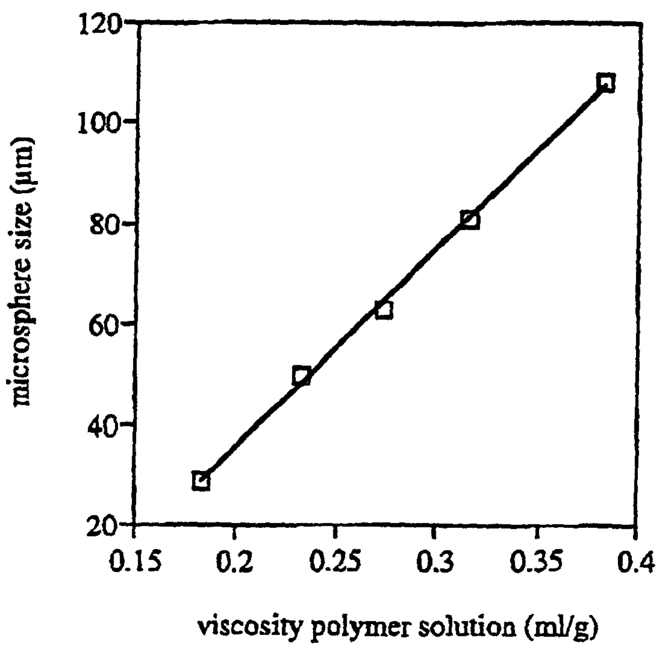


**Figure 29**.: mean diameter (volume) of lysozyme loaded 600PEG77PBT23 microspheres as a function of the viscosity of polymer solutions.

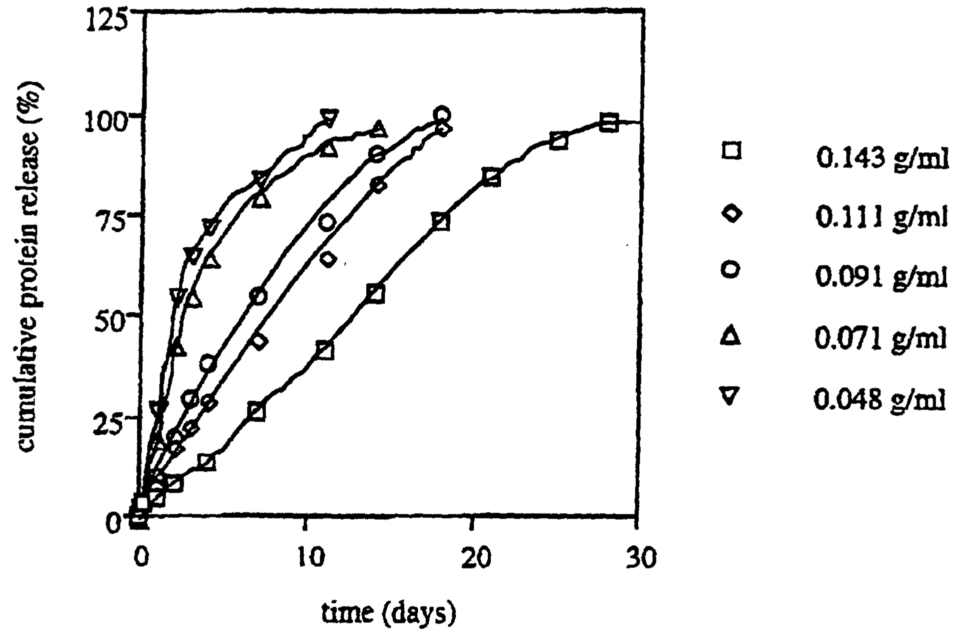


**Figure 30**: lysozyme release from 600PEG77PBT23 microspheres prepared from polymer solutions with different concentrations.

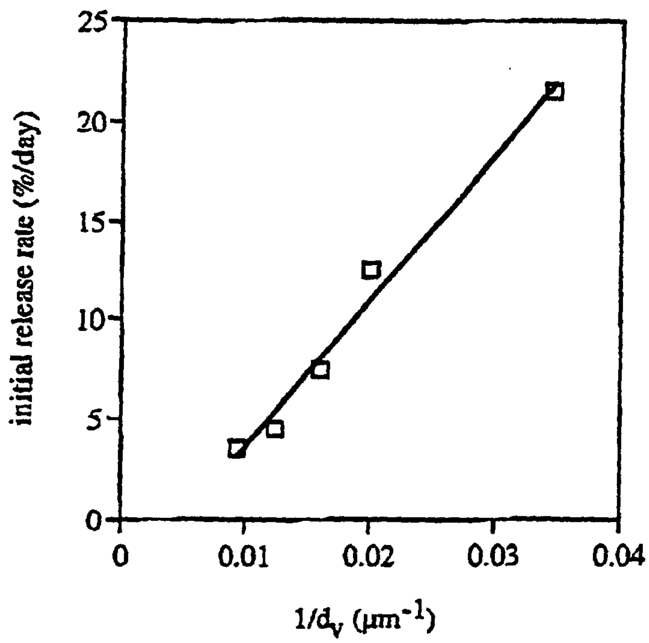


**Figure 31**: initial release rate of lysozyme from 600PEG77PBT23 microspheres as a function of the reciprocal microsphere size.

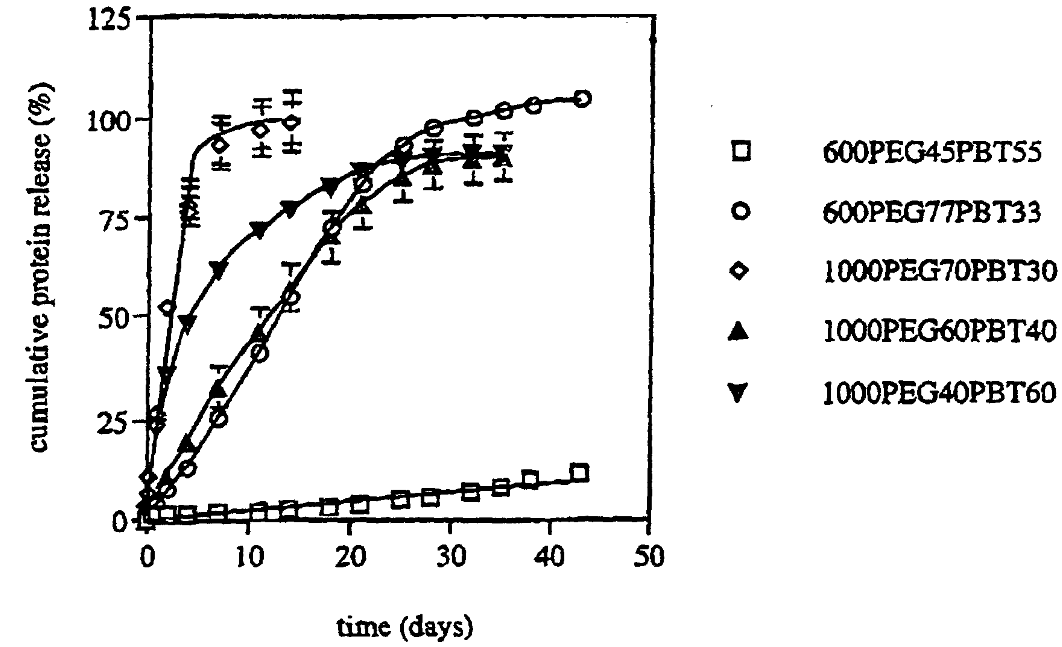


**Figure 32:** lysozyme release from PEGT/PBT microspheres as a function of copolymer composition.

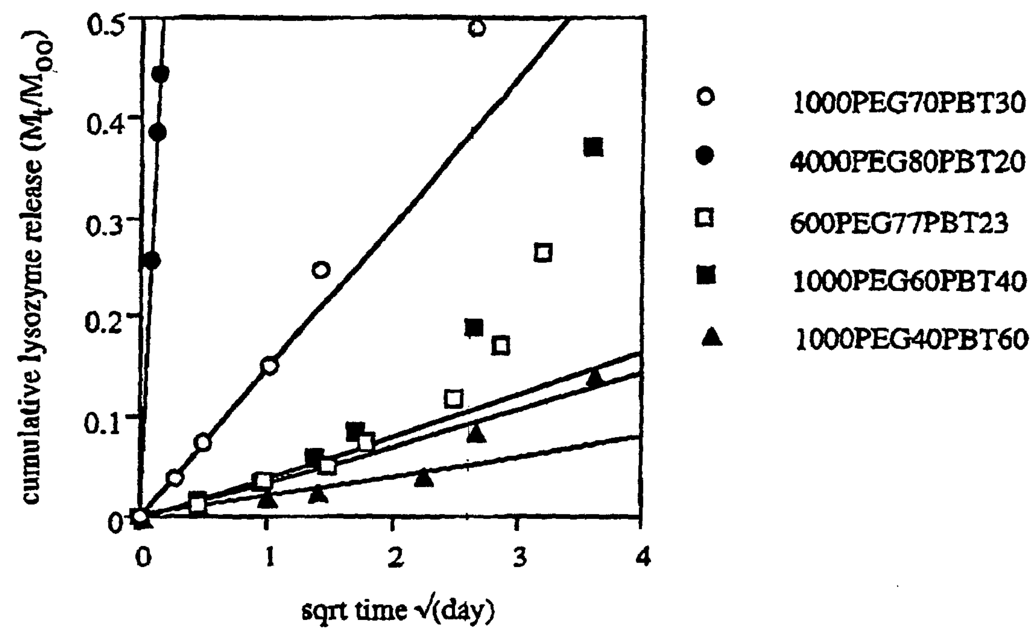


**Figure 33:** Plot of $M_t/M_\infty$ versus $(\text{time})^{1/2}$ for the release of lysozyme from PEGT/PBT matrices for different copolymers.

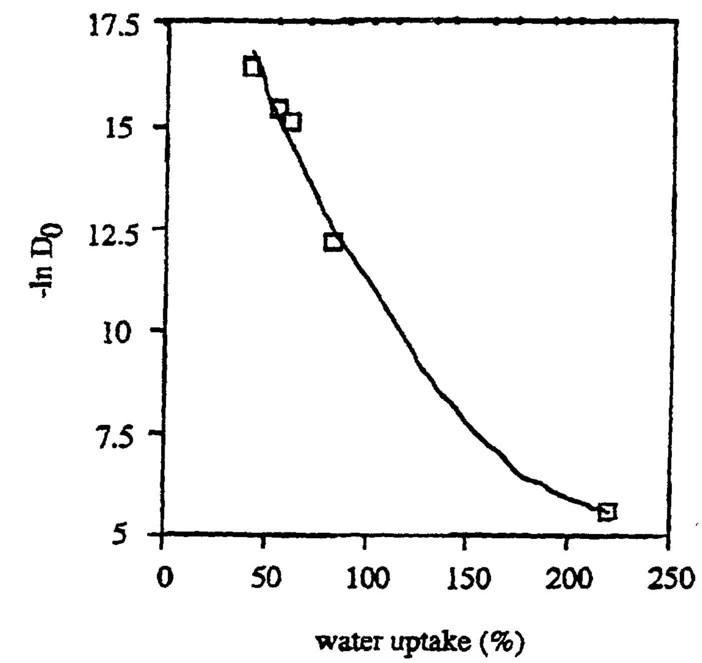


**Figure 34:** Initial diffusion coefficient of lysozyme through PEGT/PBT matrices as a function of the water uptake.

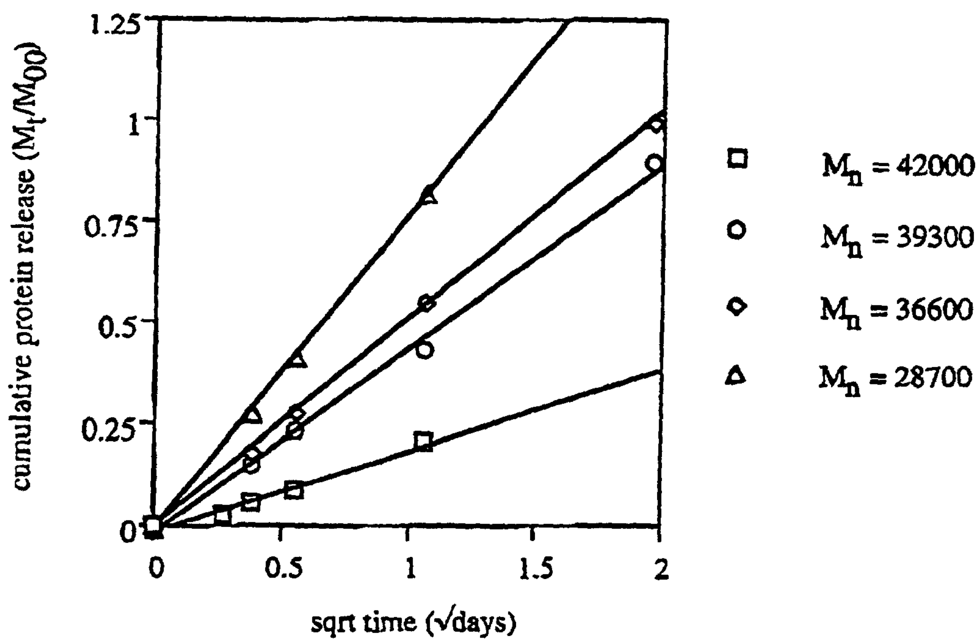


**Figure 35:** Plot of $M_t/M_\infty$ versus $(time)^{1/2}$ for the release of lysozyme from 1000PEG70PBT30 matrices with different molecular weight.

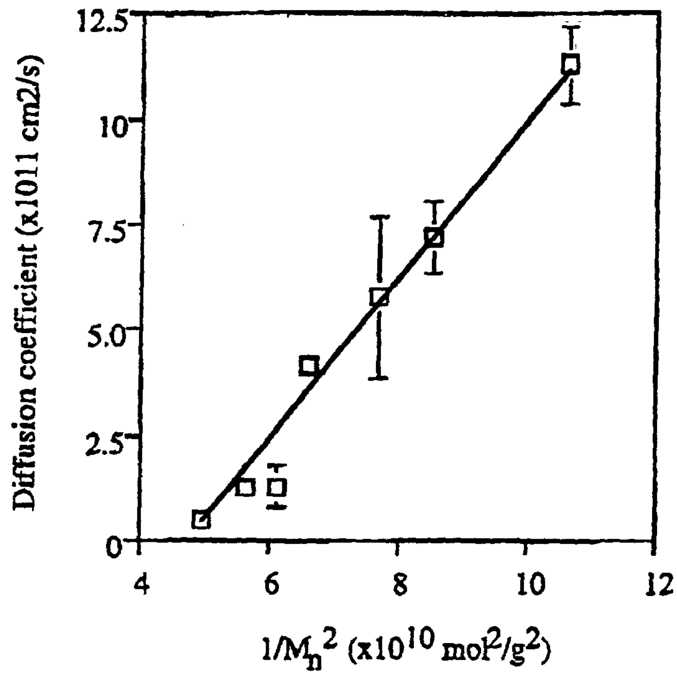


**Figure 36.** Initial diffusion coefficient of lysozyme through 1000PEG70PBT30 matrices as a function of the molecular weight.

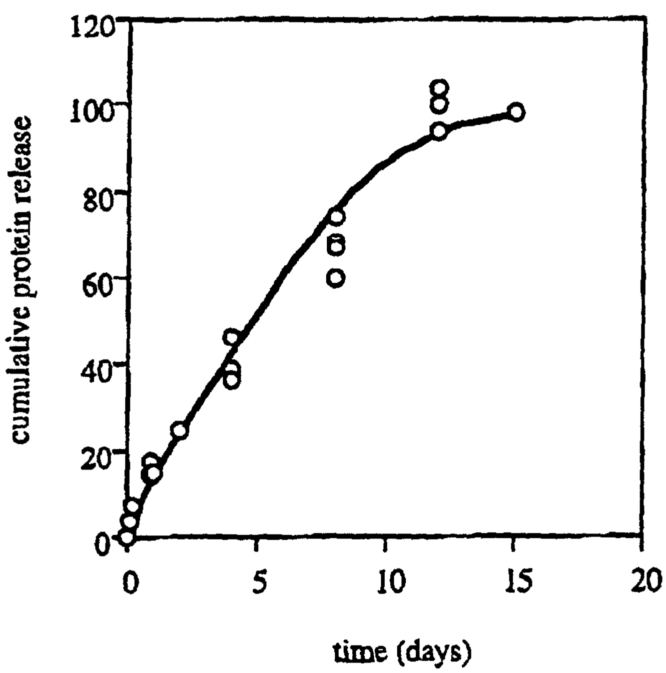


**Figure 37 A**

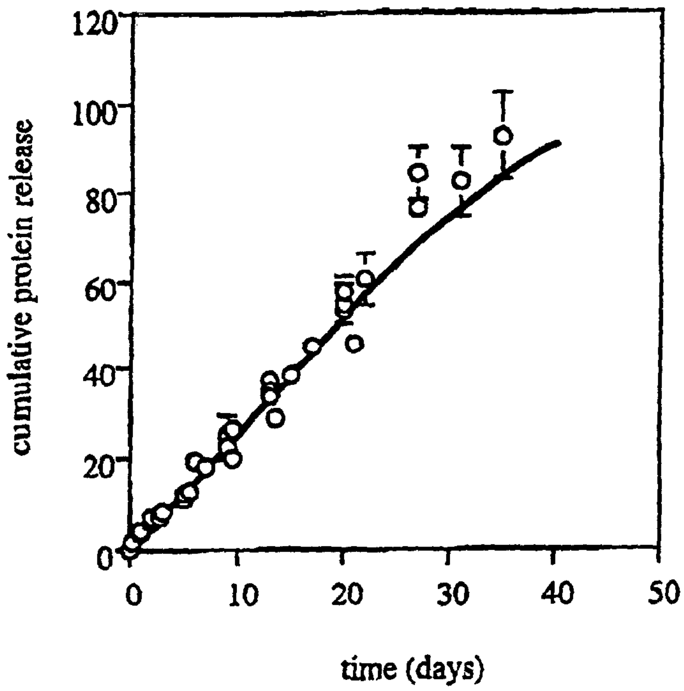


Figure 37 B

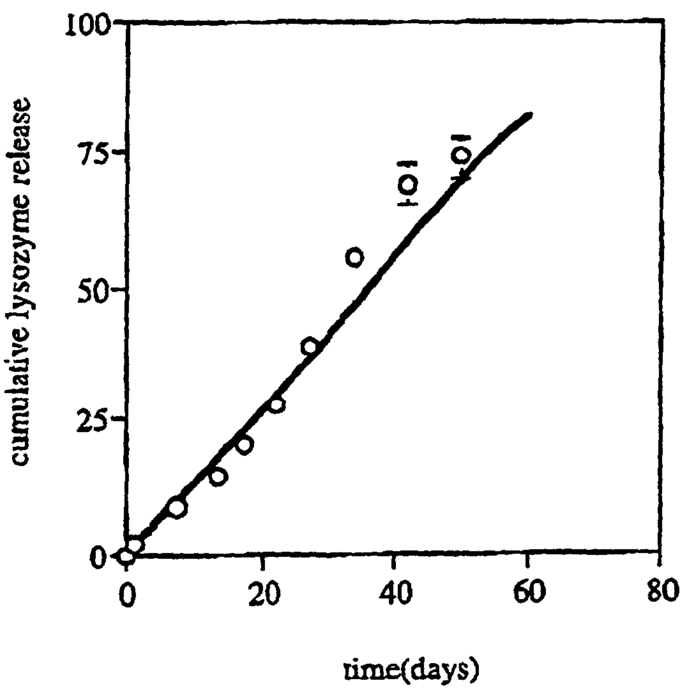


Figure 37 C

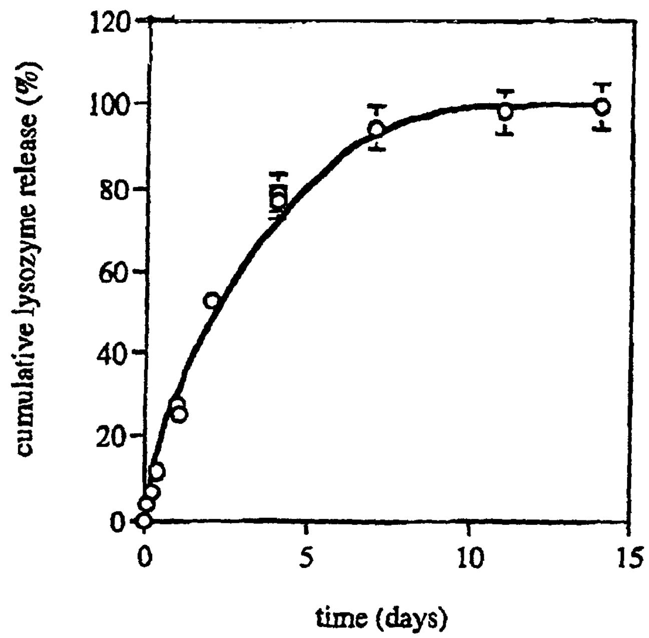


Figure 37 D

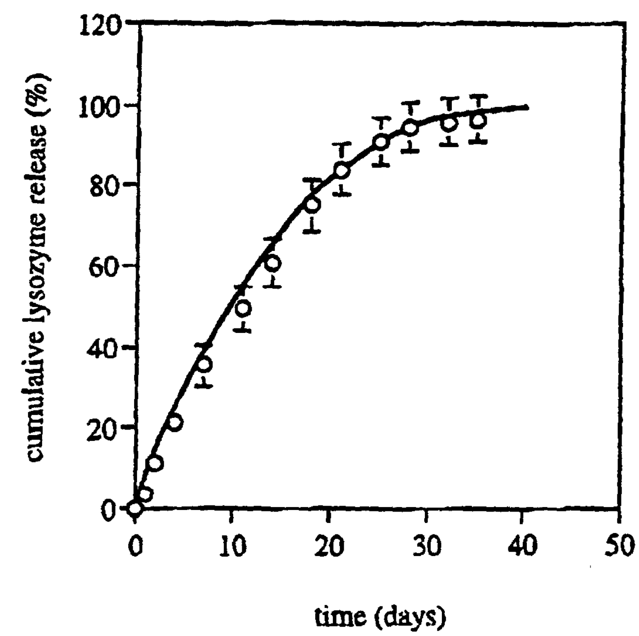


Figure 37 E

**Figure 37:** Prediction of protein release from 1000PEG70PBT30 films (A), 1000PEG60PBT40 films (B), 1000PEG40PBT60 films (C), 1000PEG70PBT30 microspheres (D) and 1000PEG60PBT40 microspheres (E).

——— calculated  o ' experimental